(19) Europäisches Patentamt
European Patent Office
Office européen des brevets

(11) EP 4 527 833 A1

(12) EUROPEAN PATENT APPLICATION
published in accordance with Art. 153(4) EPC

(43) Date of publication:
**26.03.2025 Bulletin 2025/13**

(21) Application number: **23806904.1**

(22) Date of filing: **16.05.2023**

(51) International Patent Classification (IPC):
***C07D 211/60*** (2006.01) ***C07D 205/04*** (2006.01)
***C07D 207/16*** (2006.01) ***A61K 31/452*** (2006.01)
***A61K 31/395*** (2006.01) ***A61K 31/397*** (2006.01)
***A61P 23/02*** (2006.01)

(52) Cooperative Patent Classification (CPC):
**A61K 31/395; A61K 31/397; A61K 31/452; A61P 23/02; C07D 205/04; C07D 207/16; C07D 211/60**

(86) International application number:
**PCT/CN2023/094390**

(87) International publication number:
**WO 2023/221952 (23.11.2023 Gazette 2023/47)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **18.05.2022 CN 202210551906**

(71) Applicant: **Yichang Humanwell Pharmaceutical Co., Ltd.**
**Yichang, Hubei 443005 (CN)**

(72) Inventors:
- **LI, Lie**
  **Yichang, Hubei 443005 (CN)**
- **ZHOU, Hao**
  **Yichang, Hubei 443005 (CN)**
- **LIAO, Zongquan**
  **Yichang, Hubei 443005 (CN)**
- **LIANG, Dali**
  **Yichang, Hubei 443005 (CN)**
- **LIU, Rong**
  **Yichang, Hubei 443005 (CN)**
- **LV, Jinliang**
  **Yichang, Hubei 443005 (CN)**
- **TIAN, Luanyuan**
  **Yichang, Hubei 443005 (CN)**
- **ZHOU, Xiujie**
  **Yichang, Hubei 443005 (CN)**
- **LIU, Zewen**
  **Yichang, Hubei 443005 (CN)**
- **XIAO, Xiaoli**
  **Yichang, Hubei 443005 (CN)**

(74) Representative: **Müller, Christian Stefan Gerd**
**ZSP Patentanwälte PartG mbB**
**Hansastraße 32**
**80686 München (DE)**

# (54) CYCLIC QUATERNARY AMMONIUM SALT COMPOUND, AND PREPARATION METHOD THEREFOR AND USE THEREOF

(57) Provided in the present application are a cyclic quaternary ammonium salt compound, and a preparation method therefor and the use thereof. Further provided are the cyclic quatermary ammonium salt compound as represented by general formula (I), or an isomer or a solvate thereof, and a composition thereof and the use thereof in the preparation of anesthetic or analgesic drugs. Each substituent in the general formula (I) is the same as that defined in the description.

EP 4 527 833 A1

## Description

This application claims the priority of Chinese Patent Application No. 202210551906.7, filed with the China National Intellectual Property Administration on May 18, 2022, and titled with "CYCLIC QUATERNARY AMMONIUM SALT COMPOUND, AND PREPARATION METHOD THEREFOR AND USE THEREOF", which is hereby incorporated by reference in entirety.

## FIELD

The present application relates to but is not limited to the thchnical field of pharmaceutical chemistry, and in particular to a cyclic quaternary ammonium salt compound and a preparation method and use thereof.

## BACKGROUND

Local anesthetics are a class of drugs applied topically to nerve trunks or nerve endings to temporarily, completely and reversibly block the generation and conduction of nerve impulse, leading to temporary loss of sense of pain in the localized area. These local anesthetics act by binding to specific sites on sodium channels in the nerve cell membrane, which reduces the influx of $Na^+$ and alters the membrane potential, thereby effectively blocking the conduction of nerve impulses and producing the anesthetic effect. Local anesthetics are favored in clinical practice to treat sense of pain due to their precise action, minimal risk of hyperalgesia, ease of local administration, low blood drug concentration, and few systemic side effects.

Local anesthetics currently used in clinical practice are primarily compounds formed by aromatic and amine groups linked by ester or amide bonds. Examples include procaine, tetracaine, lidocaine, bupivacaine, and ropivacaine. Although bupivacaine and ropivacaine are classified as new long-acting local anesthetics, their analgesic effect typically lasts no longer than 8 hours ("Local anesthetics: review of pharmacological considerations," Becker D. E. et al., Anesth Prog., 2012, 59(2): 90-102). This duration is generally sufficient for most surgical and invasive procedures but often falls short for managing postoperative pain and chronic pain.

Although QX-314, a quaternary ammonium salt derivative of lidocaine, can effectively block sodium ion currents after passing through the cell membrane and produce a long-lasting anesthetic effect, its permanent positive charge hinders its ability to actively pass through the lipid-soluble cell membrane ("Mechanism of frequency-dependent inhibition of sodium currents in frog myelinated nerve by the lidocaine derivative GEA," Courtney K. R., J Pharmacol Exp Ther., 1975, 195: 225-236). Studies have shown that when QX-314 is combined with local anesthetics or transient receptor potential (TRP) channel agonists, it can traverse the cell membrane and produce a prolonged local anesthetic effect. However, this combination can also lead to increased local neurotoxicity and systemic toxicity ("Anti-nociceptive and desensitizing effects of olvanil on capsaicin-induced thermal hyperalgesia in the rat," Alsalem M. et al., BMC Pharmacol Toxicol., 2016, 17(1): 31).

Currently, no new long-acting local anesthetic has been developed in clinical that combines effective local anesthesia, prolonged duration, rapid onset, good safety, and low skin irritation. Therefore, there is an urgent need to address the demand for long-term and safe postoperative analgesia.

## SUMMARY

Based on this, the present application provides a novel cyclic quaternary ammonium salt compound and a preparation method and use of the compound. This compound features a novel structure and offers several advantages, including prolonged local anesthesia from a single administration, no risk of motor nerve damage, and no neurotoxicity.

In a first aspect, the present application provides a compound characterized by the structural formula (I), or a stereoisomer or solvate thereof:

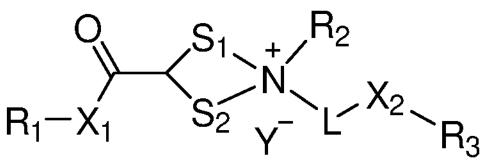

**(I)**

wherein:

$R_1$ is selected from the group consisting of aryl and heteroaryl, alternatively, $R_1$, together with $X_1$, form non-condensed or condensed azacycloalkyl;

$R_2$ is selected from the group consisting of $C_{1-8}$ alkyl and $C_{3-12}$ cycloalkyl;

$R_3$ is selected from the group consisting of aryl, heteroaryl, and heterocycloalkyl, alternatively, $R_3$, together with $X_2$, form condensed azacycloalkyl;

$X_1$ is selected from the group consisting of O, S, and $NR_4^A$, wherein $R_4^A$ is selected from the group consisting of hydrogen, deuterium, $C_{1-8}$ alkyl, $C_{3-8}$ cycloalkyl, and $C_{1-4}$ alkoxy $C_{1-4}$ alkyl, alternatively, $R_4^A$ and the N it is attached to, together with $R_1$, form non-condensed or condensed azacycloalkyl;

$X_2$ is selected from the group consisting of O, S, and $NR_4^B$, wherein $R_4^B$ is selected from the group consisting of hydrogen, deuterium, $C_{1-8}$ alkyl, $C_{3-8}$ cycloalkyl, and $C_{1-4}$ alkoxy $C_{1-4}$ alkyl, alternatively, $R_4^B$ and the N it is attached to, together with $R_3$, form condensed azacycloalkyl;

L is selected from $C_{1-8}$ alkylene;

$S_1$ and $S_2$ are each independently selected from the group consisting of a bond and $C_{1-6}$ alkylene, with the provision that $S_1$ and $S_2$ are not both bonds; and

$Y^-$ represents a pharmaceutically acceptable anion.

In a second aspect, the present application provides a method for producing the compound represented by formula (I) or the stereoisomer or solvate thereof as described in any one of the first aspect of the present application, comprising the following steps:

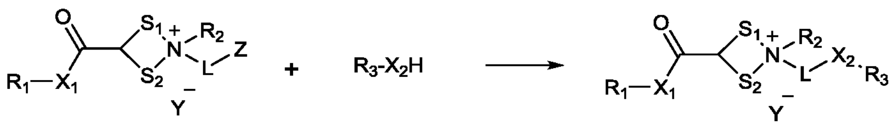

(II) (III) (I)

reacting the compound represented by formula (II) with the compound represented by formula (III) to obtain the compound represented by formula (I);

wherein, Z in formula (II) is an electron-withdrawing leaving group such as bromine, chloride or sulfonate, and the groups in formula (II) and formula (III) are defined as in formula (I).

In a third aspect, the present application provides a pharmaceutical composition comprising the compound represented by formula (I) or the stereoisomer or solvate thereof as described in any one of the first aspects of the present application, and a pharmaceutically acceptable carrier, excipient or diluent.

In a fourth aspect, the present application provides use of the compound represented by formula (I) or the stereoisomer or solvate thereof as described in any one of the first aspect of the present application, or the pharmaceutical composition in the third aspect of the present application in the manufacture of a medicament for local anesthesia or analgesia.

In a fifth aspect, the present application provides a method of local anesthesia or analgesia, comprising administering to a subject in need thereof a therapeutically effective amount of the compound represented by formula (I) or the stereoisomer or solvate thereof as described in any one of the first aspect of the present application, or the pharmaceutical composition in the third aspect of the present application, wherein the administration is topical administration via transdermal, subcutaneous, intradermal, intramuscular, near nerves, intrapulpal, intraspinal, epidural, intravenous, or mucosal routes such as eye drops.

## DETAILED DESCRIPTION

In a first aspect, the present application provides a compound characterized by the structural formula (I), or a stereoisomer or solvate thereof:

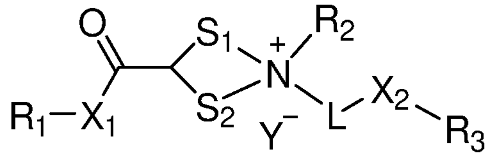

(I)

wherein:

$R_1$ is selected from the group consisting of aryl and heteroaryl, alternatively, $R_1$, together with $X_1$, form non-condensed or condensed azacycloalkyl;

$R_2$ is selected from the group consisting of $C_{1-8}$ alkyl and $C_{3-12}$ cycloalkyl;

$R_3$ is selected from the group consisting of aryl, heteroaryl, and heterocycloalkyl, alternatively, $R_3$, together with $X_2$, form condensed azacycloalkyl;

$X_1$ is selected from the group consisting of O, S, and $NR_4^A$, wherein $R_4^A$ is selected from the group consisting of hydrogen, deuterium, $C_{1-8}$ alkyl, $C_{3-8}$ cycloalkyl, and $C_{1-4}$ alkoxy $C_{1-4}$ alkyl, alternatively, $R_4^A$ and the N it is attached to, together with $R_1$, form non-condensed or condensed azacycloalkyl;

$X_2$ is selected from the group consisting of O, S, and $NR_4^B$, wherein $R_4^B$ is selected from the group consisting of hydrogen, deuterium, $C_{1-8}$ alkyl, $C_{3-8}$ cycloalkyl, and $C_{1-4}$ alkoxy $C_{1-4}$ alkyl, alternatively, $R_4^B$ and the N it is attached to, together with $R_3$, form condensed azacycloalkyl;

L is selected from $C_{1-8}$ alkylene;

$S_1$ and $S_2$ are each independently selected from the group consisting of a bond and $C_{1-6}$ alkylene, with the provision that $S_1$ and $S_2$ are not both bonds; and

$Y^-$ represents a pharmaceutically acceptable anion.

In an embodiment, in the compound represented by formula (I) or the stereoisomer or solvate thereof provided in the present application, L is not $C_3$ alkylene.

In an embodiment of the first aspect of the present application, the present application provides a compound characterized by the structural formula (I), or a stereoisomer or solvate thereof:

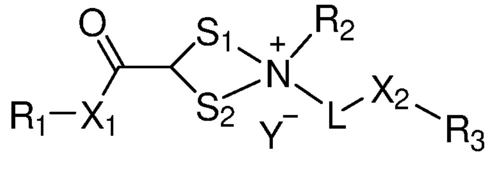

(I)

wherein:

$R_1$ is selected from the group consisting of aryl and heteroaryl, alternatively, $R_1$, together with $X_1$, form non-condensed

or condensed azacycloalkyl; wherein the aryl, the heteroaryl, the non-condensed azacycloalkyl or the condensed azacycloalkyl is optionally substituted by a group selected from the group consisting of $C_{1-6}$ alkyl, $C_{1-6}$ alkoxy, cyano, halogen, hydroxyl, amino, nitro, ester, mono-$C_{1-6}$ alkylamino, di-$C_{1-6}$ alkylamino, $C_{2-4}$ alkenyl, $C_{2-4}$ alkynyl, $C_{1-6}$ haloalkyl, $C_{1-6}$ haloalkoxy, and a combination thereof; the condensed azacycloalkyl is condensed by aryl or heteroaryl;

$R_2$ is selected from the group consisting of $C_{1-8}$ alkyl and $C_{3-12}$ cycloalkyl, wherein the $C_{1-8}$ alkyl or $C_{3-12}$ cycloalkyl is optionally substituted by a group selected from the group consisting of $C_{1-6}$ alkyl, $C_{1-6}$ alkoxy, $C_{1-6}$ haloalkyl, $C_{3-6}$ cycloalkyl, halogen, hydroxyl, cyano, amino, and a combination thereof;

$R_3$ is selected from the group consisting of aryl, heteroaryl, and heterocycloalkyl, alternatively, $R_3$, together with $X_2$, form condensed azacycloalkyl; wherein the aryl, the heteroaryl, the heterocycloalkyl, or the condensed azacycloalkyl is optionally substituted by a group selected from the group consisting of $C_{1-6}$ alkyl, $C_{1-6}$ alkoxy, $C_{1-6}$ alkoxy $C_{1-4}$ alkyl, $C_{1-6}$ haloalkyl, $C_{1-6}$ haloalkoxy, $C_{3-6}$ cycloalkyl, halogen, hydroxyl, cyano, amino, ester, nitro, sulfonyl, thiol, mono-$C_{1-6}$ alkylamino, di-$C_{1-6}$ alkylamino, $C_{2-4}$ alkenyl, $C_{2-4}$ alkynyl, optionally substituted aryl, optionally substituted heteroaryl, optionally substituted heterocycloalkyl, and a combination thereof; wherein the optionally substituted aryl, the optionally substituted heteroaryl, and the optionally substituted heterocycloalkyl refer to unsubstituted aryl, unsubstituted heteroaryl, and unsubstituted heterocycloalkyl, or are substituted by a group selected from the group consisting of $C_{1-6}$ alkyl, $C_{1-6}$ alkoxy, $C_{1-6}$ haloalkyl, $C_{1-6}$ haloalkoxy, $C_{3-6}$ cycloalkyl, halogen, hydroxyl, cyano, amino, ester, nitro, mono-$C_{1-6}$ alkylamino, di-$C_{1-6}$ alkylamino, $C_{2-4}$ alkenyl, $C_{2-4}$ alkynyl, and a combination thereof; the condensed azacycloalkyl is condensed by aryl or heteroaryl;

$X_1$ is selected from the group consisting of O, S, and $NR_4^A$, wherein $R_4^A$ is selected from the group consisting of hydrogen, deuterium, $C_{1-8}$ alkyl, $C_{3-8}$ cycloalkyl, and $C_{1-4}$ alkoxy $C_{1-4}$ alkyl, alternatively, $R_4^A$ and the N it is attached to, together with $R_1$, form non-condensed or condensed azacycloalkyl; the condensed azacycloalkyl is condensed by aryl or heteroaryl;

$X_2$ is selected from the group consisting of O, S, and $NR_4^B$, wherein $R_4^B$ is selected from the group consisting of hydrogen, deuterium, $C_{1-8}$ alkyl, $C_{3-8}$ cycloalkyl, and $C_{1-4}$ alkoxy $C_{1-4}$ alkyl, alternatively, $R_4^B$ and the N it is attached to, together with $R_3$, form condensed azacycloalkyl; the condensed azacycloalkyl is condensed by aryl or heteroaryl;

L is selected from $C_{1-8}$ alkylene; wherein the $C_{1-8}$ alkylene is optionally substituted by a group selected from the group consisting of $C_{1-4}$ alkyl, $C_{1-4}$ alkoxy, $C_{1-4}$ haloalkyl, $C_{1-4}$ haloalkoxy, $C_{3-6}$ cycloalkyl, halogen, hydroxy, cyano, amino, ester, nitro, mono-$C_{1-4}$ alkylamino, di-$C_{1-4}$ alkylamino, $C_{2-4}$ alkenyl, $C_{2-4}$ alkynyl, and a combination thereof;

$S_1$ and $S_2$ are each independently selected from the group consisting of a bond and $C_{1-6}$ alkylene, with the provision that $S_1$ and $S_2$ are not both bonds, wherein the $C_{1-6}$ alkylene optionally contains, on the main chain, one heteroatom selected from the group consisting of O, S, and $NR_5$, wherein $R_5$ is hydrogen or deuterium;

$Y^-$ represents a pharmaceutically acceptable anion.

In an embodiment of the first aspect of the present application, the present application provides a compound characterized by the structural formula (I), or a stereoisomer or solvate thereof:

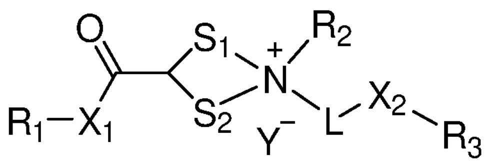

**(I)**

wherein:

$R_1$ is selected from the group consisting of aryl and heteroaryl, alternatively, $R_1$, together with $X_1$, form non-condensed or condensed azacycloalkyl; wherein the aryl, the heteroaryl, the non-condensed azacycloalkyl or the condensed azacycloalkyl is optionally substituted by a group selected from the group consisting of $C_{1-6}$ alkyl, $C_{1-6}$ alkoxy, cyano, halogen, hydroxyl, amino, nitro, ester, mono-$C_{1-6}$ alkylamino, di-$C_{1-6}$ alkylamino, $C_{2-4}$ alkenyl, $C_{2-4}$ alkynyl, $C_{1-6}$ haloalkyl, $C_{1-6}$ haloalkoxy, and a combination thereof; the condensed azacycloalkyl is condensed by aryl or heteroaryl;

$R_2$ is selected from the group consisting of $C_{1-8}$ alkyl and $C_{3-12}$ cycloalkyl, wherein the $C_{1-8}$ alkyl or $C_{3-12}$ cycloalkyl is optionally substituted by a group selected from the group consisting of $C_{1-6}$ alkyl, $C_{1-6}$ alkoxy, $C_{1-6}$ haloalkyl, $C_{3-6}$ cycloalkyl, halogen, hydroxyl, cyano, amino, and a combination thereof; and/or

$R_3$ is selected from the group consisting of aryl, heteroaryl, and heterocycloalkyl, alternatively, $R_3$, together with $X_2$, form condensed azacycloalkyl; wherein the aryl, the heteroaryl, the heterocycloalkyl, or the condensed azacycloalkyl is optionally substituted by a group selected from the group consisting of $C_{1-6}$ alkyl, $C_{1-6}$ alkoxy, $C_{1-6}$ alkoxy $C_{1-4}$ alkyl, $C_{1-6}$ haloalkyl, $C_{1-6}$ haloalkoxy, $C_{3-6}$ cycloalkyl, halogen, hydroxyl, cyano, amino, $C_{1-6}$ alkanoyl, ester, nitro, sulfonyl, thiol, mono-$C_{1-6}$ alkylamino, di-$C_{1-6}$ alkylamino, $C_{2-4}$ alkenyl, $C_{2-4}$ alkynyl, optionally substituted aryl, optionally substituted heteroaryl, optionally substituted heterocycloalkyl, and a combination thereof; wherein the optionally substituted aryl, the optionally substituted heteroaryl, and the optionally substituted heterocycloalkyl refer to unsubstituted aryl, unsubstituted heteroaryl, and unsubstituted heterocycloalkyl, or are substituted by a group selected from the group consisting of $C_{1-6}$ alkyl, $C_{1-6}$ alkoxy, $C_{1-6}$ haloalkyl, $C_{1-6}$ haloalkoxy, $C_{3-6}$ cycloalkyl, halogen, hydroxyl, cyano, amino, $C_{1-6}$ alkanoyl, ester, nitro, mono-$C_{1-6}$ alkylamino, di-$C_{1-6}$ alkylamino, $C_{2-4}$ alkenyl, $C_{2-4}$ alkynyl, and a combination thereof; the condensed azacycloalkyl is condensed by aryl or heteroaryl;

$X_1$ is selected from the group consisting of O, S, and $NR_4^A$, wherein $R_4^A$ is selected from the group consisting of hydrogen, deuterium, $C_{1-8}$ alkyl, $C_{3-8}$ cycloalkyl, and $C_{1-4}$ alkoxy $C_{1-4}$ alkyl, alternatively, $R_4^A$ and the N it is attached to, together with $R_1$, form non-condensed or condensed azacycloalkyl; the condensed azacycloalkyl is condensed by aryl or heteroaryl;

$X_2$ is selected from the group consisting of O, S, and $NR_4^B$, wherein $R_4^B$ is selected from the group consisting of hydrogen, deuterium, $C_{1-8}$ alkyl, $C_{3-8}$ cycloalkyl, and $C_{1-4}$ alkoxy $C_{1-4}$ alkyl, alternatively, $R_4^B$ and the N it is attached to, together with $R_3$, form condensed azacycloalkyl; the condensed azacycloalkyl is condensed by aryl or heteroaryl;

L is selected from $C_{1-8}$ alkylene, with the provision that L is not $C_3$ alkylene; wherein the $C_{1-8}$ alkylene is optionally substituted by a group selected from the group consisting of $C_{1-4}$ alkyl, $C_{1-4}$ alkoxy, $C_{1-4}$ haloalkyl, $C_{1-4}$ haloalkoxy, $C_{3-6}$ cycloalkyl, halogen, hydroxy, cyano, amino, ester, nitro, mono-$C_{1-4}$ alkylamino, di-$C_{1-4}$ alkylamino, $C_{2-4}$ alkenyl, $C_{2-4}$ alkynyl, and a combination thereof;

$S_1$ and $S_2$ are each independently selected from the group consisting of a bond and $C_{1-6}$ alkylene, with the provision that $S_1$ and $S_2$ are not both bonds, wherein the $C_{1-6}$ alkylene optionally contains, on the main chain, one heteroatom selected from the group consisting of O, S, and $NR_5$, wherein $R_5$ is hydrogen or deuterium;

$Y^-$ represents a pharmaceutically acceptable anion.

In an embodiment, in the compound represented by formula (I) or the stereoisomer or solvate thereof provided in the present application, $R_1$ is phenyl or naphthyl; wherein the phenyl or naphthyl is optionally substituted by a group selected from the group consisting of $C_{1-6}$ alkyl, $C_{1-6}$ alkoxy, cyano, halogen, hydroxyl, amino, nitro, ester, mono-$C_{1-6}$ alkylamino, di-$C_{1-6}$ alkylamino, $C_{2-4}$ alkenyl, $C_{2-4}$ alkynyl, $C_{1-6}$ haloalkyl, $C_{1-6}$ haloalkoxy, and a combination thereof.

In an embodiment, in the compound represented by formula (I) or the stereoisomer or solvate thereof provided in the present application, $R_1$ is phenyl, and the phenyl is optionally substituted by a group selected from the group consisting of methyl, ethyl, n-propyl, isopropyl, n-butyl, sec-butyl, tert-butyl, fluorine, chlorine, bromine, iodine, hydroxyl, amino, nitro, carbomethoxy ($CH_3OC(O)$-), carboethoxy ($CH_3CH_2OC(O)$-), and a combination thereof

In an embodiment, in the compound represented by formula (I) or the stereoisomer or solvate thereof provided in the present application, $R_1$ is selected from the group consisting of phenyl, 2-methylphenyl, 2-methoxyphenyl, 4-methylphenyl, 4-methoxyphenyl, 4-fluorophenyl, 4-trifluoromethylphenyl, 2-chlorophenyl, 4-chlorophenyl, 2-bromophenyl, 3-bromophenyl, 4-bromophenyl, 3-hydroxyphenyl, 2,6-dimethylphenyl, 2,6-dimethoxyphenyl, 3-nitrophenyl, 2,6-difluorophenyl, 3-chloro-2-methylphenyl, 2,3-dichlorophenyl, 4-hydroxyphenyl, 2,4,6-trimethylphenyl, 2,4,6-trimethoxyphenyl, and 2,4,6-trifluorophenyl, preferably 2,6-dimethylphenyl.

In an embodiment, in the compound represented by formula (I) or the stereoisomer or solvate thereof provided in the present application, $R_1$ is selected from the group consisting of phenyl, 2-methylphenyl, 2-methoxyphenyl, 4-methylphenyl, 4-methoxyphenyl, 4-fluorophenyl, 4-trifluoromethylphenyl, 2-chlorophenyl, 4-chlorophenyl, 2-bromophenyl, 3-bromophenyl, 4-bromophenyl, 3-hydroxyphenyl, 2,6-dimethylphenyl, 2,6-diethylphenyl, 2,6-dimethoxyphenyl, 3-nitrophenyl, 2,6-difluorophenyl, 2,6-dibromophenyl, 3-chloro-2-methylphenyl, 2,3-dichlorophenyl, 4-hydroxyphenyl, 2,4,6-trimethylphenyl, 2,4,6-trimethoxyphenyl, and 2,4,6-trifluorophenyl, preferably the group consisting of 2-methylphenyl, 4-fluorophenyl, 2,6-dibromophenyl, 2,6-diethylphenyl, and 2,6-dimethylphenyl, more preferably 2,6-dimethylphenyl.

In an embodiment, in the compound represented by formula (I) or the stereoisomer or solvate thereof provided in the present application, $R_2$ is selected from the group consisting of $C_{1-8}$ alkyl and $C_{3-8}$ cycloalkyl; wherein the $C_{1-8}$ alkyl or $C_{3-8}$ cycloalkyl is optionally substituted by a group selected from the group consisting of $C_{1-6}$ alkyl, $C_{1-6}$ alkoxy, $C_{1-6}$ haloalkyl, $C_{3-6}$ cycloalkyl, halogen, hydroxyl, cyano, amino, and a combination thereof.

In an embodiment, in the compound represented by formula (I) or the stereoisomer or solvate thereof provided in the present application, $R_2$ is selected from the group consisting of methyl, ethyl, n-propyl, isopropyl, n-butyl, sec-butyl, isobutyl, tert-butyl, n-pentyl, isopentyl, n-hexyl, cyclopropyl, cyclobutyl, cyclopropylmethyl, cyclobutylmethyl, cyclopentylmethyl, n-octyl, and n-heptyl, preferably the group consisting of ethyl, n-propyl, n-butyl, n-pentyl, n-hexyl, n-octyl, and n-heptyl, more preferably n-butyl.

In an embodiment, in the compound represented by formula (I) or the stereoisomer or solvate thereof provided in the present application, $R_2$ is selected from the group consisting of methyl, ethyl, n-propyl, isopropyl, n-butyl, sec-butyl, isobutyl, tert-butyl, n-pentyl, isopentyl, 4-methylpentyl, n-hexyl, cyclopropyl, cyclobutyl, cyclopropylmethyl, cyclobutylmethyl, cyclopentylmethyl, n-octyl, and n-heptyl, preferably the group consisting of ethyl, n-propyl, n-butyl, isobutyl, n-pentyl, 4-methylpentyl, n-hexyl, n-octyl, and n-heptyl; more preferably the group consisting of n-butyl, isobutyl, and 4-methylpentyl, particularly preferably n-butyl.

In an embodiment, in the compound represented by formula (I) or the stereoisomer or solvate thereof provided in the present application, $R_3$ is selected from the group consisting of aryl, heteroaryl, and three-membered to eight-membered heterocycloalkyl, alternatively, $R_3$, together with $X_2$, form condensed azacycloalkyl; wherein the aryl, the heteroaryl, the three-membered to eight-membered heterocycloalkyl, or the condensed azacycloalkyl is optionally substituted by a group selected from the group consisting of $C_{1-6}$ alkyl, $C_{1-6}$ alkoxy, $C_{1-6}$ alkoxy $C_{1-4}$ alkyl, $C_{1-6}$ haloalkyl, $C_{1-6}$ haloalkoxy, $C_{3-6}$ cycloalkyl, halogen, hydroxyl, cyano, amino, ester, nitro, sulfonyl, thiol, mono-$C_{1-6}$ alkylamino, di-$C_{1-6}$ alkylamino, $C_{2-4}$ alkenyl, $C_{2-4}$ alkynyl, optionally substituted phenyl, and a combination thereof; wherein the optionally substituted phenyl refers to unsubstituted phenyl, or is substituted by a group selected from the group consisting of $C_{1-6}$ alkyl, $C_{1-6}$ alkoxy, $C_{1-6}$ haloalkyl, $C_{1-6}$ haloalkoxy, $C_{3-6}$ cycloalkyl, halogen, hydroxy, cyano, amino, ester, nitro, mono-$C_{1-6}$ alkylamino, di-$C_{1-6}$ alkylamino, $C_{2-4}$ alkenyl, $C_{2-4}$ alkynyl, and a combination thereof; the condensed azacycloalkyl is condensed with phenyl or heteroaryl.

In an embodiment, in the compound represented by formula (I) or the stereoisomer or solvate thereof provided in the present application, $R_3$ is selected from the group consisting of aryl, heteroaryl, and three-membered to eight-membered heterocycloalkyl, alternatively, $R_3$, together with $X_2$, form condensed azacycloalkyl; wherein the aryl, the heteroaryl, the three-membered to eight-membered heterocycloalkyl, or the condensed azacycloalkyl is optionally substituted by a group selected from the group consisting of $C_{1-6}$ alkyl, $C_{1-6}$ alkoxy, $C_{1-6}$ alkoxy $C_{1-4}$ alkyl, $C_{1-6}$ haloalkyl, $C_{1-6}$ haloalkoxy, $C_{3-6}$ cycloalkyl, halogen, hydroxyl, cyano, amino, $C_{1-6}$ alkanoyl, ester, nitro, sulfonyl, thiol, mono-$C_{1-6}$ alkylamino, di-$C_{1-6}$ alkylamino, $C_{2-4}$ alkenyl, $C_{2-4}$ alkynyl, optionally substituted phenyl, and a combination thereof; optionally, adjacent substituents, together with the atoms they are attached to, form a ring; wherein the optionally substituted phenyl refers to unsubstituted phenyl, or is substituted by a group selected from the group consisting of $C_{1-6}$ alkyl, $C_{1-6}$ alkoxy, $C_{1-6}$ haloalkyl, $C_{1-6}$ haloalkoxy, $C_{3-6}$ cycloalkyl, halogen, hydroxy, cyano, amino, ester, nitro, mono-$C_{1-6}$ alkylamino, di-$C_{1-6}$ alkylamino, $C_{2-4}$ alkenyl, $C_{2-4}$ alkynyl, and a combination thereof; the condensed azacycloalkyl is condensed with phenyl or heteroaryl.

In an embodiment, in the compound represented by formula (I) or the stereoisomer or solvate thereof provided in the present disclosure, $R_3$ is selected from the group consisting of phenyl, 4-fluorobenzyl, 2-methylphenyl, 2-methoxyphenyl, 2-fluorophenyl, 2-chlorophenyl, 2-bromophenyl, 2-hydroxyphenyl, 3-methylphenyl, 3-methoxyphenyl, 3-fluorophenyl, 3-chlorophenyl, 3-bromophenyl, 3-hydroxyphenyl, 4-methylphenyl, 4-methoxyphenyl, 4-fluorophenyl, 4-chlorophenyl, 4-bromophenyl, 4-hydroxyphenyl, 4-trifluoromethylphenyl, 2,4-dimethylphenyl, 2,4-dimethoxyphenyl, 2,4-difluorophenyl, 2,4-dichlorophenyl, 2,4-dihydroxyphenyl, 2,6-dimethylphenyl, 2,6-dimethoxyphenyl, 2,6-difluorophenyl, 2,6-dichlorophenyl, 2,6-dihydroxyphenyl, 2,4,6-trimethylphenyl, 2,4,6-trimethoxyphenyl, 2,4,6-trifluorophenyl, 2-amino-5-fluorophenyl, 2-isopropyl-5-methylphenyl, 4-cyanophenyl, 4-ethoxycarbonylphenyl, 2,6-di-tert-butyl-4-methylphenyl, 4-nitrophenyl, and 4-sulfonylphenyl, alternatively, $R_3$, together with $X_2$, form condensed piperidinyl, condensed piperazinyl, or condensed pyrrolidinyl, wherein the condensed piperidinyl, the condensed piperazinyl, or the condensed

pyrrolidinyl is condensed with a group selected from the group consisting of phenyl, thienyl, furanyl, pyrrolyl, thiazolyl, isothiazolyl, imidazolyl, triazolyl, pyridinyl, and pyrimidinyl; and the condensed piperidinyl, the condensed piperazinyl or the condensed pyrrolidinyl is optionally substituted by a group selected from the group consisting of methyl, ethyl, n-propyl, isopropyl, n-butyl, sec-butyl, tert-butyl, fluorine, chlorine, bromine, iodine, hydroxyl, amino, nitro, carbomethoxy, carboethoxy, phenyl, substituted phenyl, and a combination thereof; preferably, $R_3$ is selected from the group consisting of phenyl, 2-methylphenyl, 3-methylphenyl, 4-methylphenyl, 4-fluorophenyl, 2-amino-5-fluorophenyl, 2-isopropyl-5-methylphenyl, 4-cyanophenyl, 4-ethoxycarbonylphenyl, 2,6-di-tert-butyl-4-methylphenyl, 4-nitrophenyl, 4-sulfonylphenyl, and 4-hydroxyphenyl; alternatively, $R_3$, together with $X_2$, form imidazopiperazinyl, thienopiperazinyl, or pyrrolopiperazinyl; optionally, the imidazopiperazinyl, thienopiperazinyl, or pyrrolopiperazinyl is substituted by a group selected from the group consisting of methyl, ethyl, n-propyl, isopropyl, n-butyl, sec-butyl, tert-butyl, fluorine, chlorine, bromine, iodine, hydroxyl, amino, nitro, carbomethoxy, carboethoxy, phenyl, and a combination thereof.

In an embodiment, in the compound represented by formula (I) or the stereoisomer or solvate thereof provided in the present disclosure, $R_3$ is selected from the group consisting of phenyl, 4-fluorobenzyl, 2-methylphenyl, 2-methoxyphenyl, 2-fluorophenyl, 2-chlorophenyl, 2-bromophenyl, 2-hydroxyphenyl, 3-methylphenyl, 3-methoxyphenyl, 3-fluorophenyl, 3-chlorophenyl, 3-bromophenyl, 3-hydroxyphenyl, 3-cyanophenyl, 3-trifluoromethylphenyl, 4-methylphenyl, 4-methoxyphenyl, 4-fluorophenyl, 4-chlorophenyl, 4-bromophenyl, 4-hydroxyphenyl, 4-trifluoromethylphenyl, 2,4-dimethylphenyl, 2,4-dimethoxyphenyl, 2,4-difluorophenyl, 2,4-dichlorophenyl, 2,4-dihydroxyphenyl, 3,4-difluorophenyl, 3,4-dichlorophenyl, 3,5-difluorophenyl, 2,6-dimethylphenyl, 2,6-dimethoxyphenyl, 2,6-difluorophenyl, 2,6-dichlorophenyl, 2,6-dihydroxyphenyl, 2,4,6-trimethylphenyl, 2,4,6-trimethoxyphenyl, 2,4,6-trifluorophenyl, 2-amino-5-fluorophenyl, 2-isopropyl-5-methylphenyl, 4-cyanophenyl, 4-ethoxycarbonylphenyl, 2,6-di-tert-butyl-4-methylphenyl, 4-nitrophenyl, 4-sulfonylphenyl, 2,2-dimethyl-2,3-dihydrobenzofuran-7-yl, naphthyl, and 6-acetylnaphth-2-yl; alternatively, $R_3$, together with $X_2$, form condensed piperidinyl, condensed piperazinyl, or condensed pyrrolidinyl, wherein the condensed piperidinyl, the condensed piperazinyl, or the condensed pyrrolidinyl is condensed with a group selected from the group consisting of phenyl, thienyl, furanyl, pyrrolyl, thiazolyl, isothiazolyl, imidazolyl, triazolyl, pyridinyl, and pyrimidinyl; and the condensed piperidinyl, the condensed piperazinyl or the condensed pyrrolidinyl is optionally substituted by a group selected from the group consisting of methyl, ethyl, n-propyl, isopropyl, n-butyl, sec-butyl, tert-butyl, fluorine, chlorine, bromine, iodine, hydroxyl, amino, nitro, carbomethoxy, carboethoxy, phenyl, substituted phenyl, and a combination thereof; preferably, $R_3$ is selected from the group consisting of phenyl, 2-methylphenyl, 3-methylphenyl, 4-methylphenyl, 4-fluorophenyl, 3-cyanophenyl, 3-trifluoromethylphenyl, 3,4-difluorophenyl, 3,4-dichlorophenyl, 3,5-difluorophenyl, 2-amino-5-fluorophenyl, 2-isopropyl-5-methylphenyl, 4-cyanophenyl, 4-ethoxycarbonylphenyl, 2,6-di-tert-butyl-4-methylphenyl, 4-nitrophenyl, 4-sulfonylphenyl, 4-hydroxyphenyl, 2,2-dimethyl-2,3-dihydrobenzofuran-7-yl, and 6-acetylnaphth-2-yl; alternatively, $R_3$, together with $X_2$, form imidazopiperazinyl, thienopiperazinyl, or pyrrolopiperazinyl; optionally, the imidazopiperazinyl, thienopiperazinyl, or pyrrolopiperazinyl is substituted by a group selected from the group consisting of methyl, ethyl, n-propyl, isopropyl, n-butyl, sec-butyl, tert-butyl, fluorine, chlorine, bromine, iodine, hydroxyl, amino, nitro, carbomethoxy, carboethoxy, phenyl, and a combination thereof.

In an embodiment, in the compound represented by formula (I) or the stereoisomer or solvate thereof provided in the present disclosure, L is selected from $C_{3-6}$ alkylene; wherein the $C_{3-6}$ alkylene is optionally substituted by a group selected from the group consisting of $C_{1-4}$ alkyl, $C_{1-4}$ alkoxy, $C_{1-4}$ haloalkyl, $C_{1-4}$ haloalkoxy, $C_{3-6}$ cycloalkyl, halogen, hydroxyl, cyano, amino, ester, nitro, mono-$C_{1-4}$ alkylamino, di-$C_{1-4}$ alkylamino, $C_{2-4}$ alkenyl, $C_{2-4}$ alkynyl, and a combination thereof.

In an embodiment, in the compound represented by formula (I) or the stereoisomer or solvate thereof provided in the present disclosure, L is selected from $C_{3-6}$ alkylene, with the provision that L is not $C_3$ alkylene; wherein the $C_{3-6}$ alkylene is optionally substituted by a group selected from the group consisting of $C_{1-4}$ alkyl, $C_{1-4}$ alkoxy, $C_{1-4}$ haloalkyl, $C_{1-4}$ haloalkoxy, $C_{3-6}$ cycloalkyl, halogen, hydroxyl, cyano, amino, ester, nitro, mono-$C_{1-4}$ alkylamino, di-$C_{1-4}$ alkylamino, $C_{2-4}$ alkenyl, $C_{2-4}$ alkynyl, and a combination thereof.

In an embodiment, in the compound represented by formula (I) or the stereoisomer or solvate thereof provided in the present disclosure, L is selected from the group consisting of $-(CH_2)_3-$, $-(CH_2)_4-$, $-(CH_2)_5-$, $-(CH_2)_6-$, and $-(CH_2)_2CHCH_3(CH_2)_2-$, preferably $-(CH_2)_4-$ or $-(CH_2)_5-$.

In an embodiment, in the compound represented by formula (I) or the stereoisomer or solvate thereof provided in the present disclosure, L is selected from the group consisting of $-(CH_2)_4-$, $-(CH_2)_5-$, $-(CH_2)_6-$, and $-(CH_2)_2CHCH_3(CH_2)_2-$, preferably $-(CH_2)_4-$ or $-(CH_2)_5-$.

In an embodiment, in the compound represented by formula (I) or the stereoisomer or solvate thereof provided in the present disclosure, $X_1$ is selected from the group consisting of O, NH, $NCH_3$, $NCH_2CH_3$, and $N(CH_2)_2CH_3$, preferably, $X_1$ is NH.

In an embodiment, in the compound represented by formula (I) or the stereoisomer or solvate thereof provided in the present disclosure, $X_2$ is selected from the group consisting of O, NH, $NCH_3$, $NCH_2CH_3$, and $N(CH_2)_2CH_3$, alternatively, $X_2$, together with $R_3$, form condensed piperidinyl, condensed piperazinyl or condensed pyrrolidinyl, wherein the condensed piperidinyl, the condensed piperazinyl or the condensed pyrrolidinyl is condensed with a group selected

from the group consisting of phenyl, thienyl, furanyl, pyrrolyl, thiazolyl, isothiazolyl, imidazolyl, triazolyl, pyridinyl, and pyrimidinyl; and the condensed piperidinyl, the condensed piperazinyl or the condensed pyrrolidinyl is optionally substituted by a group selected from the group consisting of methyl, ethyl, n-propyl, isopropyl, n-butyl, sec-butyl, tert-butyl, fluorine, chlorine, bromine, iodine, hydroxyl, amino, nitro, carbomethoxy, carboethoxy, phenyl, substituted phenyl, and a combination thereof; preferably, $X_2$ is O or NH, alternatively, $X_2$, together with $R_3$, form imidazopiperazinyl, thienopiperazinyl, or pyrrolopiperazinyl; optionally, the imidazopiperazinyl, thienopiperazinyl, or pyrrolopiperazinyl is substituted by a group selected from the group consisting of methyl, ethyl, n-propyl, isopropyl, n-butyl, sec-butyl, tert-butyl, fluorine, chlorine, bromine, iodine, hydroxyl, amino, nitro, carbomethoxy, carboethoxy, phenyl, and a combination thereof.

In an embodiment, in the compound represented by formula (I) or the stereoisomer or solvate thereof provided in the present disclosure, when one of $S_1$ and $S_2$ is a bond, the other is selected from the group consisting of $-(CH_2)_2-$, $-(CH_2)_3-$, $-(CH_2)_4-$, $-(CH_2)_5-$, and $-(CH_2)_6-$.

In an embodiment, in the compound represented by formula (I) or the stereoisomer or solvate thereof provided in the present disclosure, $Y^-$ is selected from the group consisting of a halogen anion, sulfate, acetate, tartrate, p-toluenesulfonate, methanesulfonate, and citrate, preferably the group consisting of $Cl^-$, $Br^-$, and $CH_3COO^-$; more preferably $Br^-$.

In an embodiment, in the compound represented by formula (I) or the stereoisomer or solvate thereof provided in the present disclosure, the compound has the structure characterized by formula (IV):

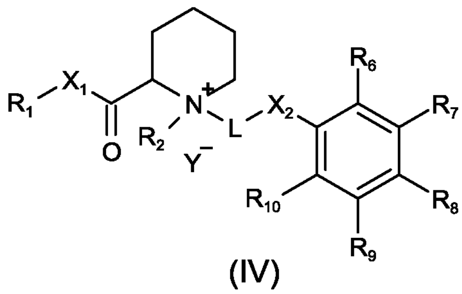

**(IV)**

wherein $X_1$ is $NR_4^A$, wherein $R_4^A$ is selected from the group consisting of hydrogen, deuterium, $C_{1-8}$ alkyl, $C_{3-8}$ cycloalkyl, and $C_{1-4}$ alkoxy $C_{1-4}$ alkyl, preferably $X_1$ is NH;

$X_2$ is selected from the group consisting of O, S and $NR_4^B$, wherein $R_4^B$ is selected from the group consisting of hydrogen, deuterium, $C_{1-8}$ alkyl, $C_{3-8}$ cycloalkyl, and $C_{1-4}$ alkoxy $C_{1-4}$ alkyl; preferably $X_2$ is selected from the group consisting of O, NH, $NCH_3$, $NCH_2CH_3$, and $N(CH_2)_2CH_3$, more preferably $X_2$ is O or NH;

$R_6$, $R_7$, $R_8$, $R_9$, and $R_{10}$ are each independently selected from the group consisting of hydrogen, fluorine, chlorine, bromine, iodine, hydroxyl, cyano, nitro, amino, ester, $C_{1-4}$ alkyl, $C_{1-4}$ alkoxy, and $C_{1-4}$ alkoxy$C_{1-4}$ alkyl;

$R_1$, $R_2$, L and $Y^-$ are as defined above in formula (I).

In an embodiment, in the compound represented by formula (I) or the stereoisomer or solvate thereof provided in the present disclosure, $R_6$, $R_7$, $R_8$, $R_9$, and $R_{10}$ are each independently selected from the group consisting of hydrogen, fluorine, chlorine, bromine, iodine, hydroxyl, cyano, nitro, amino, ester, $C_{1-4}$ alkyl, $C_{1-4}$ alkoxy, and $C_{1-4}$ alkoxy$C_{1-4}$ alkyl; optionally, adjacent substituents, together with the atoms they are attached to, form a ring.

In an embodiment, in the compound represented by formula (I) or the stereoisomer or solvate thereof provided in the present disclosure, the compound has the structure characterized by formula (V):

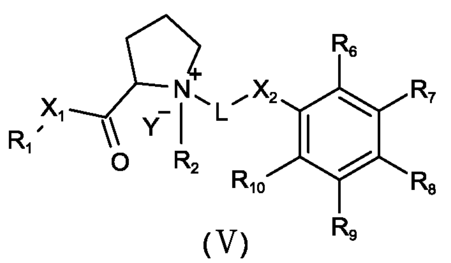

(V)

wherein $X_1$ is $NR_4^A$, wherein $R_4^A$ is selected from the group consisting of hydrogen, deuterium, $C_{1-8}$ alkyl, $C_{3-8}$ cycloalkyl, and $C_{1-4}$ alkoxy $C_{1-4}$ alkyl, preferably $X_1$ is NH;

$X_2$ is selected from the group consisting of O, S and $NR_4^B$, wherein $R_4^B$ is selected from the group consisting of hydrogen, deuterium, $C_{1-8}$ alkyl, $C_{3-8}$ cycloalkyl, and $C_{1-4}$ alkoxy $C_{1-4}$ alkyl, preferably $X_2$ is selected from the group consisting of O, NH, $NCH_3$, $NCH_2CH_3$, and $N(CH_2)_2CH_3$, more preferably $X_2$ is O or NH;

$R_6$, $R_7$, $R_8$, $R_9$, and $R_{10}$ are each independently selected from the group consisting of hydrogen, fluorine, chlorine, bromine, iodine, hydroxyl, cyano, nitro, amino, ester, $C_{1-4}$ alkyl, $C_{1-4}$ alkoxy, and $C_{1-4}$ alkoxy $C_{1-4}$ alkyl;

$R_1$, $R_2$, L and $Y^-$ are as defined above in formula (I).

In an embodiment, in the compound represented by formula (I) or the stereoisomer or solvate thereof provided in the present disclosure, the compound has the structure characterized by formula (V); $R_6$, $R_7$, $R_8$, $R_9$, and $R_{10}$ are each independently selected from the group consisting of hydrogen, fluorine, chlorine, bromine, iodine, hydroxyl, cyano, nitro, amino, ester, $C_{1-4}$ alkyl, $C_{1-4}$ alkoxy, and $C_{1-4}$ alkoxy $C_{1-4}$ alkyl; optionally, adjacent substituents, together with the atoms they are attached to, form a ring.

In an embodiment, in the compound represented by formula (I) or the stereoisomer or solvate thereof provided in the present disclosure, the compound has the structure characterized by formula (VI):

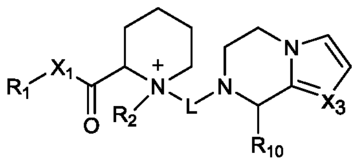

(VI)

wherein $X_1$ is $NR_4^A$, wherein $R_4^A$ is selected from the group consisting of hydrogen, deuterium, $C_{1-8}$ alkyl, $C_{3-8}$ cycloalkyl, and $C_{1-4}$ alkoxy $C_{1-4}$ alkyl, preferably $X_1$ is NH;

$X_3$ is C or N;

$R_{10}$ is selected from the group consisting of hydrogen, $C_{1-4}$ alkyl, $C_{1-4}$ alkoxy, $C_{1-4}$ alkoxy $C_{1-4}$ alkyl, and aryl, wherein the aryl is optionally substituted by a group selected from the group consisting of fluorine, chlorine, bromine, iodine, hydroxyl, cyano, nitro, amino, ester, $C_{1-4}$ alkyl, $C_{1-4}$ alkoxy, and $C_{1-4}$ alkoxy $C_{1-4}$ alkyl;

$R_1$, $R_2$, L and $Y^-$ are as defined above in formula (I).

In an embodiment, in the compound represented by formula (I) or the stereoisomer or solvate thereof provided in the present disclosure, the compound has the structure characterized by formula (VII):

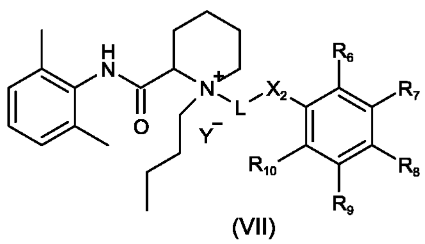

(VII)

wherein $X_2$ is selected from the group consisting of O, S and $NR_4^B$, wherein $R_4^B$ is selected from the group consisting of hydrogen, deuterium, $C_{1-8}$ alkyl, $C_{3-8}$ cycloalkyl, and $C_{1-4}$ alkoxy $C_{1-4}$ alkyl, preferably $X_2$ is selected from the group consisting of O, NH, $NCH_3$, $NCH_2CH_3$, and $N(CH_2)_2CH_3$, more preferably $X_2$ is O or NH;

$R_6$, $R_7$, $R_8$, $R_9$, and $R_{10}$ are each independently selected from the group consisting of hydrogen, fluorine, chlorine, bromine, iodine, hydroxyl, cyano, nitro, amino, ester, $C_{1-4}$ alkyl, $C_{1-4}$ alkoxy, and $C_{1-4}$ alkoxy $C_{1-4}$ alkyl;

L and $Y^-$ are as defined above in formula (I).

In an embodiment, in the compound represented by formula (I) or the stereoisomer or solvate thereof provided in the present disclosure, the compound has the structure characterized by formula (VII), wherein $R_6$, $R_7$, $R_8$, $R_9$, and $R_{10}$ are each independently selected from the group consisting of hydrogen, fluorine, chlorine, bromine, iodine, hydroxyl, cyano, nitro, amino, ester, $C_{1-4}$ alkyl, $C_{1-4}$ alkoxy, and $C_{1-4}$ alkoxy $C_{1-4}$ alkyl; optionally, adjacent substituents, together with the atoms they are attached to, form a ring.

In a specific embodiment, the compound is selected from the group consisting of the following structures:

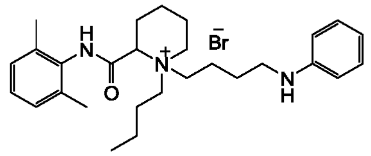 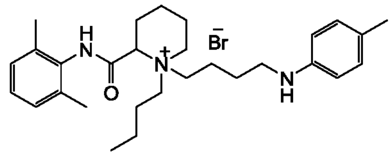 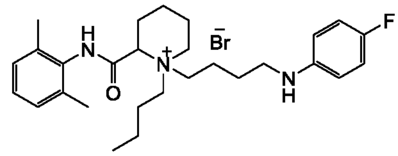

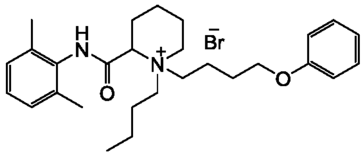 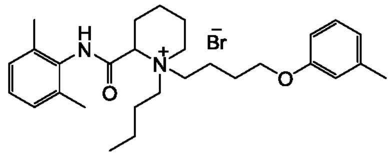 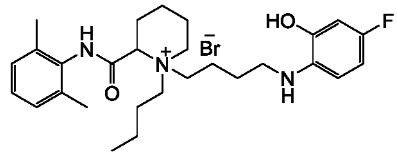

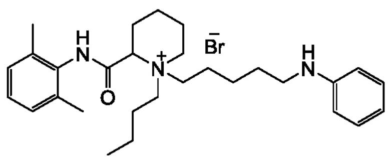 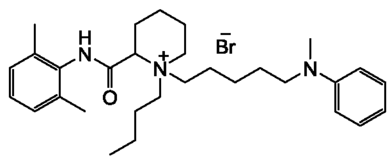 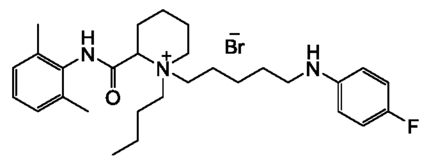

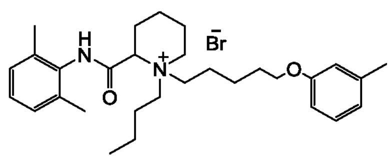 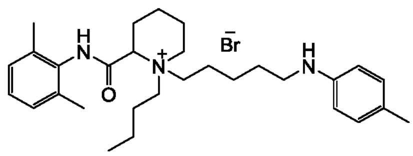 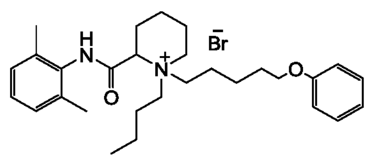

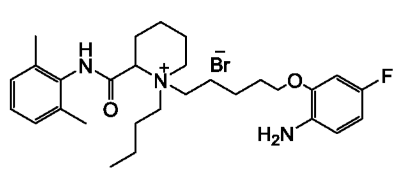 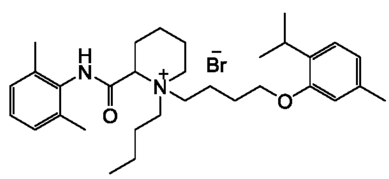 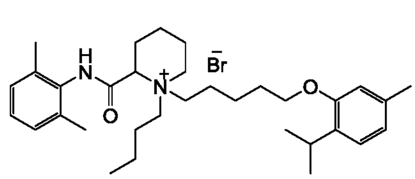

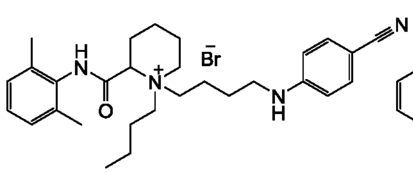 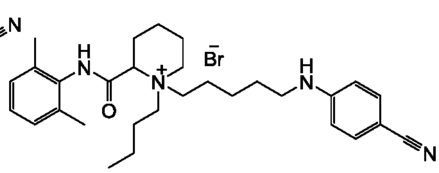 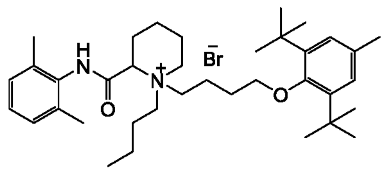

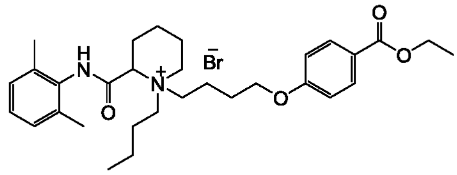
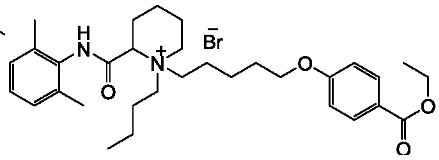
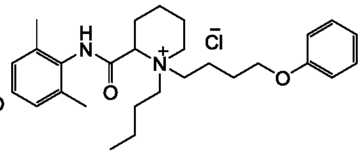

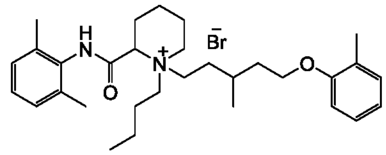
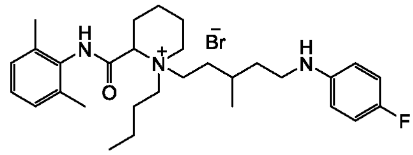
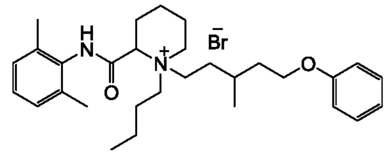

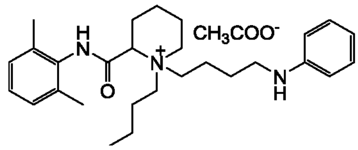
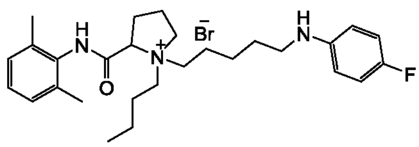
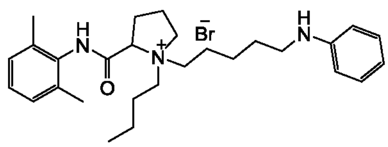

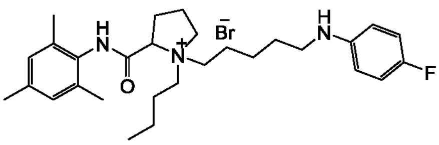
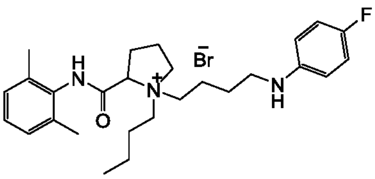
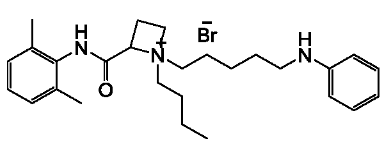

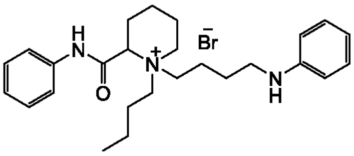
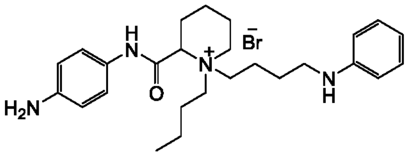
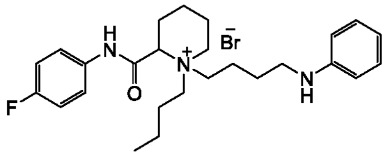

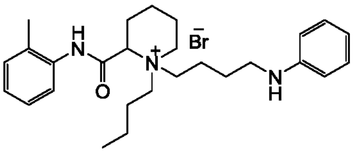
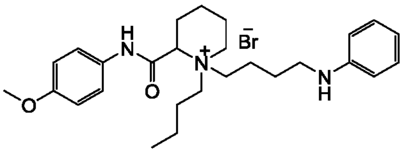
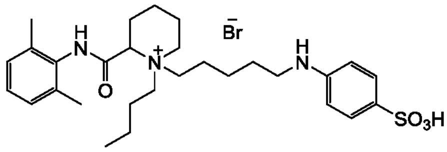

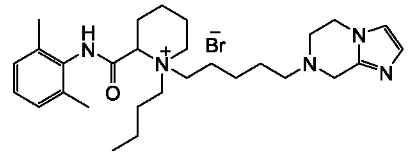
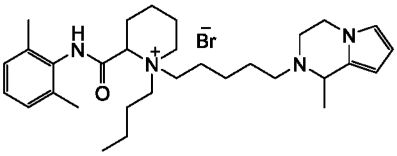
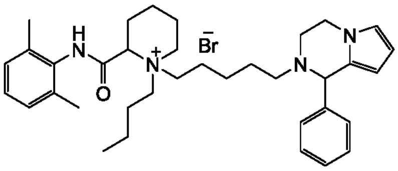

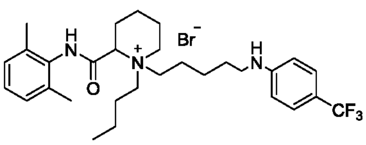
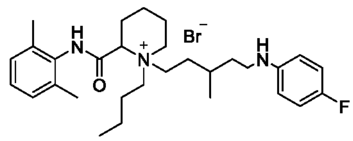
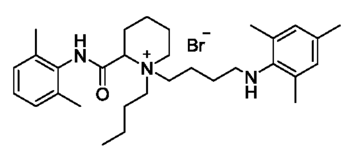

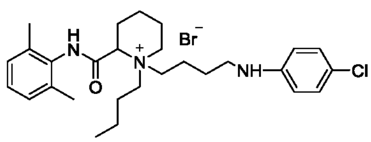
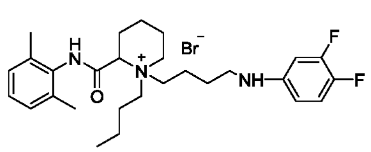
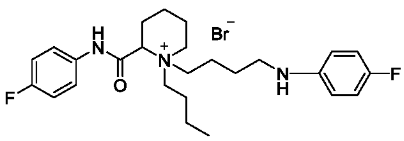

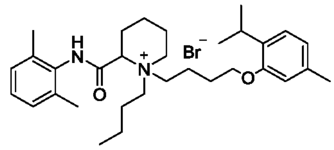
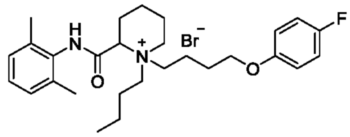
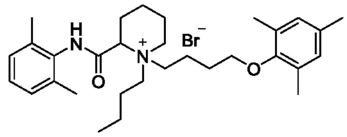

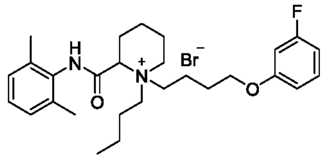
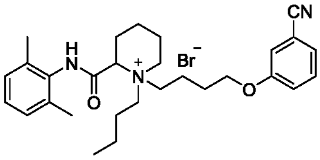
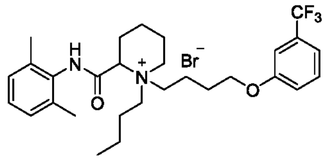

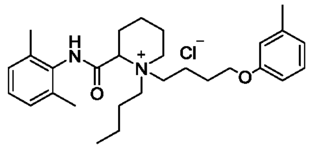
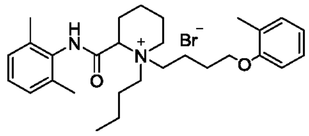
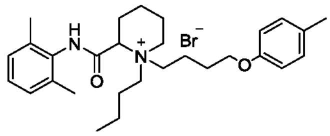

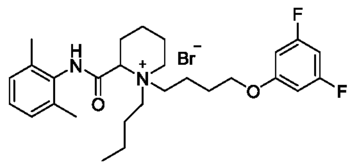
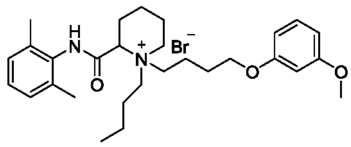

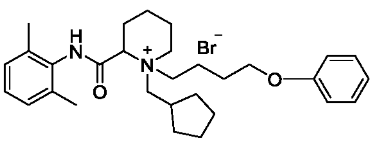
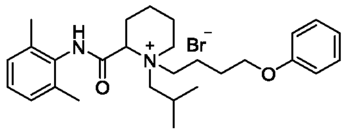
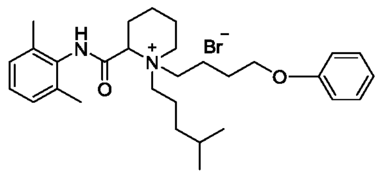

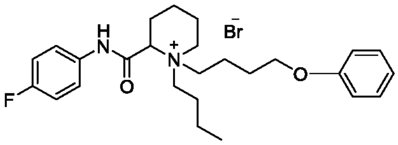
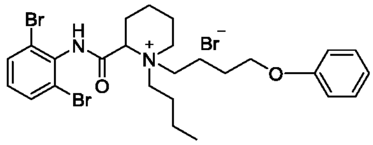
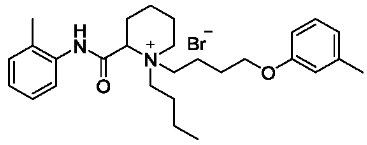

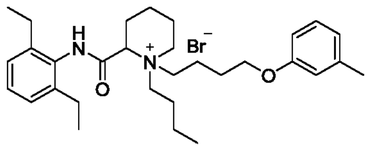
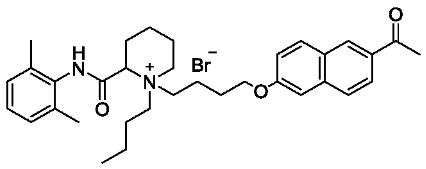
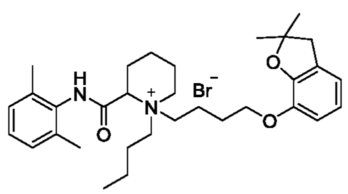

In another specific embodiment, the compound is selected from the group consisting of the following structures:

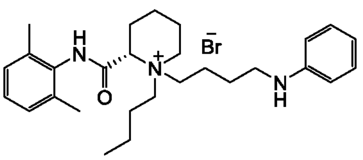
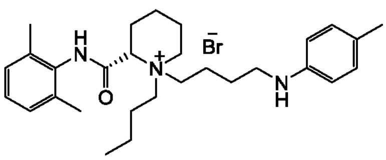
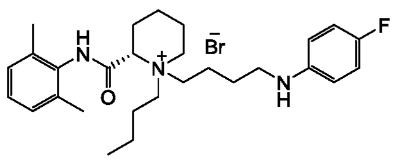

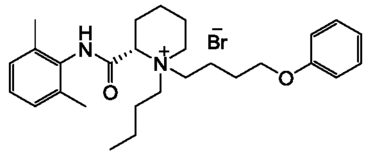
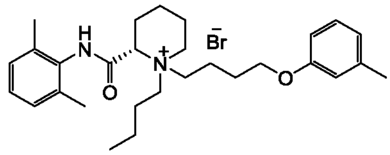
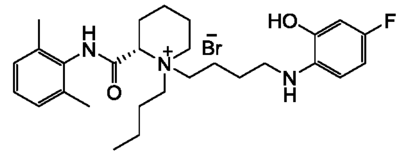

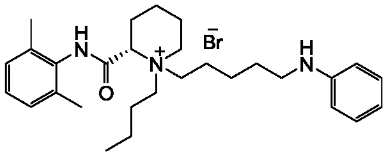
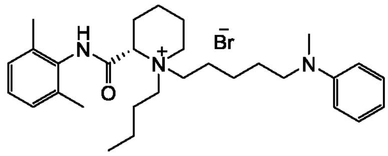
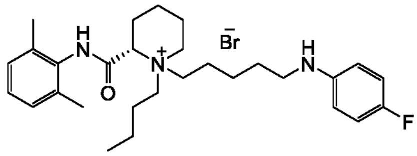

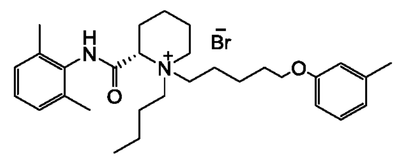
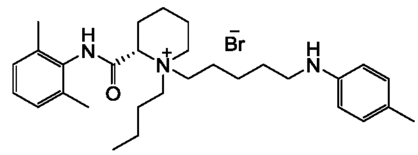
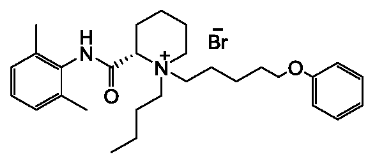

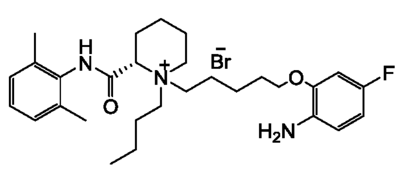
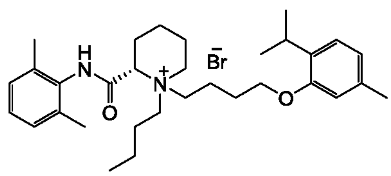
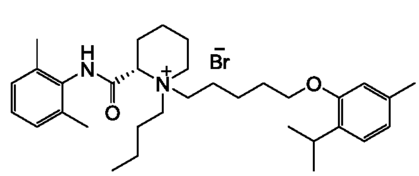

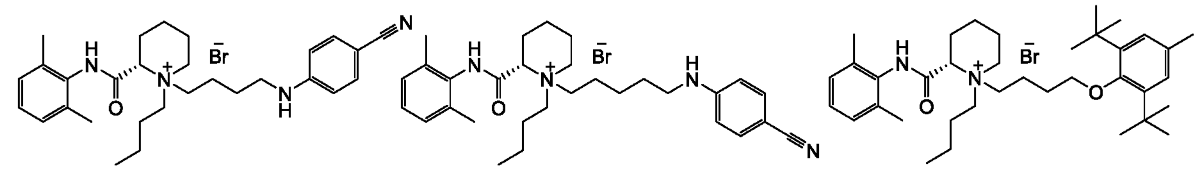

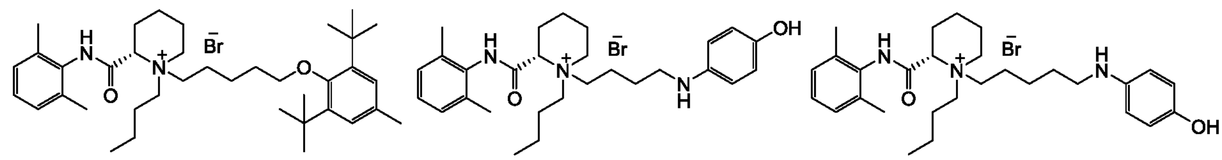

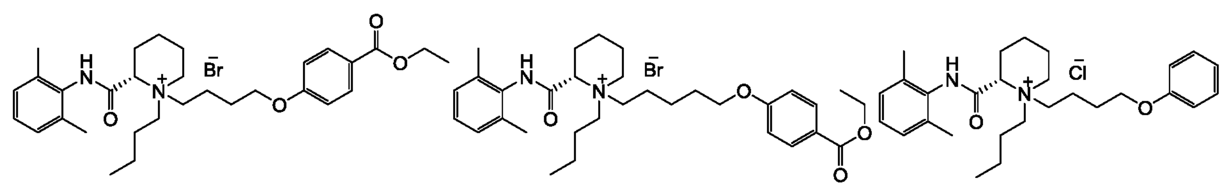

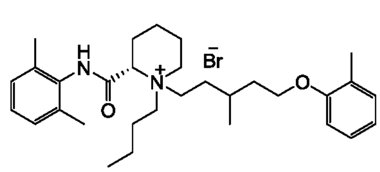
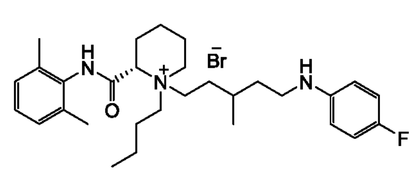
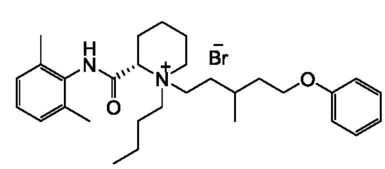

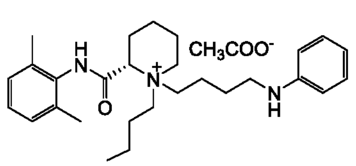
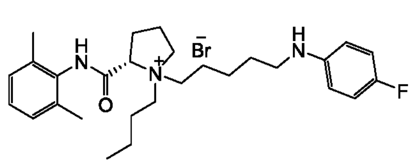
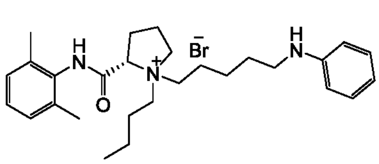

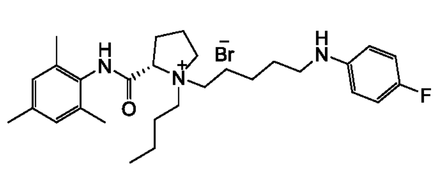
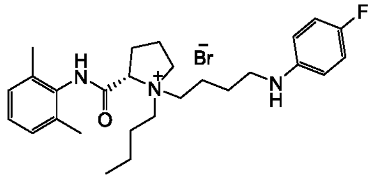
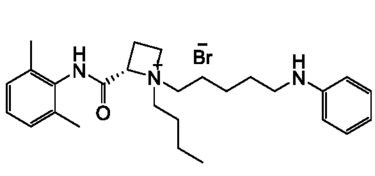

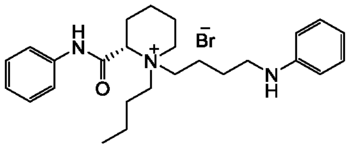
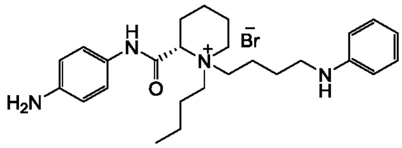
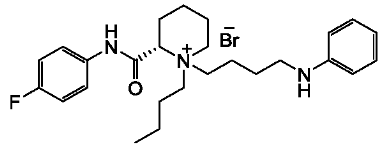

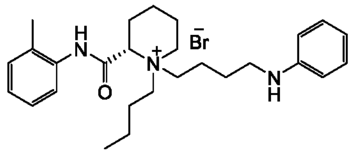
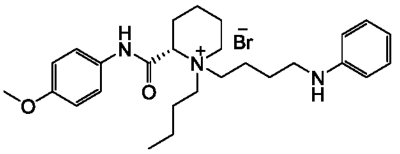
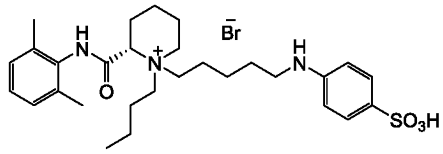

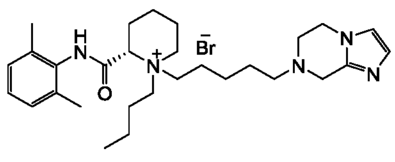
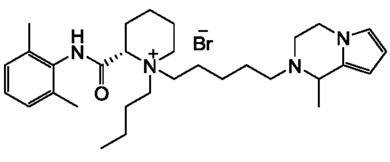
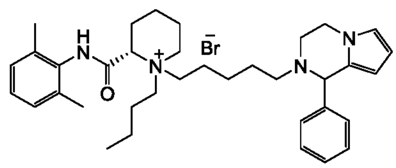

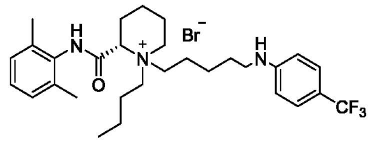
**17**

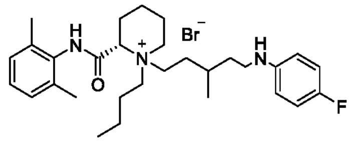
**18**

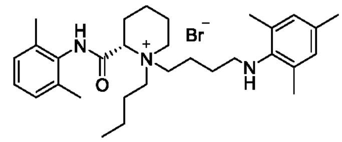
**19**

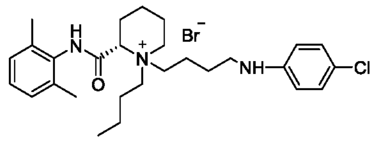
**20**

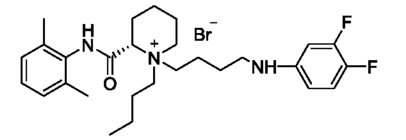
**21**

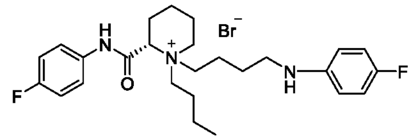
**22**

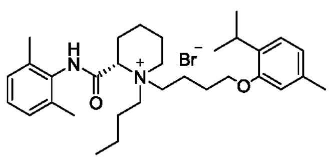
**23**

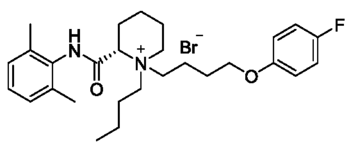
**24**

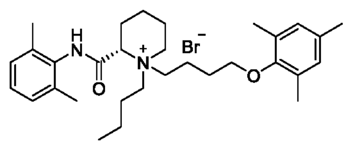
**25**

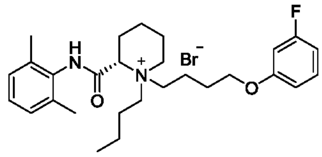
**26**

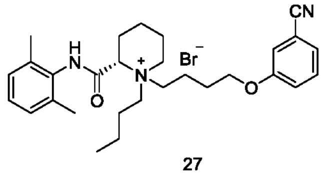
**27**

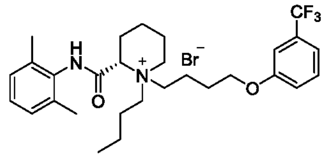
**28**

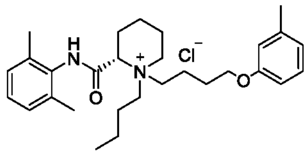
29

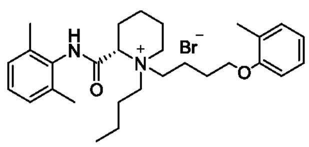
30

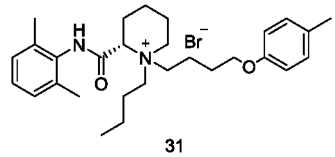
31

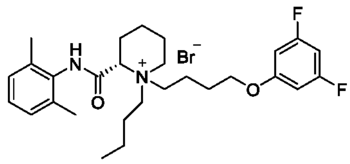
32

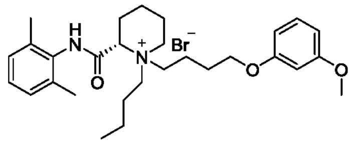
33

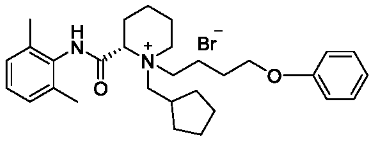
36

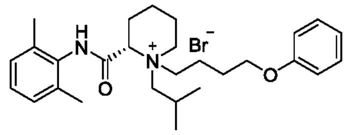
37

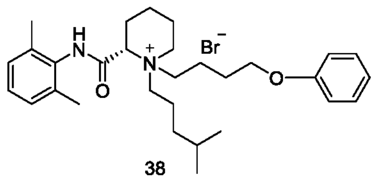
38

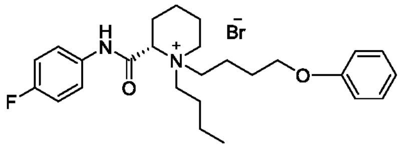
39

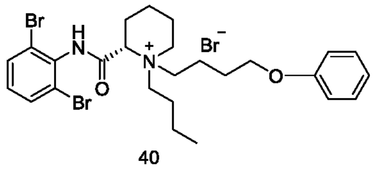
40

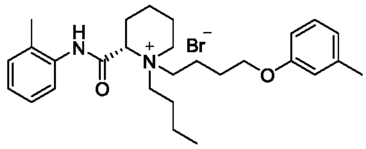
41

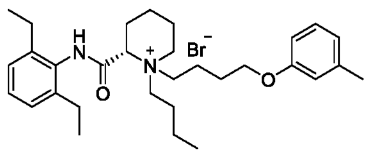
42

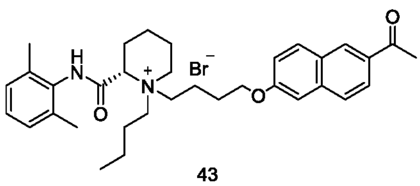
43

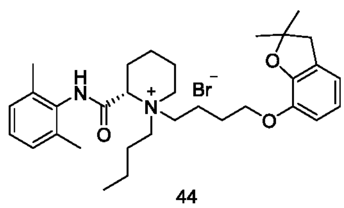
44

The second aspect of the present application involves a method for producing the compound represented by general formula (I) or the stereoisomer or solvate thereof as described in any one of the first aspect of the present application, comprising the following steps:

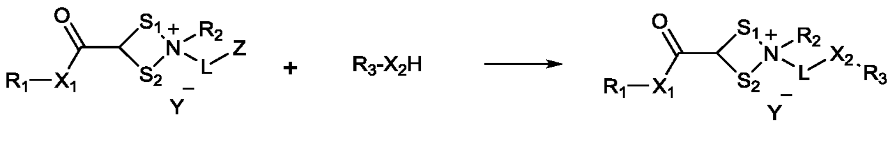

(II) (III) (I)

reacting the compound represented by formula (II) with the compound represented by formula (III) to obtain the compound represented by formula (I);

wherein, Z in formula (II) is an electron-withdrawing leaving group such as bromine, chloride or sulfonate, and the groups in formula (II) and formula (III) are defined as in formula (I).

In the method of the present application, formula (II) can be produced by the following method:

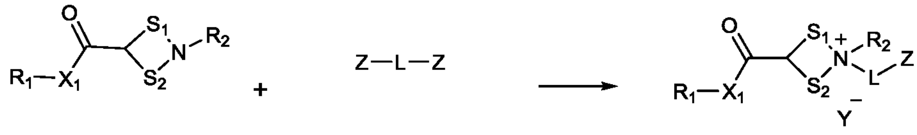

(II-1) (II-2) (II)

wherein, the groups in formula (II-1) and formula (II-2) are defined as above.

The third aspect of the present application involves a pharmaceutical composition, comprising the compound represented by general formula (I) or the stereoisomer or solvate thereof as described in any one of the first aspect of the present application and a pharmaceutically acceptable carrier, excipient or diluent.

The fourth aspect of the present application involves use of the compound represented by general formula (I) or the stereoisomer or solvate thereof as described in any one of the first aspect of the present application or the pharmaceutical composition in the third aspect of the present application in the manufacture of a medicament for local anesthesia or analgesia.

According to the use described in any one of the fourth aspect of the present application, the local anesthesia is selected from the group consisting of conduction anesthesia, surface anesthesia, and infiltration anesthesia; and the analgesia is suitable for pain selected from the group consisting of chronic pain, acute pain, inflammatory pain, cancer pain, neuropathic pain, musculoskeletal pain, primary pain, intestinal pain, and idiopathic pain.

According to the use described in any one of the fourth aspect of the present application, the medicament is administered topically via transdermal, subcutaneous, intradermal, intramuscular, near nerves, intrapulpal, intraspinal, epidural, intravenous, or mucosal routes such as eye drops.

The fifth aspect of the present application involves a method of local anesthesia or analgesia, comprising administering to a subject in need thereof a therapeutically effective amount of the compound represented by general formula (I) or the stereoisomer or solvate thereof as described in any one of the first aspect of the present application or the pharmaceutical composition in the third aspect of the present application. The administration is topical administration via transdermal, subcutaneous, intradermal, intramuscular, near nerves, intrapulpal, intraspinal, epidural, intravenous, or mucosal routes such as eye drops.

Beneficial effects of the present application

The present application provides a cyclic quaternary ammonium salt compound with a novel structural type. Experimental results indicate that the compound of the present application offers a prolonged local anesthetic effect at a single administration, without causing motor nerve damage. It demonstrates excellent safety, rapid onset, and minimal skin irritation. The present application provides long-acting local anesthetics with novel structures to address the limitations of existing clinical options, such as short duration, tissue and neurotoxicity, and skin irritation. It represents a significant advancement in medical strategies for anesthesia and analgesia.

The terms used in the specification and claims of the present application are defined as follows. For any specific term, if its definition in this application differs from the generally accepted meaning understood by those skilled in the art, the definition provided in this application shall prevail. If a term is not explicitly defined in this application, it shall be understood according to its commonly accepted meaning in the art.

In the present application, the names of compounds correspond to their structural formulas. In cases where there is a discrepancy between the compound names and their structural formulas, the structural formulas shall prevail. Alternatively, it may be inferred based on the specific context of the present disclosure and the expertise of those skilled in the art.

The term "alkyl" as used herein refers to a linear or branched monovalent saturated hydrocarbon group.

The term "$C_{1-8}$ alkyl" refers to a linear or branched alkyl group with 1 to 8, i.e., 1, 2, 3, 4, 5, 6, 7 or 8 carbon atoms, typically methyl, ethyl, n-propyl, isopropyl, n-butyl, isobutyl, sec-butyl, tert-butyl, neopentyl, pentyl, hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, or n-octyl. Similarly, the term "$C_{1-4}$ alkyl" refers to a linear or branched alkyl group with 1, 2, 3 or 4 carbon atoms, including methyl, ethyl, n-propyl, isopropyl, n-butyl, isobutyl, sec-butyl and tert-butyl. The alkyl in the present application is preferably $C_{1-6}$ alkyl, more preferably $C_{1-4}$ alkyl.

The term "alkylene" as used herein refers to a saturated linear or branched aliphatic hydrocarbon group that has two residues derived from an alkane by removal of two hydrogen atoms from either the same carbon atom or different carbon atoms of the alkane. It is a linear or branched group containing 1 to 16 carbon atoms, preferably an alkylene group containing 1 to 6 carbon atoms. Non-limiting examples of "alkylene" include, but are not limited to, $-CH_2-$, $-(CH_2)_2-$,

$-(CH_2)_3-$, $-(CH_2)_4-$, $-(CH_2)_5-$, and $-(CH_2)_6-$.

The term "alkenylene" as used herein refers to a group formed by formally removing two hydrogen atoms from an alkene. Non-limiting examples of "alkenylene" include, but are not limited to,-CH=CH-, and

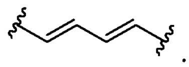

The term "alkynylene" as used herein refers to a group formed by formally removing two hydrogen atoms from an alkyne. Non-limiting examples of "alkynylene" include, but are not limited to

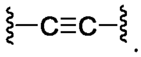

The term "alkoxy" as used herein refers to -O-(alkyl) and -O-(unsubstituted cycloalkyl), where "alkyl" is as defined above. Non-limiting examples of "alkoxy" include, but are not limited to, methoxy, ethoxy, propoxy, butoxy, cyclopropyloxy, cyclobutyloxy, cyclopentyloxy, and cyclohexyloxy.

The term "$C_{2-4}$ alkenyl" as used herein refers to an alkenyl group containing 2 to 4 carbon atoms, with either 1 or 2 carbon-carbon double bonds. When the alkenyl group has more than one double bond, these double bonds may be conjugated or non-conjugated. Non-limiting examples of "$C_{2-4}$ alkenyl" include, but are not limited to, vinyl and ethenyl.

The term "$C_{2-4}$ alkynyl" as used herein refers to an alkynyl group containing 2 to 4 carbon atoms, with either 1 or 2 carbon-carbon triple bonds. When the alkynyl group has more than one triple bond, these triple bonds may be conjugated or non-conjugated. A non-limiting example of "$C_{2-4}$ alkynyl" includes, but is not limited to, ethynyl.

The term "cycloalkyl" as used herein refers to a saturated carbocyclic group containing 3, 4, 5, 6, 7, 8, 9, or 10 carbon atoms. The cycloalkyl group can be either a monocyclic or polycyclic condensed system, and it may be fused to an aromatic ring. Non-limiting examples of "cycloalkyl" include, but are not limited to, cyclopropyl, cyclobutyl, cyclopentyl, and cyclohexyl.

The term "halogen" as used herein refers to fluorine, chlorine, bromine, and iodine atoms.

The term "aryl" as used herein refers to a monocyclic or bicyclic aromatic system containing at least one unsaturated aromatic ring, preferably an aromatic group with 6-10, i.e., 6, 7, 8, 9 or 10 carbon atoms. Non-limiting examples of "aryl" include, but are not limited to, phenyl, naphthyl, 1,2,3,4-tetrahydronaphthyl, and indenyl.

The term "heteroaryl" as used herein refers to a monocyclic or bicyclic unsaturated aromatic ring system optionally substituted by at least one heteroatom independently selected from the group consisting of N, O, and S, preferably an aromatic heterocyclic group with 5-10, i.e., 5, 6, 7, 8, 9 or 10 atoms. Non-limiting examples of "heteroaryl" include, but are not limited to, thienyl, 2-pyridyl, 3-pyridyl, thiazolyl, isothiazolyl, furanyl, pyrrolyl, triazolyl, and imidazolyl.

The term "heterocycloalkyl" as used herein refers to a monocyclic or bicyclic saturated ring system optionally substituted by at least one and up to four heteroatoms independently selected from the group consisting of N, O, and S, preferably a heterocyclic group having 4-10, i.e., 4, 5, 6, 7, 8, 9 or 10 atoms, provided that the ring does not contain two adjacent O or S atoms. Non-limiting examples of "heterocycloalkyl" include, but are not limited to, pyrrolidinyl, piperidinyl, morpholinyl, and piperazinyl.

The term "haloalkyl" as used herein refers to an alkyl group that is substituted by one or more halogen atoms, where the alkyl is as defined above.

The term "haloalkoxy" as used herein refers to an alkoxy group that is substituted by one or more halogen atoms, where the alkoxy is as defined above.

The term "solvate" as used herein refers to a physical combination of a compound of the present disclosure with one or more solvent molecules. Representative solvates include organic solvates such as methanolates, ethanolates, and acetonitrile, as well as hydrates such as monohydrates, sesquihydrates, and dihydrates.

The term "hydroxyl" as used herein refers to -OH.

The term "amino" as used herein refers to $-NH_2$.

The term "cyano" as used herein refers to -CN.

The term "nitro" as used herein refers to $-NO_2$.

The term "sulfonyl" as used herein refers to $-SO_3H$.

The term "ethoxycarbonyl" as used herein refers to-COOEt.

The term "ester" as used herein refers to -C(O)O(alkyl) or -C(O)O(cycloalkyl), where alkyl and cycloalkyl are as defined above.

The term "acyl" as used herein refers to -C(O)(alkyl), -C(O)H or -C(O)O(cycloalkyl), where alkyl and cycloalkyl are as defined above.

The term "adjacent substituents, together with the atoms they are attached to, form a ring" as used herein refers to a carbon ring or a heterocyclic ring that contains O, N or S. The ring may optionally be substituted by alkyl or alkoxy.

The term "long-acting" as used herein indicate that a single drug is considered to be long-acting when the duration of its anesthetic effect is longer than that of levobupivacaine hydrochloride at a standard concentration.

The terms "optional" or "optionally" indicate that the described event or circumstance may occur but is not required to occur. The description covers both scenarios where the event or circumstance does and does not occur. For example, "aryl optionally substituted by alkyl" means that the alkyl may or may not be present, and includes both instances where the aryl is substituted by alkyl and where it is not.

Additional features and advantages of the present application will be described in the following description and will partly become evident from the description or through the implementation of the present application. Further advantages can be realized and achieved through the embodiments described in the description.

## DETAILED DESCRIPTION

The following specific examples illustrate the implementation process and the beneficial effects of the present disclosure. These examples are intended to help readers better understand the essence and characteristics of the present disclosure and are not meant to limit the implementation scope of the present application. The examples provided are for illustrative purposes only and should not be construed as limiting the scope of the present application.

The structures of the compounds were determined using nuclear magnetic resonance (NMR) and/or mass spectrometry (MS). NMR chemical shifts ($\delta$) are reported in units of $10^{-6}$. NMR measurements were conducted with a Bruker Ultrashield™ 400 MHz Plus NMR spectrometer, with deuterated dimethyl sulfoxide (DMSO-$d_6$), deuterated chloroform ($CDCl_3$), and deuterated methanol ($CD_3OD$) used as solvents, and with tetramethylsilane (TMS) used as the internal standard.

MS was conducted using a Waters QDa MS KAD3195 portable mass spectrometer.

HPLC was performed with a Waters 2545-2767-2489 high-pressure liquid chromatograph, with an Epic Polar 5u 120A 25 cm × 30 mm column.

Thin layer chromatography was carried out using Xiya Reagent GF254 silica gel plates.

For column chromatography, 200-300 mesh silica gel from Qingdao Marine Chemical Co., Ltd. was used as the carrier.

The starting materials known in the present application can be synthesized using methods well-established in the art or purchased from reagent suppliers such as Aladdin, Bidex Pharmaceuticals, and WuXi AppTec Labnetwork.

Abbreviations related to chemical synthesis:

TLC: Thin Layer Chromatography

HPLC: High Performance Liquid Chromatography

MS: Mass Spectrometry

$^1$H NMR: Proton Nuclear Magnetic Resonance Spectroscopy

$^{13}$C NMR: Carbon-13 Nuclear Magnetic Resonance Spectroscopy

V/m: Volume-to-mass ratio, indicating that the preceding equivalent (eq) refers to the ratio of volume to mass

m/m: Mass-to-mass ratio, indicating that the preceding equivalent (eq) refers to the ratio of mass to mass

### Example 1

(2S)-1-Butyl-2-((2,6-dimethylphenyl)carbamoyl)-1-(4-(phenylamino)butyl)piperidine bromide

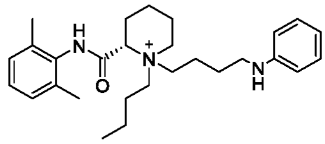

1

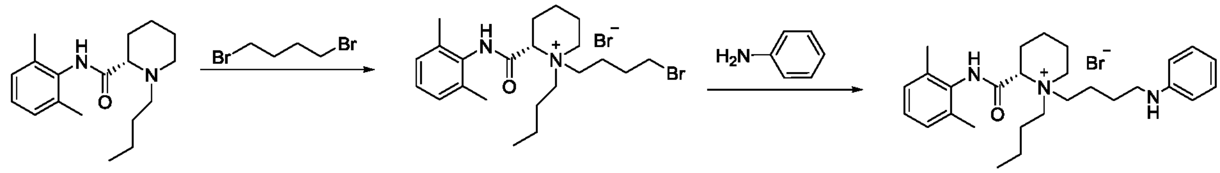

1a 1b 1

Step 1

Levobupivacaine 1a (17.36 mmol, 1.0 eq, 5 g) was weighed and added with 1,4-dibromobutane (2.0 eq of 1a, V/m, 10 ml). The mixture was stirred and heated to 100°C. The reaction progress was monitored to be completed using TLC. Column chromatography was performed on silica gel using an eluent system of $CH_2Cl_2$: MeOH=20:1. The eluate was collected and concentrated by rotary evaporation to yield an orange-yellow oily liquid, Compound 1b (4.5 g, 59.13% yield, HPLC > 90%). ESI-MS m/z: 423.34[M]$^+$, 425.34[M+2H]$^+$.

Step 2

Compound 1b (1.14 mmol, 1 eq, 0.50 g) was weighed and dissolved in analytical-grade acetonitrile (10 eq of 1b, V/m, 5.0 ml) with stirring. Analytical-grade sodium bicarbonate (1 eq of 1b, m/m, 0.50 g) and aniline (1.34 mmol, 1.2 eq, 0.14 g) were added. The mixture was heated to 80°C for reaction, and the reaction progress was monitored to be completed using TLC. The reaction mixture was filtered, and the filter cake was washed with acetonitrile (30 eq of 1b, V/m, 15 ml, three times). The filtrate was collected and subjected to preparative separation to obtain a white solid, Compound 1 (0.10 g, 15.85% yield, HPLC > 98%).

MS m/z=436.40[M]$^+$.

$^1$H NMR (400MHz, $CD_3OD$): δ12.26(s, 1H), 10.09(s, 1H), 7.12-6.97(m, 5H), 6.72-6.53(m, 3H), 6.42(s, 1H), 4.56(t, 1H), 3.38-3.11(m, 8H), 2.21-2.13(m, 6H), 1.98-1.92(m, 2H), 1.78-1.69(m, 6H), 1.58-1.45(m, 2H), 1.43-1.25(m, 4H), 0.97-0.82(t, 3H).

$^{13}$C NMR (400 MHz, $CD_3OD$): δ14.5, 17.4, 18.1, 19.0, 19.4, 22.4, 23.9, 26.9, 29.0, 50.8, 52.7, 55.9, 79.3, 114.5, 121.7, 128.1, 128.9, 129.6, 131.8, 137.6, 144.9, 161.8, 172.3.

**Example 2**

(2S)-1-Butyl-2-((2,6-dimethylphenyl)carbamoyl)-1-(4-(p-toluidino)butyl)piperidine bromide

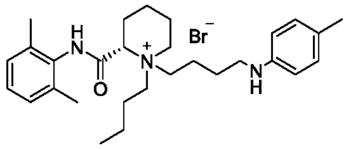

2

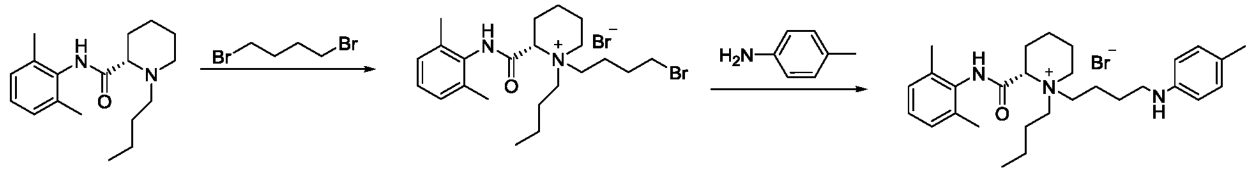

1a 1b 2

Step 1

Levobupivacaine 1a (17.36 mmol, 1.0 eq, 5 g) was weighed and added with 1,4-dibromobutane (2.0 eq of 1a, V/m, 10 ml). The mixture was stirred and heated to 100°C. The reaction progress was monitored to be completed using TLC. Column chromatography was performed on silica gel using an eluent system of $CH_2Cl_2$: MeOH=20:1. The eluate was collected and concentrated by rotary evaporation to yield an orange-yellow oily liquid, Compound 1b (4.5 g, 59.13% yield,

HPLC > 90%). ESI-MS m/z: 423.34[M]+, 425.34[M+2H]+.

Step 2

Compound 1b (1.14 mmol, 1 eq, 0.50 g) was weighed and dissolved in analytical-grade acetonitrile (10 eq of 1b, V/m, 5.0 ml) with stirring. Analytical-grade sodium bicarbonate (1 eq of 1b, m/m, 0.50 g) and p-toluidine (1.34 mmol, 1.2 eq, 0.14 g) were added. The mixture was heated to 80°C for reaction, and the reaction progress was monitored to be completed using TLC. The reaction mixture was filtered and the filter cake was washed with acetonitrile (30 eq of 1b, V/m, 15 ml, three times). The filtrate was collected and subjected to preparative separation to obtain a white solid, Compound 2 (0.11 g, 17.06% yield, HPLC > 98%).
MS m/z (ESI)=450.34[M]+.
$^1$H NMR (400MHz, $CD_3OD$): δ12.29(s, 1H), 10.11(s, 1H), 7.16-6.99(m, 5H), 6.55-6.43(m, 3H), 4.58(t, 1H), 3.35-3.14(m, 8H), 2.36(s, 3H), 2.21-2.11(m, 6H), 1.97-1.91(m, 2H), 1.70-1.61(m, 6H), 1.51-1.49(m, 2H), 1.41-1.21(m, 4H), 0.99-0.85(t, 3H).
$^{13}$C NMR (400 MHz, $CD_3OD$): δ 14.1, 17.6, 18.3, 19.0, 19.4, 21.0, 22.8, 23.7, 26.9, 29.0, 50.9, 51.9, 56.2, 79.9, 113.3, 113.5, 121.7, 127.1, 128.0, 129.6, 129.8, 131.9, 137.8, 144.5, 162.0, 172.5.

**Example 3**

(2S)-1-Butyl-2-((2,6-dimethylphenyl)carbamoyl)-1-(4-(p-fluorophenylamino)butyl)pipe ridine bromide

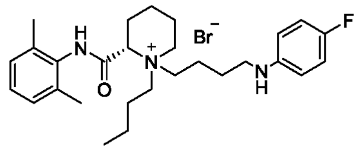

3

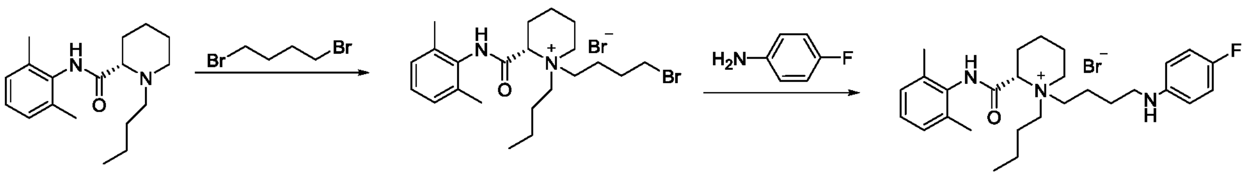

1a 1b 3

Step 1

Levobupivacaine 1a (17.36 mmol, 1.0 eq, 5 g) was weighed and added with 1,4-dibromobutane (2.0 eq of 1a, V/m, 10 ml). The mixture was stirred and heated to 100°C. The reaction progress was monitored to be completed using TLC. Column chromatography was performed on silica gel using an eluent system of $CH_2Cl_2$: MeOH=20:1. The eluate was collected and concentrated by rotary evaporation to yield an orange-yellow oily liquid, Compound 1b (4.5 g, 59.13% yield, HPLC > 90%). ESI-MS m/z: 423.34[M]+, 425.34[M+2H]+.

Step 2

Compound 1b (1.14 mmol, 1 eq, 0.50 g) was weighed and dissolved in analytical-grade acetonitrile (10 eq of 1b, V/m, 5.0 ml) with stirring. Analytical-grade sodium bicarbonate (1 eq of 1b, m/m, 0.50 g) and 4-fluoroaniline (1.34 mmol, 1.2 eq, 0.14 g) were added. The mixture was heated to 80°C for reaction and the reaction progress was monitored to be completed using TLC. The reaction mixture was filtered and the filter cake was washed with acetonitrile (30 eq of 1b, V/m, 15 ml, three times). The filtrate was collected and subjected to preparative separation to obtain a yellow solid, Compound 3 (0.11 g, 16.96% yield, HPLC > 98%).
MS m/z (ESI)=454.30[M]+.
$^1$H NMR (400MHz, $CD_3OD$): δ12.22(s, 1H), 10.09(s, 1H), 7.09-6.97(m, 7H), 6.43(s, 1H), 4.51(t, 1H), 3.35-3.17(m, 8H), 2.33-2.10(m, 6H), 1.92-1.80(m, 2H), 1.71-1.62(m, 6H), 1.52-1.45(m, 2H), 1.32-1.21(m, 4H), 0.97-0.82(t, 3H).

$^{13}C$ NMR (400 MHz, $CD_3OD$): δ14.0, 17.5, 18.3, 19.0, 19.5, 22.3, 23.5, 26.5, 28.9, 50.9, 52.2, 55.8, 79.6, 114.8, 116.7, 119.1, 126.9, 127.9, 131.0, 137.9, 144.0, 155.9, 162.8, 172.1.

**Example 4**

(2S)-1-Butyl-2-((2,6-dimethylphenyl)carbamoyl)-1-(4-(phenoxy)butyl)piperidine bromide

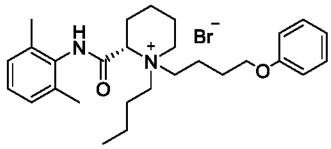

4

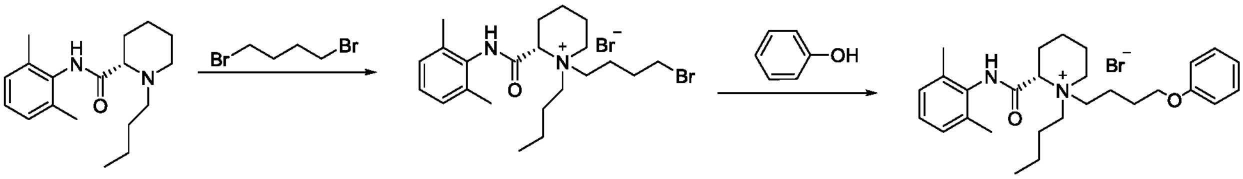

1a 1b 4

Step 1

Levobupivacaine 1a (17.36 mmol, 1.0 eq, 5 g) was weighed and added with 1,4-dibromobutane (2.0 eq of 1a, V/m, 10 ml). The mixture was stirred and heated to 100°C. The reaction progress was monitored to be completed using TLC. Column chromatography was performed on silica gel using an eluent system of $CH_2Cl_2$: MeOH=20:1. The eluate was collected and concentrated by rotary evaporation to yield an orange-yellow oily liquid, Compound 1b (4.5 g, 59.13% yield, HPLC > 90%). ESI-MS m/z: 423.34$[M]^+$, 425.34$[M+2H]^+$.

Step 2

Compound 1b (1.14 mmol, 1 eq, 0.50 g) was weighed and dissolved in analytical-grade acetonitrile (10 eq of 1b, V/m, 5.0 ml) with stirring. Analytical-grade sodium bicarbonate (1 eq of 1b, m/m, 0.50 g) and phenol (1.34 mmol, 1.2 eq, 0.14 g) were added. The mixture was heated to 80°C for reaction and the reaction progress was monitored to be completed using TLC. The reaction mixture was filtered and the filter cake was washed with acetonitrile (30 eq of 1b, V/m, 15 ml, three times). The filtrate was collected and subjected to preparative separation to obtain a white solid, Compound 4 (0.12 g, 19.04% yield, HPLC > 98%).

MS m/z=437.50$[M]^+$.

$^{1}H$ NMR (400MHz, $CD_3OD$): δ12.23(s, 1H), 10.02(s, 1H), 7.27-7.20(m, 2H), 7.12-7.05(m, 3H), 6.98-6.93(m, 3H), 4.51-4.58(t, 1H), 4.07-4.01(t, 1H), 3.41-3.20(m, 6H), 2.25-2.10(m, 6H), 1.97-1.90(m, 2H), 1.78-1.61(m, 8H), 1.46-1.31(m, 4H), 0.94-0.88(t, 3H).

$^{13}C$ NMR (400 MHz, $CD_3OD$): δ13.9, 17.3, 17.9, 19.1, 19.5, 22.3, 23.4, 26.5, 28.6, 51.3, 56.7, 56.9, 68.8, 79.9, 113.5, 114.3, 120.7, 126.9, 127.9, 129.9, 130.9, 137.3, 159.3, 161.6, 172.2.

**Example 5**

(2S)-1-Butyl-2-((2,6-dimethylphenyl)carbamoyl)-1-(4-(m-tolyloxy)butyl)piperidine bromide

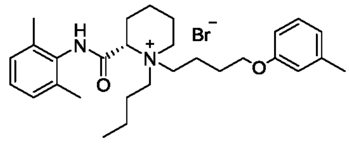

5

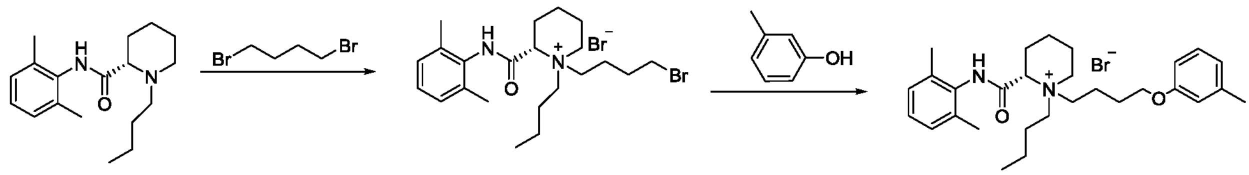

1a 1b 5

Step 1

Levobupivacaine 1a (17.36 mmol, 1.0 eq, 5 g) was weighed and added with 1,4-dibromobutane (2.0 eq of 1a, V/m, 10 ml). The mixture was stirred and heated to 100°C. The reaction progress was monitored to be completed using TLC. Column chromatography was performed on silica gel using an eluent system of $CH_2Cl_2$: MeOH=20:1. The eluate was collected and concentrated by rotary evaporation to yield an orange-yellow oily liquid, Compound 1b (4.5 g, 59.13% yield, HPLC > 90%). ESI-MS m/z: 423.34$[M]^+$, 425.34$[M+2H]^+$.

Step 2

Compound 1b (1.14 mmol, 1 eq, 0.50 g) was weighed and dissolved in analytical-grade acetonitrile (10 eq of 1b, V/m, 5.0 ml) with stirring. Analytical-grade sodium bicarbonate (1 eq of 1b, m/m, 0.50 g) and 3-methylphenol (1.34 mmol, 1.2 eq, 0.14 g) were added. The mixture was heated to 80°C for reaction and the reaction progress was monitored to be completed using TLC. The reaction mixture was filtered and the filter cake was washed with acetonitrile (30 eq of 1b, V/m, 15 ml, three times). The filtrate was collected and subjected to preparative separation to obtain a white solid, Compound 5 (0.13 g, 20.17% yield, HPLC > 98%).

MS m/z=451.60$[M]^+$.

$^1H$ NMR (400MHz, $CD_3OD$): δ12.22(s, 1H), 10.06(s, 1H), 7.18-7.15(t, 1H), 7.05(s, 3H), 6.79-6.72(m, 3H), 4.56(t, 1H), 4.09-4.05(m, 2H), 3.39-3.12(m, 6H), 2.30(s, 3H), 2.28-2.06(m, 6H), 1.96-1.91(m, 2H), 1.75-1.59(m, 8H), 1.45-1.23(m, 4H), 0.93(t, 3H).

$^{13}C$ NMR (400 MHz, $CD_3OD$): δ13.9, 17.5, 17.9, 19.1, 19.6, 21.8, 22.5, 23.5, 26.4, 28.5, 51.9, 56.9, 57.2, 68.9, 79.9, 111.3, 113.5, 113.9, 120.7, 126.9, 128.2, 129.5, 130.8, 137.9, 139.8, 157.9, 161.8, 172.8.

**Example 6**

(2S)-1-(4-(2-amino-5-fluorophenoxy)butyl)-1-butyl-2-((2,6-dimethylphenyl)carbamoyl) piperidine bromide

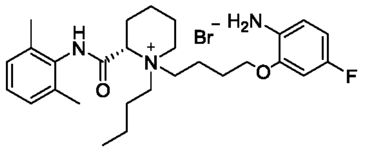

6

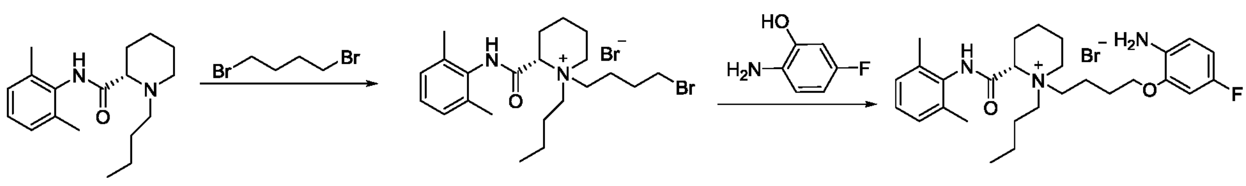

1a 1b 6

Step 1

Levobupivacaine 1a (17.36 mmol, 1.0 eq, 5 g) was weighed and added with 1,4-dibromobutane (2.0 eq of 1a, V/m, 10 ml). The mixture was stirred and heated to 100°C. The reaction progress was monitored to be completed using TLC. Column chromatography was performed on silica gel using an eluent system of $CH_2Cl_2$: MeOH=20:1. The eluate was collected and concentrated by rotary evaporation to yield an orange-yellow oily liquid, Compound 1b (4.5 g, 59.13% yield, HPLC > 90%). ESI-MS m/z: 423.34$[M]^+$, 425.34$[M+2H]^+$.

Step 2

Compound 1b (1.14 mmol, 1 eq, 0.50 g) was weighed and dissolved in analytical-grade acetonitrile (10 eq of 1b, V/m, 5.0 ml) with stirring. Analytical-grade sodium bicarbonate (1 eq of 1b, m/m, 0.50 g) and 2-amino-5-fluorophenol (1.34 mmol, 1.2 eq, 0.14 g) were added. The mixture was heated to 80°C for reaction and the reaction progress was monitored to be completed using TLC. The reaction mixture was filtered and the filter cake was washed with acetonitrile (30 eq of 1b, V/m, 15 ml, three times). The filtrate was collected and subjected to preparative separation to obtain a yellow solid, Compound 6 (0.10 g, 15.79% yield, HPLC > 98%).

MS m/z=470.36$[M]^+$.

$^1H$ NMR (400MHz, $CD_3OD$): δ12.25(s, 1H), 10.06(s, 1H), 10.03(s, 1H), 7.09(s, 3H), 6.89-6.82(m, 1H), 6.79(s, 1H), 6.57-6.51(m, 1H), 6.49-6.42(m, 1H), 4.57(t, 1H), 3.38-3.16(m, 8H), 2.25-2.08(m, 6H), 1.99-1.80(m, 2H), 1.74-1.56(m, 6H), 1.55-1.45(m, 2H), 1.45-1.19(m, 4H), 1.45-1.23(m, 4H), 0.88(t, 3H).

$^{13}C$ NMR (400 MHz, $CD_3OD$): δ14.2, 17.8, 18.5, 19.3, 19.6, 22.5, 23.3, 26.5, 28.8, 51.5, 52.0, 56.8, 79.8, 104.9, 109.1, 113.8, 116.7, 126.8, 127.9, 130.9, 135.7, 137.3, 143.5, 161.6, 172.2.

**Example 7**

(2S)-1-Butyl-2-((2,6-dimethylphenyl)carbamoyl)-1-(5-(phenylamino)pentyl)piperidine bromide

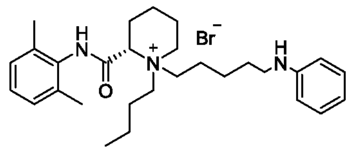

7

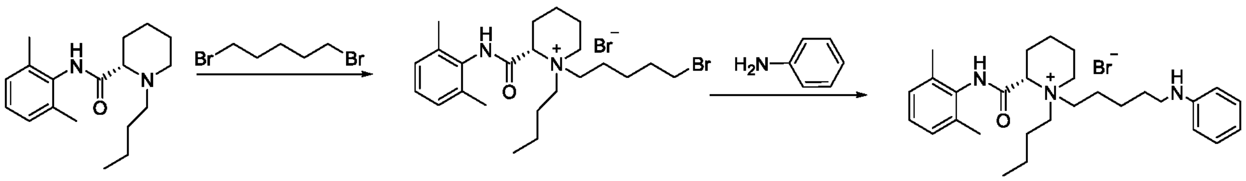

1a 1c 7

Step 1

Levobupivacaine 1a (17.36 mmol, 1.0 eq, 5 g) was weighed and added with 1,5-dibromopentane (2.0 eq of levobupivacaine 1a, V/m, 10 ml). The mixture was stirred and heated to 100°C. The reaction progress was monitored to be completed using TLC. Column chromatography was performed on silica gel using an eluent system of $CH_2Cl_2$: MeOH=20:1. The eluate was collected and concentrated by rotary evaporation to yield an orange-yellow oily liquid, Compound 1c (4.5 g, 59.13% yield, HPLC > 90%). ESI-MS m/z: 437.30$[M]^+$, 439.30$[M+2H]^+$.

Step 2

Compound 1c (1.14 mmol, 1 eq, 0.50 g) was weighed and dissolved in analytical-grade acetonitrile (10 eq of 1c, V/m, 5.0 ml) with stirring. Analytical-grade sodium bicarbonate (1 eq of 1c, m/m, 0.50 g) and aniline (1.34 mmol, 1.2 eq, 0.13 g) were added. The mixture was heated to 80°C for reaction and the reaction progress was monitored to be completed using HPLC. The reaction mixture was filtered and the filter cake was washed with acetonitrile (30 eq of 1c, V/m, 15 ml,

three times). The filtrate was collected and subjected to preparative separation to obtain a white powdery solid, Compound 7 (0.12 g, 18.61% yield, HPLC > 98%).

MS m/z=450.60[M]$^+$.

$^1$H NMR (400MHz, $CD_3OD$): δ12.24(s, 1H), 10.04(s, 1H), 7.09-7.03(m, 5H), 6.68-6.54(m, 3H), 6.45(s, 1H), 4.60(t, 1H), 3.40-3.05(m, 8H), 2.23-2.11(m, 6H), 1.96-1.90(m, 2H), 1.79-1.56(m, 8H), 1.38-1.20(m, 6H), 0.91(t, 3H).

$^{13}$C NMR (400 MHz, $CD_3OD$): δ14.0, 17.8, 19.2, 19.5, 22.4, 23.3, 25.5, 25.7, 31.6, 44.1, 51.8, 56.9, 56.7, 79.9, 113.5, 113.6, 121.0, 129.0, 128.7, 129.7, 130.9, 137.3, 145.2, 161.5, 172.2.

**Example 8**

(2S)-1-Butyl-2-((2,6-dimethylphenyl)carbamoyl)-1-(5-(methyl(phenyl)amino)pentyl)pi peridine bromide

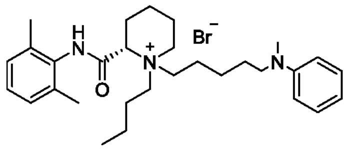

8

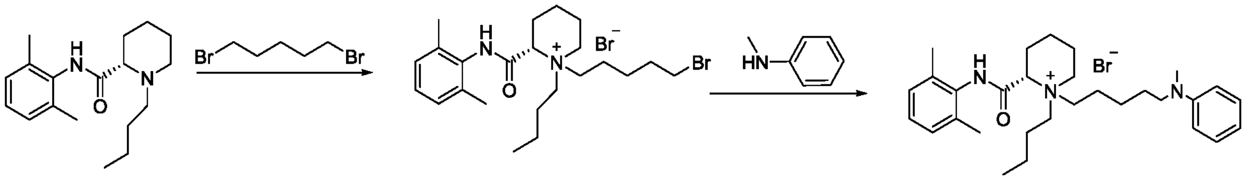

1a 1c 8

Step 1

Levobupivacaine 1a (17.36 mmol, 1.0 eq, 5 g) was weighed and added with 1,5-dibromopentane (2.0 eq of levobupivacaine 1a, V/m, 10 ml). The mixture was stirred and heated to 100°C. The reaction progress was monitored to be completed using TLC. Column chromatography was performed on silica gel using an eluent system of $CH_2Cl_2$: MeOH=20:1. The eluate was collected and concentrated by rotary evaporation to yield an orange-yellow oily liquid, Compound 1c (4.5 g, 59.13% yield, HPLC > 90%). ESI-MS m/z: 437.30[M]$^+$, 439.30[M+2H]$^+$.

Step 2

Compound 1c (1.14 mmol, 1 eq, 0.50 g) was weighed and dissolved in analytical-grade acetonitrile (10 eq of 1c, V/m, 5.0 ml) with stirring. Analytical-grade sodium bicarbonate (1 eq of 1c, m/m, 0.50 g) and N-methylaniline (1.34 mmol, 1.2 eq, 0.13 g) were added. The mixture was heated to 80°C for reaction and the reaction progress was monitored to be completed using HPLC. The reaction mixture was filtered and the filter cake was washed with acetonitrile (30 eq of 1c, V/m, 15 ml, three times). The filtrate was collected and subjected to preparative separation to obtain a white powdery solid, Compound 8 (0.11 g, 16.70% yield, HPLC > 98%).

MS m/z=464.50[M]$^+$.

$^1$H NMR (400MHz, $CD_3OD$): δ12.20(s, 1H), 10.01(s, 1H), 7.29-7.20(m, 2H), 7.09-7.01(m, 3H), 6.98-6.92(m, 2H), 6.86-6.80(m, 1H), 4.60-4.54(m, 1H), 3.39-3.10(m, 8H), 2.75(s, 3H), 2.25-2.10(m, 6H), 1.93-1.88(m, 2H), 1.79-1.60(m, 6H), 1.58-1.45(m, 2H), 1.41-1.25(m, 6H), 0.90(t, 3H).

$^{13}$C NMR (400 MHz, $CD_3OD$): δ13.8, 17.6, 19.0 , 19.3, 22.2, 23.1, 25.3, 25.8, 26.3, 29.0, 41.7, 51.8, 55.9, 56.5, 56.8, 79.7, 114.3, 121.9, 127.7, 129.6, 130.6, 137.1, 149.5, 172.0.

**Example 9**

(2S)-1-Butyl-2-((2,6-dimethylphenyl)carbamoyl)-1-(5-(p-toluylamino)pentyl)piperidine bromide

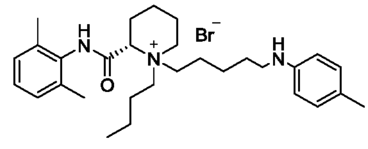

9

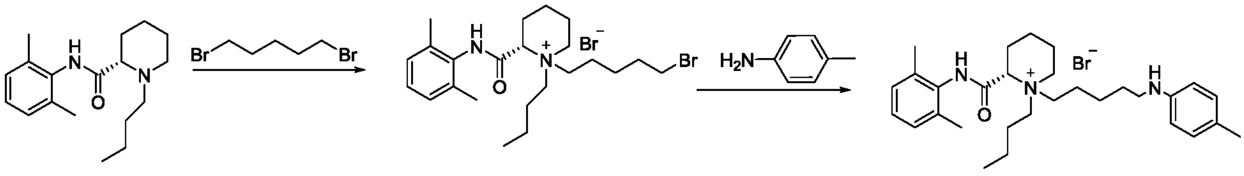

1a 1c 9

Step 1

Levobupivacaine 1a (17.36 mmol, 1.0 eq, 5 g) was weighed and added with 1,5-dibromopentane (2.0 eq of levobupivacaine 1a, V/m, 10 ml). The mixture was stirred and heated to 100°C. The reaction progress was monitored to be completed using TLC. Column chromatography was performed on silica gel using an eluent system of $CH_2Cl_2$: MeOH=20:1. The eluate was collected and concentrated by rotary evaporation to yield an orange-yellow oily liquid, Compound 1c (4.5 g, 59.13% yield, HPLC > 90%). ESI-MS m/z: 437.30$[M]^+$, 439.30$[M+2H]^+$.

Step 2

Compound 1c (1.14 mmol, 1 eq, 0.50 g) was weighed and dissolved in analytical-grade acetonitrile (10 eq of 1c, V/m, 5.0 ml) with stirring. Analytical-grade sodium bicarbonate (1 eq of 1c, m/m, 0.50 g) and p-toluidine (1.34 mmol, 1.2 eq, 0.13 g) were added. The mixture was heated to 80°C for reaction and the reaction progress was monitored to be completed using HPLC. The reaction mixture was filtered and the filter cake was washed with acetonitrile (30 eq of 1c, V/m, 15 ml, three times). The filtrate was collected and subjected to preparative separation to obtain a white powdery solid, Compound 9 (0.13 g, 19.73% yield, HPLC > 98%).

MS m/z=464.70$[M]^+$.

$^1$H NMR (400MHz, $CD_3OD$): δ12.26(s, 1H), 10.03(s, 1H), 7.10-7.01(m, 5H), 6.49-6.43(m, 3H), 4.60-4.56(s, 1H), 3.30-3.05(m, 8H), 2.33(s, 3H), 2.20-2.11(m, 6H), 1.98-1.90(m, 2H), 1.79-1.53(m, 8H), 1.42-1.16(m, 6H), 0.94-0.83(m, 3H).

$^{13}$C NMR (400 MHz, $CD_3OD$): δ13.8, 17.6, 19.5, 19.8, 22.0, 22.6, 23.5, 25.5, 25.7, 26.5, 31.6, 43.9, 51.7, 56.8, 56.9, 79.8, 113.8, 114.9, 126.9, 127.8, 129.7, 129.9, 130.5, 137.6, 172.5.

**Example 10**

(2S)-1-Butyl-2-((2,6-dimethylphenyl)carbamoyl)-1-(5-(p-fluorophenylamino)pentyl)pip eridine bromide

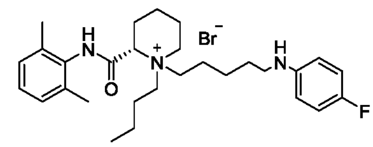

10

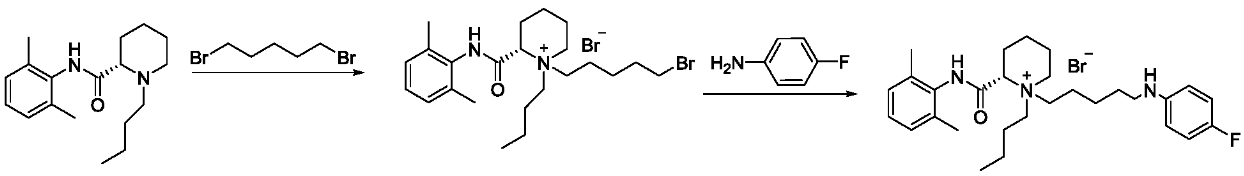

1a 1c 10

Step 1

Levobupivacaine 1a (17.36 mmol, 1.0 eq, 5 g) was weighed and added with 1,5-dibromopentane (2.0 eq of levobupivacaine 1a, V/m, 10 ml). The mixture was stirred and heated to 100°C. The reaction progress was monitored to be completed using TLC. Column chromatography was performed on silica gel using an eluent system of $CH_2Cl_2$: MeOH=20:1. The eluate was collected and concentrated by rotary evaporation to yield an orange-yellow oily liquid, Compound 1c (4.5 g, 59.13% yield, HPLC > 90%). ESI-MS m/z: 437.30$[M]^+$, 439.30$[M+2H]^+$.

Step 2

Compound 1c (1.14 mmol, 1 eq, 0.50 g) was weighed and dissolved in analytical-grade acetonitrile (10 eq of 1c, V/m, 5.0 ml) with stirring. Analytical-grade sodium bicarbonate (1 eq of 1c, m/m, 0.50 g) and p-fluoroaniline (1.34 mmol, 1.2 eq, 0.13 g) were added. The mixture was heated to 80°C for reaction and the reaction progress was monitored to be completed using HPLC. The reaction mixture was filtered and the filter cake was washed with acetonitrile (30 eq of 1c, V/m, 15 ml, three times). The filtrate was collected and subjected to preparative separation to obtain a yellow powdery solid, Compound 10 (0.13 g, 19.61% yield, HPLC > 98%).

MS m/z=468.50$[M]^+$.

$^1$H NMR (400MHz, $CD_3OD$): δ12.26(s, 1H), 10.09(s, 1H), 7.12-6.97(m, 5H), 6.72-6.53(m, 3H), 6.42(s, 1H), 4.56(t, 1H), 3.38-3.11(m, 8H), 2.21-2.13(m, 6H), 1.98-1.92(m, 2H), 1.78-1.69(m, 6H), 1.58-1.45(m, 2H), 1.43-1.25(m, 4H), 0.97-0.82(t, 3H).

$^{13}$C NMR (400 MHz, $CD_3OD$): δ14.5, 17.4, 18.1, 19.0, 19.4, 22.4, 23.9, 26.9, 29.0, 50.8, 52.7, 55.9, 79.3, 114.5, 121.7, 128.1, 128.9, 129.6, 131.8, 137.6, 144.9, 161.8, 172.3.

**Example 11**

(2S)-1-Butyl-1-(5-(5,6-dihydroimidazol[1,2-a]pyrazin-7(8H)-yl)pentyl)-2-((2,6-dimethyl phenyl)carbamoyl)piperidine bromide

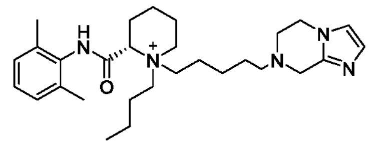

11

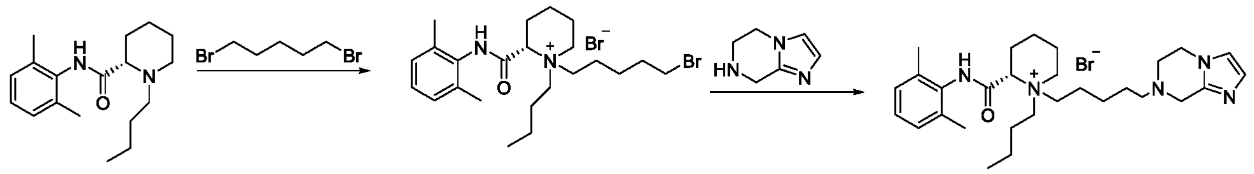

1a 1c 11

Step 1

Levobupivacaine 1a (17.36 mmol, 1.0 eq, 5 g) was weighed and added with 1,5-dibromopentane (2.0 eq of levobupivacaine 1a, V/m, 10 ml). The mixture was stirred and heated to 100°C. The reaction progress was monitored to be completed using TLC. Column chromatography was performed on silica gel using an eluent system of $CH_2Cl_2$: MeOH=20:1. The eluate was collected and concentrated by rotary evaporation to yield an orange-yellow oily liquid, Compound 1c (4.5 g, 59.13% yield, HPLC > 90%). ESI-MS m/z: 437.30$[M]^+$, 439.30$[M+2H]^+$.

Step 2

Compound 1c (1.14 mmol, 1 eq, 0.50 g) was weighed and dissolved in analytical-grade acetonitrile (10 eq of 1c, V/m, 5.0 ml) with stirring. Analytical-grade sodium bicarbonate (1 eq of 1c, m/m, 0.50 g) and 5,6,7,8-tetrahydroimidazol [1,2-a]pyrazine (1.34 mmol, 1.2 eq, 0.13 g) were added. The mixture was heated to 80°C for reaction and the reaction

progress was monitored to be completed using HPLC. The reaction mixture was filtered and the filter cake was washed with acetonitrile (30 eq of 1c, V/m, 15 ml, three times). The filtrate was collected and subjected to preparative separation to obtain a yellow powdery solid, Compound 11 (0.12 g, 21.40% yield, HPLC > 98%).

MS m/z=480.55[M]$^+$.

$^1$H NMR (400MHz, $CD_3OD$): δ12.20(s, 1H), 10.01(s, 1H), 7.08-7.02(m, 3H), 6.93-6.90(m, 1H), 6.76-6.72(m, 1H), 4.59(t, 1H), 4.10(t, 2H), 3.63(s, 1H), 3.39-3.10(m, 6H), 2.85(t, 2H), 2.49(t, 2H), 2.25-2.13(m, 6H), 1.99-1.88(m, 2H), 1.78-1.60(m, 6H), 1.43-1.17(m, 8H), 0.90(t, 3H).

$^{13}$C NMR (400 MHz, $CD_3OD$): δ13.1, 17.9, 19.5, 19.6, 22.5, 23.6, 25.4, 25.6, 26.3, 28.3, 39.4, 56.7, 56.7, 56.9, 57.1, 57.3, 79.9, 118.6, 126.4, 126.9, 127.8, 130.9, 137.3, 152.9, 172.5.

**Example 12**

(2S)-1-Butyl-2-((2,6-dimethylphenyl)carbamoyl)-1-(5-(1-methyl-3,4-dihydropyrrolo[1, 2-a]pyrazin-2(1H)-yl)pentyl)piperidine bromide

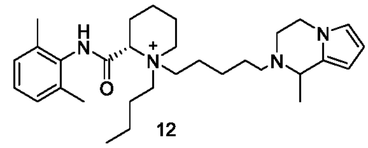

12

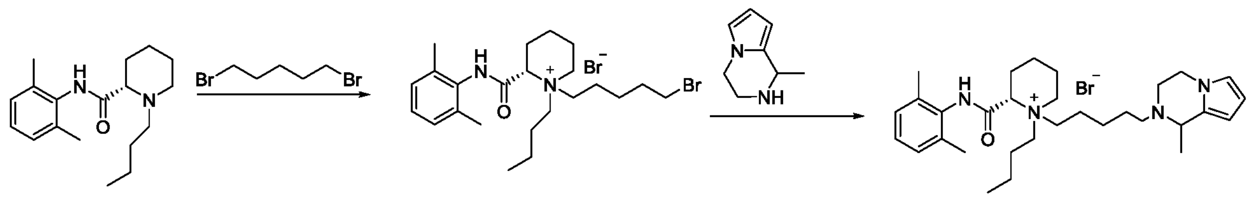


1a 1c 12

Step 1

Levobupivacaine 1a (17.36 mmol, 1.0 eq, 5 g) was weighed and added with 1,5-dibromopentane (2.0 eq of levobupivacaine 1a, V/m, 10 ml). The mixture was stirred and heated to 100°C. The reaction progress was monitored to be completed using TLC. Column chromatography was performed on silica gel using an eluent system of $CH_2Cl_2$: MeOH=20:1. The eluate was collected and concentrated by rotary evaporation to yield an orange-yellow oily liquid, Compound 1c (4.5 g, 59.13% yield, HPLC > 90%). ESI-MS m/z: 437.30[M]$^+$, 439.30[M+2H]$^+$.

Step 2

Compound 1c (1.14 mmol, 1 eq, 0.50 g) was weighed and dissolved in analytical-grade acetonitrile (10 eq of 1c, V/m, 5.0 ml) with stirring. Analytical-grade sodium bicarbonate (1 eq of 1c, m/m, 0.50 g) and 1-methyl-1,2,3,4-tetrahydropyrrolo[1,2-a] pyrazine (1.34 mmol, 1.2 eq, 0.13 g) were added. The mixture was heated to 80°C for reaction and the reaction progress was monitored to be completed using HPLC. The reaction mixture was filtered and the filter cake was washed with acetonitrile (30 eq of 1c, V/m, 15 ml, three times). The filtrate was collected and subjected to preparative separation to obtain a yellow powdery solid, Compound 12 (0.11 g, 20.88% yield, HPLC > 98%).

MS m/z=493.48[M]$^+$.

$^1$H NMR (400MHz, $CD_3OD$): δ12.23(s, 1H), 10.06(s, 1H), 7.09-7.01(m, 4H), 5.98-6.03(m, 1H), 5.76-5.70(m, 1H), 4.59(t, 1H), 4.06-3.93(m, 3H), 3.35-3.19(m, 6H), 2.75-2.53(m, 2H), 2.46-2.39(m, 2H), 2.25-2.07(m, 6H), 1.97-1.90(m, 2H), 1.82-1.59(m, 6H), 1.47-1.16(m, 11H), 0.92-0.86(m, 3H).

$^{13}$C NMR (400 MHz, $CD_3OD$): δ13.9, 17.6, 18.6, 19.4, 19.7, 22.6, 23.4, 25.4, 25.7, 26.7, 28.6, 43.4, 51.7, 54.1, 54.9, 56.7, 56.9, 63.7, 79.4, 108.4, 108.6, 122.8, 126.6, 127.2, 130.4, 132.8, 137.4, 172.9.

**Example 13**

(2S)-1-Butyl-2-((2,6-dimethylphenyl)carbamoyl)-1-(5-(1-phenyl-3,4-dihydropyrrolo[1, 2-a]pyrazin-2(1H)-yl)pentyl)piperidine bromide

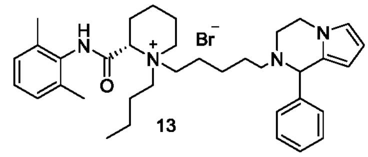

13

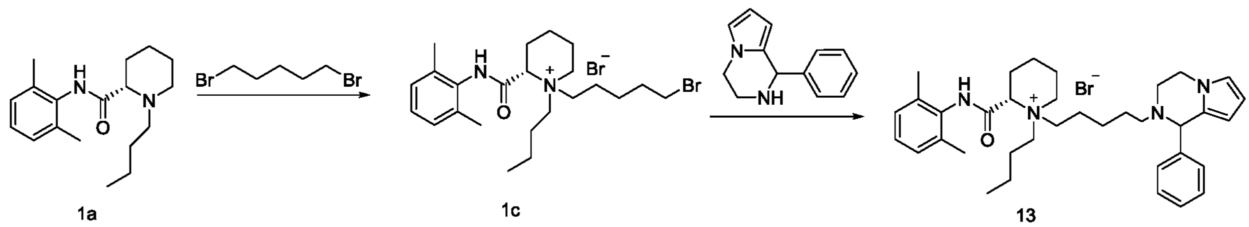

1a 1c 13

Step 1

Levobupivacaine 1a (17.36 mmol, 1.0 eq, 5 g) was weighed and added with 1,5-dibromopentane (2.0 eq of levobupivacaine 1a, V/m, 10 ml). The mixture was stirred and heated to 100°C. The reaction progress was monitored to be completed using TLC. Column chromatography was performed on silica gel using an eluent system of $CH_2Cl_2$: MeOH=20:1. The eluate was collected and concentrated by rotary evaporation to yield an orange-yellow oily liquid, Compound 1c (4.5 g, 59.13% yield, HPLC > 90%). ESI-MS m/z: 437.30$[M]^+$, 439.30$[M+2H]^+$.

Step 2

Compound 1c (1.14 mmol, 1 eq, 0.50 g) was weighed and dissolved in analytical-grade acetonitrile (10 eq of 1c, V/m, 5.0 ml) with stirring. Analytical-grade sodium bicarbonate (1 eq of 1c, m/m, 0.50 g) and 1-phenyl-1,2,3,4-tetrahydropyrrolo[1, 2-a]pyrazine (1.34 mmol, 1.2 eq, 0.13 g) were added. The mixture was heated to 80°C for reaction and the reaction progress was monitored to be completed using HPLC. The reaction mixture was filtered and the filter cake was washed with acetonitrile (30 eq of 1c, V/m, 15 ml, three times). The filtrate was collected and subjected to preparative separation to obtain a yellow powdery solid, Compound 13 (0.14 g, 21.98% yield, HPLC > 98%).

MS m/z=555.70$[M]^+$.

$^1H$ NMR (400MHz, $CD_3OD$): δ12.30(s, 1H), 10.13(s, 1H), 7.45-7.18(m, 5H), 7.01-7.09(m, 4H), 5.98-6.02(m, 1H), 5.74-5.71(m, 1H), 5.19(s, 1H), 4.62-4.47(m, 1H), 3.99-3.96(m, 2H), 3.38-3.10(m, 6H), 2.74-2.61(m, 2H), 2.48-2.41(m, 2H), 2.25-2.08(m, 6H), 1.99-1.90(m, 2H), 1.77-1.59(m, 6H), 1.45-1.13(m, 8H), 0.96-0.87(m, 3H).

$^{13}C$ NMR (400 MHz, $CD_3OD$): δ13.7, 17.1, 19.4, 19.4, 22.9, 23.4, 25.7, 26.4, 28.4, 43.7, 51.7, 51.7, 53.1, 54.7, 56.4, 56.7, 71.6, 79.0, 107.9, 108.6, 119.4, 122.7, 126.6, 127.0, 127.4, 128.1, 130.5, 134.9, 137.5, 138.4, 173.2.

**Example 14**

(2S)-1-Butyl-2-((2,6-dimethylphenyl)carbamoyl)-1-(5-(phenoxy)pentyl)piperidine bromide

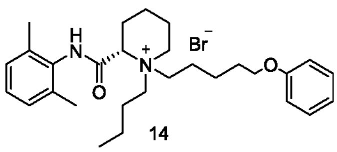

14

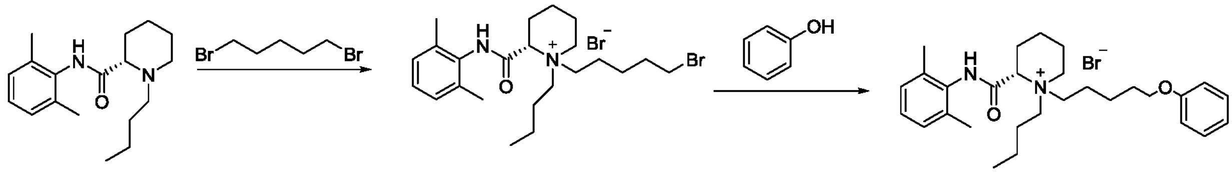

1a 1c 14

Step 1

Levobupivacaine 1a (17.36 mmol, 1.0 eq, 5 g) was weighed and added with 1,5-dibromopentane (2.0 eq of levobupivacaine 1a, V/m, 10 ml). The mixture was stirred and heated to 100°C. The reaction progress was monitored to be completed using TLC. Column chromatography was performed on silica gel using an eluent system of $CH_2Cl_2$: MeOH=20:1. The eluate was collected and concentrated by rotary evaporation to yield an orange-yellow oily liquid, Compound 1c (4.5 g, 59.13% yield, HPLC > 90%). ESI-MS m/z: 437.30$[M]^+$, 439.30$[M+2H]^+$.

Step 2

Compound 1c (1.14 mmol, 1 eq, 0.50 g) was weighed and dissolved in analytical-grade acetonitrile (10 eq of 1c, V/m, 5.0 ml) with stirring. Analytical-grade sodium bicarbonate (1 eq of 1c, m/m, 0.50 g) and phenol (1.34 mmol, 1.2 eq, 0.13 g) were added. The mixture was heated to 80°C for reaction and the reaction progress was monitored to be completed using HPLC. The reaction mixture was filtered and the filter cake was washed with acetonitrile (30 eq of 1c, V/m, 15 ml, three times). The filtrate was collected and subjected to preparative separation to obtain a white powdery solid, Compound 14 (0.13 g, 20.17% yield, HPLC > 98%).

MS m/z=451.50$[M]^+$.

$^1$H NMR (400MHz, $CD_3OD$): δ12.26(s, 1H), 10.09(s, 1H), 7.12-6.97(m, 5H), 6.72-6.53(m, 3H), 6.42(s, 1H), 4.56(t, 1H), 3.38-3.11(m, 8H), 2.21-2.13(m, 6H), 1.98-1.92(m, 2H), 1.78-1.69(m, 6H), 1.58-1.45(m, 2H), 1.43-1.25(m, 4H), 0.97-0.82(t, 3H).

**Example 15**

(2S)-1-Butyl-2-((2,6-dimethylphenyl)carbamoyl)-1-(5-(m-tolyloxy)pentyl)piperidine bromide

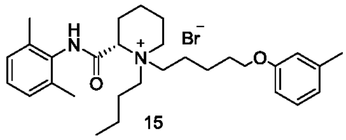

15

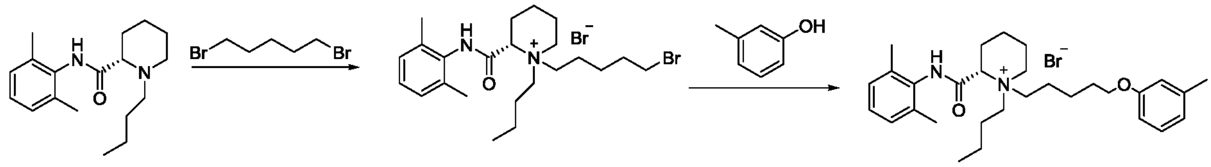

1a 1c 15

Step 1

Levobupivacaine 1a (17.36 mmol, 1.0 eq, 5 g) was weighed and added with 1,5-dibromopentane (2.0 eq of levobupivacaine 1a, V/m, 10 ml). The mixture was stirred and heated to 100°C. The reaction progress was monitored to be completed using TLC. Column chromatography was performed on silica gel using an eluent system of $CH_2Cl_2$: MeOH=20:1. The eluate was collected and concentrated by rotary evaporation to yield an orange-yellow oily liquid, Compound 1c (4.5 g, 59.13% yield, HPLC > 90%). ESI-MS m/z: 437.30$[M]^+$, 439.30$[M+2H]^+$.

Step 2

Compound 1c (1.14 mmol, 1 eq, 0.50 g) was weighed and dissolved in analytical-grade acetonitrile (10 eq of 1c, V/m, 5.0 ml) with stirring. Analytical-grade sodium bicarbonate (1 eq of 1c, m/m, 0.50 g) and 3-methylphenol (1.34 mmol, 1.2 eq, 0.13 g) were added. The mixture was heated to 80°C for reaction and the reaction progress was monitored to be completed using HPLC. The reaction mixture was filtered and the filter cake was washed with acetonitrile (30 eq of 1c, V/m, 15 ml, three times). The filtrate was collected and subjected to preparative separation to obtain a white powdery solid, Compound 15 (0.12 g, 18.22% yield, HPLC > 98%).

MS m/z=465.80[M]$^+$.

$^1$H NMR (400MHz, $CD_3OD$): δ12.26(s, 1H), 10.09(s, 1H), 7.12-6.97(m, 5H), 6.72-6.53(m, 3H), 6.42(s, 1H), 4.56(t, 1H), 3.38-3.11(m, 8H), 2.21-2.13(m, 6H), 1.98-1.92(m, 2H), 1.78-1.69(m, 6H), 1.58-1.45(m, 2H), 1.43-1.25(m, 4H), 0.97-0.82(t, 3H).

$^{13}$C NMR (400 MHz, $CD_3OD$): δ14.5, 17.4, 18.1, 19.0, 19.4, 22.4, 23.9, 26.9, 29.0, 50.8, 52.7, 55.9, 79.3, 114.5, 121.7, 128.1, 128.9, 129.6, 131.8, 137.6, 144.9, 161.8, 172.3.

## Example 16

(2S)-1-(5-(2-amino-5-fluorophenoxy)pentyl)-1-butyl-2-((2,6-dimethylphenyl)carbamoyl )piperidine bromide

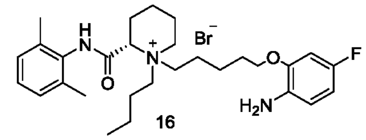

16

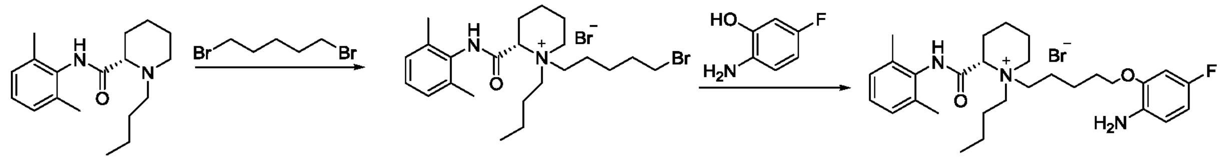

1a 1c 16

Step 1

Levobupivacaine 1a (17.36 mmol, 1.0 eq, 5 g) was weighed and added with 1,5-dibromopentane (2.0 eq of levobupivacaine 1a, V/m, 10 ml) was added. The mixture was stirred and heated to 100°C. The reaction progress was monitored to be completed using TLC. Column chromatography was performed on silica gel using an eluent system of $CH_2Cl_2$: MeOH=20:1. The eluate was collected and concentrated by rotary evaporation to yield an orange-yellow oily liquid, Compound 1c (4.5 g, 59.13% yield, HPLC > 90%). ESI-MS m/z: 437.30[M]$^+$, 439.30[M+2H]$^+$.

Step 2

Compound 1c (1.14 mmol, 1 eq, 0.50 g) was weighed and dissolved in analytical-grade acetonitrile (10 eq of 1c, V/m, 5.0 ml) with stirring. Analytical-grade sodium bicarbonate (1 eq of 1c, m/m, 0.50 g) and 2-amino-5-fluorophenol (1.34 mmol, 1.2 eq, 0.13 g) were added. The mixture was heated to 80°C for reaction and the reaction progress was monitored to be completed using HPLC. The reaction mixture was filtered and the filter cake was washed with acetonitrile (30 eq of 1c, V/m, 15 ml, three times). The filtrate was collected and subjected to preparative separation to obtain a yellow powdery solid, Compound 16 (0.12 g, 17.71% yield, HPLC > 98%).

MS m/z=484.56[M]$^+$.

$^1$H NMR (400MHz, $CD_3OD$): δ12.26(s, 1H), 10.09(s, 1H), 7.12-6.97(m, 5H), 6.72-6.53(m, 3H), 6.42(s, 1H), 4.56(t, 1H), 3.38-3.11(m, 8H), 2.21-2.13(m, 6H), 1.98-1.92(m, 2H), 1.78-1.69(m, 6H), 1.58-1.45(m, 2H), 1.43-1.25(m, 4H), 0.97-0.82(t, 3H).

$^{13}$C NMR (400 MHz, $CD_3OD$): δ14.5, 17.4, 18.1, 19.0, 19.4, 22.4, 23.9, 26.9, 29.0, 50.8, 52.7, 55.9, 79.3, 114.5, 121.7, 128.1, 128.9, 129.6, 131.8, 137.6, 144.9, 161.8, 172.3.

**Example 17**

(2S)-1-Butyl-2-((2,6-dimethylphenyl)carbamoyl)-1-(5-((4-(trifluoromethyl)phenyl)amin o)pentyl)piperidine bromide

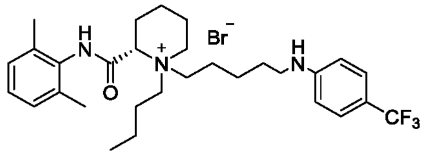

17

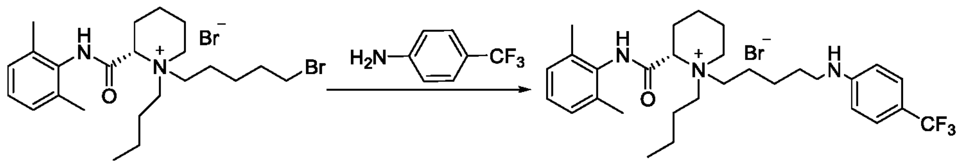

1c 17

Compound 1c (1.14 mmol, 1 eq, 0.50 g) was weighed and dissolved in analytical-grade acetonitrile (10 eq of 1c, V/m, 5.0 ml) with stirring. Analytical-grade sodium bicarbonate (1 eq of 1c, *m/m,* 0.50 g) and 4-trifluoromethylaniline (1.34 mmol, 1.2 eq, 0.14 g) were added. The mixture was heated to 80°C for reaction and the reaction progress was monitored to be completed using HPLC. The reaction mixture was filtered and the filter cake was washed with acetonitrile (30 eq of 1c, V/m, 15 ml, three times). The filtrate was collected and subjected to preparative separation to obtain a white powdery solid, Compound 17 (0.18 g, 26.47% yield, HPLC > 98%).

MS m/z=518.41 $[M]^+$.

$^1$H NMR (400MHz, $CD_3OD$): δ12.21(s, 1H), 11.92(s, 1H), 10.06(s, 1H), 7.23-7.15(m, 2H), 7.03-6.93(m, 2H), 6.88-6.78(m, 3H), 4.63-4.56(t, 1H), 4.19-4.04(t, 1H), 3.41-3.27(m, 8H), 2.35-2.19(m, 6H), 1.96-1.88(m, 2H), 1.76-1.59(m, 8H), 1.45-1.33(m, 4H), 0.96-0.87(t, 3H).

$^{13}$C NMR (400 MHz, $CD_3OD$): δ14.2, 16.7, 17.6, 18.3, 19.6, 22.4, 23.8, 26.2, 28.5, 43.1, 51.9, 56.4, 56.3, 68.4, 79.7, 113.2, 114.7, 120.1, 124.1, 126.7, 127.1, 129.5, 130.6, 137.3, 159.1, 161.8, 172.7.

**Example 18**

(2S)-1-Butyl-2-((2,6-dimethylphenyl)carbamoyl)- 1-(5-(((4-fluorophenyl)amino)-3-meth ylpentyl)piperidine bromide

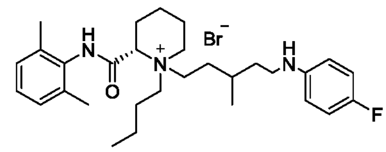

18

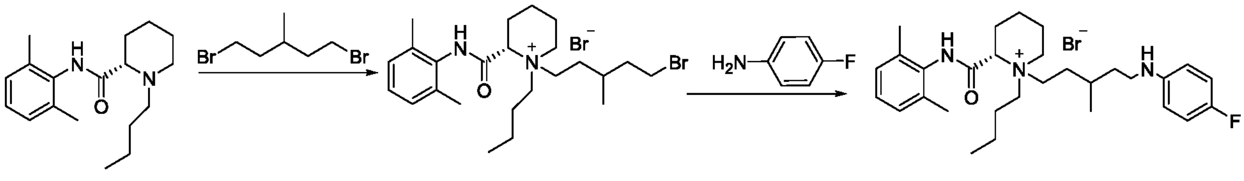

1a 2d 18

Step 1

Levobupivacaine 1a (17.36 mmol, 1.0 eq, 5 g) was weighed and added with 1,5-dibromo-3-methylpentane (2.0

eq of levobupivacaine 1a, V/m, 10 ml). The mixture was stirred and heated to 100°C. The reaction progress was monitored to be completed using TLC. Column chromatography was performed on silica gel using an eluent system of $CH_2Cl_2$: MeOH=20:1. The eluate was collected and concentrated by rotary evaporation to yield an orange-yellow oily liquid, Compound 2d (4.3 g, 46.74% yield, HPLC > 90%). ESI-MS *m*/*z:* 451.33[M]$^+$, 453.33[M+2H]$^+$.

Step 2

Compound 2d (0.94 mmol, 1 eq, 0.50 g) was weighed and dissolved in analytical-grade acetonitrile (10 eq of 2d, V/m, 5.0 ml) with stirring. Analytical-grade sodium bicarbonate (1 eq of 2d, *m*/*m,* 0.50 g) and 4-fluoroaniline (1.13 mmol, 1.2 eq, 0.13 g) were added. The mixture was heated to 80°C for reaction and the reaction progress was monitored to be completed using HPLC. The reaction mixture was filtered and the filter cake was washed with acetonitrile (30 eq of 2d, V/m, 15 ml, three times). The filtrate was collected and subjected to preparative separation to obtain a white powdery solid, Compound 18 (0.19 g, 36.05% yield, HPLC > 98%).

MS m/z=482.41[M]$^+$.

$^1$H NMR (400MHz, $CD_3OD$): δ12.16(s, 1H), 11.75(s, 1H), 10.05(s, 1H), 7.21-7.18(m, 2H), 7.04-6.94(m, 2H), 6.91-6.82(m, 3H), 4.68-4.59(t, 1H), 4.16-4.09(t, 1H), 3.46-3.29(m, 8H), 2.39-2.28(m, 6H), 1.96-1.90(m, 2H), 1.74-1.58(m, 8H), 1.43-1.29(m, 3H), 0.93-0.80(m, 6H).

$^{13}$C NMR (400 MHz, $CD_3OD$): δ14,1, 16.6, 17.5, 18.3, 19.7, 20.6, 22.4, 23.8, 26.1, 28.6, 43.4, 52.1, 56.8, 57.3, 69.1, 78.2, 113.1, 113.5, 121.1, 126.3, 127.2, 129.8, 130.6, 136.1, 158.1, 161.8, 172.7.

**Example 19**

(2S)-1-Butyl-2-((2,6-dimethylphenyl)carbamoyl)-1-(4-(symmetrical trimethylamino)butyl)piperidine bromide

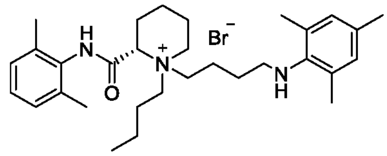

19

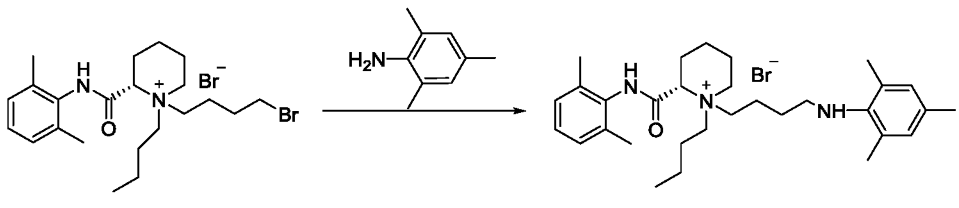

1b

19

Compound 1b (1.14 mmol, 1 eq, 0.50 g) was weighed and dissolved in analytical-grade acetonitrile (10 eq of 1b, V/m, 5.0 ml) with stirring. Analytical-grade sodium bicarbonate (1 eq of 1b, *m*/*m,* 0.50 g) and 2,4,6-trimethylaniline (1.34 mmol, 1.2 eq, 0.18 g) were added. The mixture was heated to 80°C for reaction and the reaction progress was monitored to be completed using TLC. The reaction mixture was filtered and the filter cake was washed with acetonitrile (30 eq of 1b, V/m, 15 ml, three times). The filtrate was collected and subjected to preparative separation to obtain a white solid, Compound 19 (0.16 g, 24.75% yield, HPLC > 98%).

MS m/z (ESI)=478.48[M]$^+$.

$^1$H NMR (400MHz, $CD_3OD$): δ12.23(s, 1H), 10.11(s, 1H), 7.20-7.12(m, 2H), 6.92-6.86(m, 4H), 4.55-4.49(t, 1H), 4.15-4.03(t, 1H), 3.38-3.21(m, 6H), 2.22-2.13(m, 6H), 1.99-1.91(m, 2H), 1.79-1.60(m, 8H), 1.43-1.30(m, 4H), 0.99-0.80(t, 12H).

$^{13}$C NMR (400 MHz, $CD_3OD$): δ13.7, 17.6, 17.9, 18.3, 18.6, 19.1, 19.6, 21.9, 23.1, 23.4, 26.6, 28.9, 51.4, 56.3, 56.8, 68.6, 79.9, 113.4, 114.3, 120.2, 126.7, 127.6, 129.5, 130.3, 137.9, 158.6, 161.3, 172.7.

**Example 20**

(2S)-1-Butyl-1-(4-((4-chlorophenyl)amino)butyl)-2-((2,6-dimethylphenyl)carbamoyl)pi peridine bromide

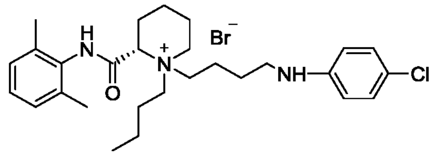

20

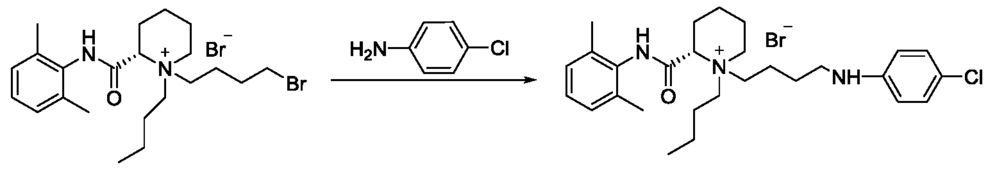

1b 20

Compound 1b (1.14 mmol, 1 eq, 0.50 g) was weighed and dissolved in analytical-grade acetonitrile (10 eq of 1b, V/m, 5.0 ml) with stirring. Analytical-grade sodium bicarbonate (1 eq of 1b, *m*/*m,* 0.50 g) and 4-chloroaniline (1.34 mmol, 1.2 eq, 0.17g) were added. The mixture was heated to 80°C for reaction and the reaction progress was monitored to be completed using TLC. The reaction mixture was filtered and the filter cake was washed with acetonitrile (30 eq of 1b, V/m, 15 ml, three times). The filtrate was collected and subjected to preparative separation to obtain a white solid, Compound 20 (0.10 g, 16% yield, HPLC > 98%).

MS m/z (ESI)=470.39$[M]^+$.

$^1$H NMR (400MHz, $CD_3OD$): δ12.01(s, 1H), 10.19(s, 1H), 7.29-7.21(m, 2H), 7.16-7.07(m, 2H), 6.99-6.92(m, 3H), 6.43(s, 1H), 4.55-4.50(t, 1H), 4.06-4.00(t, 1H), 3.40-3.23(m, 6H), 2.27-2.13(m, 6H), 1.98-1.90(m, 2H), 1.79-1.63(m, 8H), 1.44-1.32(m, 4H), 0.96-0.89(t, 3H).

$^{13}$C NMR (400 MHz, $CD_3OD$): δ13.5, 17.6, 17.7, 19.3, 19.2, 22.6, 23.9, 26.3, 29.4, 51.3, 56.7, 57.9, 66.8, 78.9, 113.7, 114.3, 120.6, 126.4, 127.3, 129.7, 130.9, 136.3, 158.3, 160.4, 170.5.

**Example 21**

(2S)-1-Butyl-1-(4-((3,4-difluorophenyl)amino)butyl)-2-((2,6-dimethylphenyl)carbamoy l)piperidine bromide

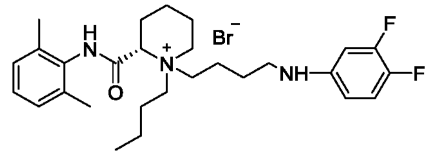

21

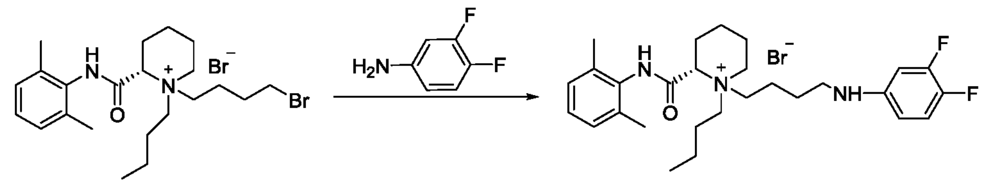

1b 21

Compound 1b (1.14 mmol, 1 eq, 0.50 g) was weighed and dissolved in analytical-grade acetonitrile (10 eq of 1b, V/m, 5.0 ml) with stirring. Analytical-grade sodium bicarbonate (1 eq of 1b, *m*/*m,* 0.50 g) and 3,4-difluoroaniline (1.34 mmol, 1.2 eq, 0.17 g) were added. The mixture was heated to 80°C for reaction and the reaction progress was monitored to be

completed using TLC. The reaction mixture was filtered and the filter cake was washed with acetonitrile (30 eq of 1b, V/m, 15 ml, three times). The filtrate was collected and subjected to preparative separation to obtain a white solid, Compound 21 (0.13 g, 21.18% yield, HPLC > 98%).

MS m/z (ESI)=472.31[M]$^+$.

$^1$H NMR (400MHz, $CD_3OD$): δ12.09(s, 1H), 10.33(s, 1H), 7.28-7.20(m, 2H), 7.15-7.09(m, 1H), 6.98-6.90(m, 3H), 6.42(s, 1H), 4.51-4.45(t, 1H), 4.31-4.26(t, 1H), 3.43-3.26(m, 6H), 2.28-2.16(m, 6H), 1.96-1.91(m, 2H), 1.79-1.60(m, 8H), 1.43-1.30(m, 4H), 0.95-0.87(t, 3H).

$^{13}$C NMR (400 MHz, $CD_3OD$): δ13.4, 15.9, 16.3, 18.9, 19.5, 23.6, 25.9, 27.3, 28.7, 51.3, 56.8, 57.4, 66.3, 78.7, 113.6, 114.6, 122.8, 124.4, 125.3, 129.6, 130.9, 136.3, 158.3, 160.4, 170.5.

**Example 22**

(2S)-1-Butyl-2-((4-fluorophenyl)carbamoyl)-1-(4-(m-tolyloxy)butyl)piperidine bromide

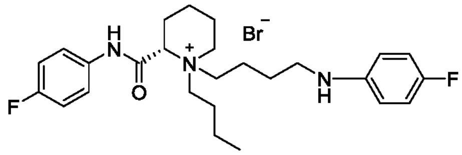

22

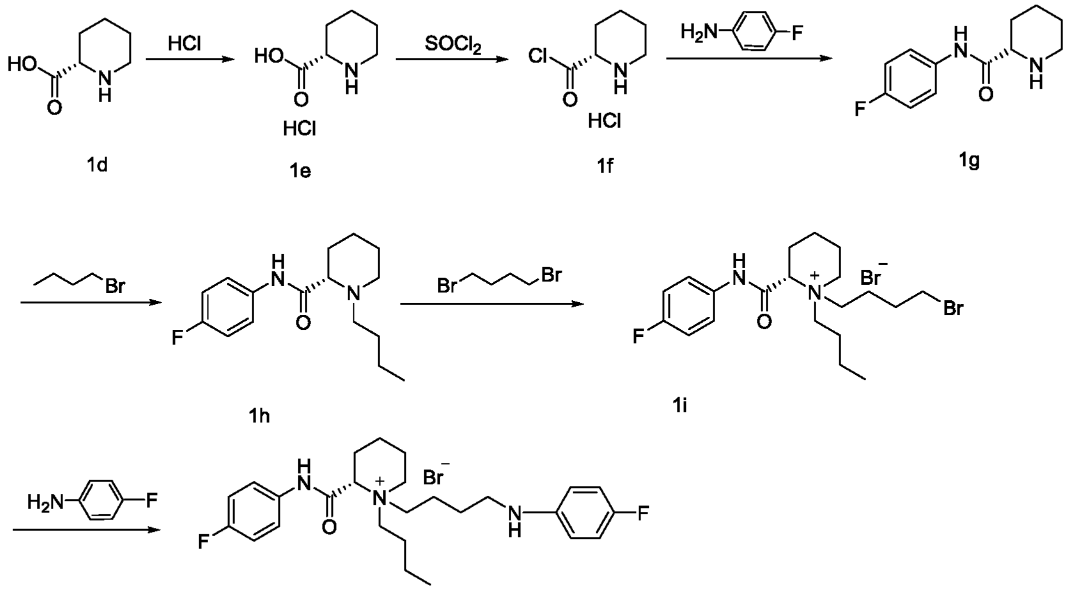


1d 1e 1f 1g

1h 1i

22

Step 1

(L)-2-Piperidinic acid 1d (7.75 mmol, 1 eq, 1 g) was weighed and dissolved in an HCl ethanol solution (20 eq of 1d, V/m, 20 ml) with stirring. The mixture was heated to 30°C and stirred for 2 h. The solvent was then removed by spinning to yield 1.28 g of white solid 1e.

Step 2

1.28 g of white solid 1e was weighed, and dichlorothionyl (10 eq of 1e, V/m, 10 ml) was added and stirred. Then, 5 drops of DMF solution were added. Once the white solid was completely dissolved, the reaction was heated to 40°C and stirred for 3.5 h. The solvent was then removed by spinning to yield 1.41 g of brown solid 1f.

Step 3

4-Fluoroaniline (15.5 mmol, 2 eq, 2.09 g) was dissolved in 10 ml of toluene. 1f (7.75 mmol, 1 eq, 1.41 g) was weighed and dissolved in toluene (10 eq of 1f, V/m, 20.0 ml) with stirring. The toluene solution of 4-fluoroaniline was added dropwise over 5 min. The reaction progress was monitored to be completed by TLC. The reaction solution was extracted with 50 ml of saturated sodium bicarbonate solution, and the pH was adjusted to 10. The organic phase was collected and concentrated by rotary evaporation. Column chromatography was performed on silica gel using an eluent system of $CH_2Cl_2$: MeOH=20:1. The eluate was collected and concentrated by rotary evaporation to yield a yellow-brown solid, 1g (0.83 g, 43.73% yield, HPLC > 98%). ESI-MS m/z: 223.13[M+1]$^+$.

Step 4

1g (3.73 mmol, 1 eq, 0.83 g) was weighed and dissolved in analytical-grade acetonitrile (10 eq of 1g, V/m, 8.0 ml) with stirring. Analytical-grade potassium carbonate (4.48 mmol, 1.2 eq, 0.57 g) and bromobutane (4.48 mmol, 1.2 eq, 0.61 g) were added. The mixture was heated to 80°C for reaction and the reaction progress was monitored to be completed using TLC. The reaction mixture was filtered and the filter cake was washed with 15 ml of acetonitrile for three times. The filtrate was collected and concentrated by rotary evaporation to yield a yellow-brown solid, 1h (0.94 g, 90.6% yield, HPLC > 98%). ESI-MS m/z: 279.3[M+1]$^+$.

Step 5

0.94 g of yellow-brown solid 1h was dissolved in 1,4-dibromobutane (20 eq of 1h, V/m, 3 ml). The mixture was heated to 100°C, and the reaction progress was monitored to be completed using TLC. Column chromatography was performed on silica gel using an eluent system of $CH_2Cl_2$: MeOH=20:1. The eluate was collected and concentrated by rotary evaporation to yield a yellow-brown solid, 1i (450 mg, 13.25% yield, HPLC > 98%). MS m/z=413.3[M]$^+$, 415.3[M+2]$^+$.

Step 6

1i (0.37 mmol, 1 eq, 0.15 g) was weighed and dissolved in analytical-grade acetonitrile (10 eq of 1i, V/m, 5.0 ml) with stirring. Analytical-grade potassium carbonate (0.44 mmol, 1.2 eq, 0.06 g) and 4-fluoroaniline (0.74 mmol, 2 eq, 0.07 g) were added. The mixture was heated to 80°C for reaction and the reaction progress was monitored to be completed using TLC. The reaction mixture was filtered and the filter cake was washed with acetonitrile (30 eq of 1i, V/m, 10 ml, three times). The filtrate was collected and subjected to preparative separation to obtain a white solid, Compound 22 (16.06 mg, 9.33% yield, HPLC > 98%).

MS m/z=444.36[M]$^+$.

$^1$H NMR (400MHz, $CD_3OD$): δ9.97(s, 1H), 7.60(d, 2H), 7.17-7.15(m, 3H), 6.78(s, 3H), 4.06(t, 2H), 2.34-2.28(m, 9H), 1.75-1.74(m, 4H), 1.36-1.30(m, 10H), 0.89(t, 3H).

$^{13}$C NMR (400 MHz, $CD_3OD$): δ13.8, 20.4, 21.6, 23.0, 24.5, 27.3, 30.4, 35.0, 36.5, 51.0, 53.0, 68.4, 111.4, 113.2, 115.7, 120.6, 126.1, 129.2, 131.5, 139.0, 157.3, 161.0, 162.9.

Example 23

(2S)-1-Butyl-2-((2,6-dimethylphenyl)carbamoyl)-1-(4-(2-isopropyl-5-methylphenoxy)b utyl)piperidine bromide

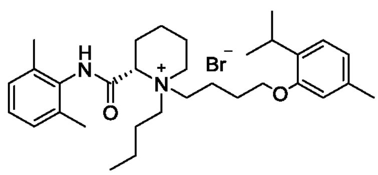

23

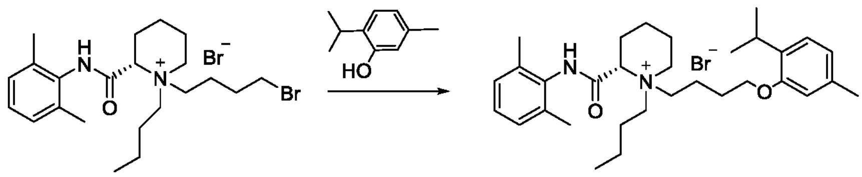

1b

23

Compound 1b (1.14 mmol, 1 eq, 0.50 g) was weighed and dissolved in analytical-grade acetonitrile (10 eq of 1b, V/m, 5.0 ml) with stirring. Analytical-grade potassium carbonate (1.2 eq of 1b, *m/m,* 0.50 g) and thymol (1.34 mmol, 1.2 eq, 0.14 g) were added. The mixture was heated to 80°C for reaction and the reaction progress was monitored to be completed using TLC. The reaction mixture was filtered and the filter cake was washed with acetonitrile (30 eq of 1b, V/m, 15 ml, three times). The filtrate was collected and subjected to preparative separation to obtain a white solid, Compound 23 (0.02 g, 2.92% yield, HPLC > 98%).

MS m/z=493.45[M]$^+$.

$^1$H NMR (400MHz, $CD_3OD$): δ10.02 (s, 1H), 7.23 (m, 1H), 7.05-6.87 (m, 4H), 6.71 (d, 1H), 4.58-4.42 (m, 1H), 4.06-3.91 (m, 2H), 3.22-3.05 (m, 7H), 2.23 (s, 3H), 2.13-1.92 (m, 6H), 1.90-1.81 (m, 2H), 1.78-1.45 (m, 8H), 1.30-1.20 (m, 10H), 0.89 (t, 3H).

$^{13}$C NMR (400 MHz, $CD_3OD$): δ13.8, 17.2, 19.0, 19.3, 21.6, 22.2, 23.1, 23.6, 26.3, 27.6, 28.1, 51.8, 56.5, 56.8, 68.7, 79.7, 111.5, 126.8, 127.7, 128.6, 129.7, 130.7, 137.1, 137.7, 149.5, 172.0.

Example 24

(2S)-1-Butyl-2-((2,6-dimethylphenyl)carbamoyl)-1-(4-(4-fluorophenoxy)butyl)piperidin e bromide

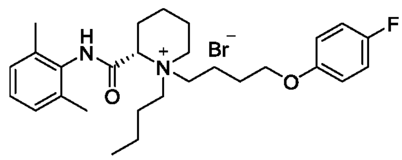

24

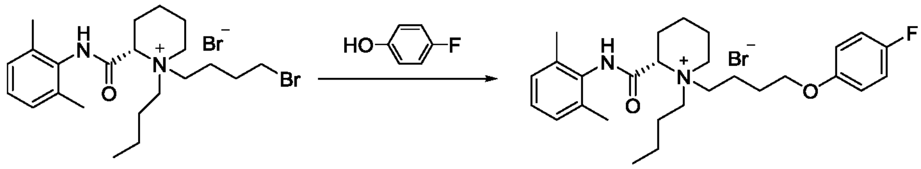

1b

24

Compound 1b (0.71 mmol, 1 eq, 0.30 g) was weighed and dissolved in analytical-grade acetonitrile (10 eq of 1b, V/m, 5.0 ml) with stirring. Analytical-grade potassium carbonate (1.2 eq of 1b, *m/m,* 0.50 g) andd p-fluorophenol (1.42 mmol, 2 eq, 0.2 g) were added. The mixture was heated to 80°C for reaction and the reaction progress was monitored to be completed using TLC. The reaction mixture was filtered and the filter cake was washed with acetonitrile (30 eq of 1b, V/m, 15 ml, three times). The filtrate was collected and subjected to preparative separation to obtain a white solid, Compound 24 (123.5 mg, 23.29% yield, HPLC > 98%).

MS m/z=455.43[M]$^+$.

$^1$H NMR (400MHz, $CD_3OD$): δ9.70(s, 1H), 7.59(d, 2H), 7.19-7.09(m, 6H), 4.06(t, 2H), 3.04(t, 1H), 2.34(t, 4H), 1.99-1.72(m, 6H), 1.55-1.30(m, 10H), 0.89(t, 3H).

$^{13}$C NMR (400 MHz, $CD_3OD$): δ13.8, 20.7, 21.5, 22.0, 23.3, 25.7, 27.6, 27.9, 59.1, 60.1, 60.4, 68.4, 78.5, 116.0, 116.1, 120.6, 134.1, 154.5, 155.0, 162.9, 172.0.

## Example 25

(2S)-1-Butyl-2-((2,6-dimethylphenyl)carbamoyl)-1-(4-(symmetrical mesityloxy) butyl)piperidine bromide

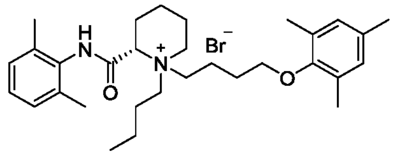

25

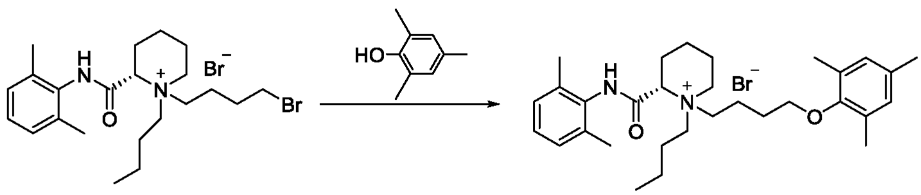

1b 25

Compound 1b (0.83 mmol, 1 eq, 0.35 g) was weighed and dissolved in analytical-grade acetonitrile (10 eq of 1b, V/m, 5.0 ml) with stirring. Analytical-grade potassium carbonate (1.2 eq of 1b, *m*/*m,* 0.50 g) and 2,4,6-trimethylphenol (1.66 mmol, 2 eq, 0.22 g) were added. The mixture was heated to 80°C for reaction and the reaction progress was monitored to be completed using TLC. The reaction mixture was filtered and the filter cake was washed with acetonitrile (30 eq of 1b, V/m, 15 ml, three times). The filtrate was collected and subjected to preparative separation to obtain a white solid, Compound 25 (176.87 mg, 31.8% yield, HPLC > 98%).

MS m/z=479.47$[M]^+$.

$^1$H NMR (400MHz, $CD_3OD$): δ9.70(s, 1H), 7.59(dd, 2H), 7.14 (t, 2H), 6.97(s, 2H), 4.06(t, 2H), 3.04(t, 1H), 2.34(t, 4H), 2.18-2.15(m, 9H), 1.99-1.72(m, 6H), 1.55-1.30(m, 10H), 0.89(t, 3H).

$^{13}$C NMR (400 MHz, $CD_3OD$): δ13.8, 16.0, 20.7, 21.9, 22.0, 23.3, 25.7, 27.6, 27.9, 59.1, 60.1, 60.4, 69.0, 78.5, 115.7, 120.6, 122.0, 129.8, 131.2, 134.1, 151.5, 162.9, 172.0.

## Example 26

(2S)-1-Butyl-2-((2,6-dimethylphenyl)carbamoyl)-1-(4-(3-fluorophenoxy)butyl)piperidin e bromide

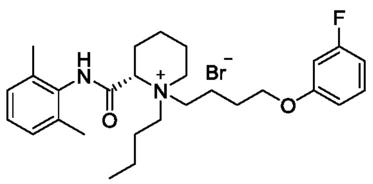

26

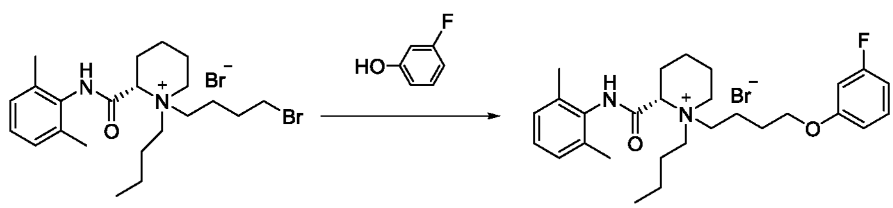

1b 26

Compound 1b (0.83 mmol, 1 eq, 0.35 g) was weighed and dissolved in analytical-grade acetonitrile (10 eq of 1b, V/m, 5.0 ml) with stirring. Analytical-grade potassium carbonate (1.2 eq of 1b, *m*/*m,* 0.50 g) and m-fluorophenol (1.66

mmol, 2 eq, 0.15 g) were added. The mixture was heated to 80°C for reaction and the reaction progress was monitored to be completed using TLC. The reaction mixture was filtered and the filter cake was washed with acetonitrile (30 eq of 1b, V/m, 15 ml, three times). The filtrate was collected and subjected to preparative separation to obtain a white solid, Compound 26 (164.81 mg, 30.7%yield, HPLC > 98%).

MS m/z=455.44[M]$^+$.

$^1$H NMR (400MHz, $CD_3OD$): δ9.70(s, 1H), 7.72(d, 1H), 7.59 (d, 2H), 7.44(td, 1H), 7.14-7.13 (m, 3H), 6.71(d, 1H), 4.06(t, 2H), 3.04(t, 1H), 2.34(t, 4H), 1.99-1.72(m, 6H), 1.55-1.30(m, 10H), 0.89(t, 3H).

$^{13}$C NMR (400 MHz, $CD_3OD$): δ13.8, 20.7, 21.5, 22.0, 23.3, 25.7, 27.6, 27.9, 59.1, 60.1, 60.4, 68.4, 78.5, 102.5, 107.1, 110.0, 115.7, 120.6, 129.6, 134.1, 159.0, 159.7, 162.9, 172.0.

### Example 27

(2S)-1-Butyl-1-(4-(3-cyanophenoxy)butyl)-2-((2,6-dimethylphenyl)carbamoyl)piperidin e bromide

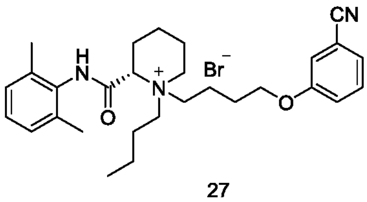

27

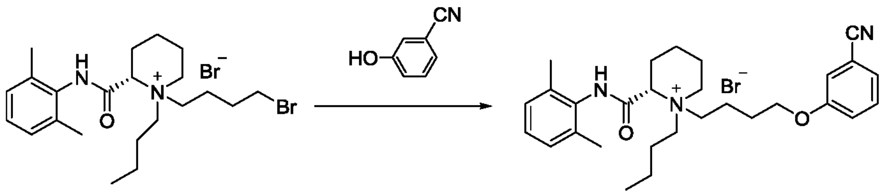

1b 27

Compound 1b (0.71 mmol, 1 eq, 0.3 g) was weighed and dissolved in analytical-grade acetonitrile (10 eq of 1b, V/m, 5.0 ml) with stirring. Analytical-grade potassium carbonate (1.2 eq of 1b, *m*/*m,* 0.50 g) and 3-hydroxybenzonitrile (1.42 mmol, 2 eq, 0.17 g) were added. The mixture was heated to 80°C for reaction and the reaction progress was monitored to be completed using TLC. The reaction mixture was filtered and the filter cake was washed with acetonitrile (30 eq of 1b, V/m, 15 ml, three times). The filtrate was collected and subjected to preparative separation to obtain a white solid, Compound 27 (101.52 mg, 22.1% yield, HPLC > 98%).

MS m/z=462.40[M]$^+$.

$^1$H NMR (400MHz, $CD_3OD$): δ10.02(s, 1H), 7.72(s, 1H), 7.60-7.53 (m, 2H), 7.22(d, 1H), 7.05 (s, 3H), 4.58(t, 1H), 4.06(t, 2H), 3.27-3.17(m, 6H), 2.17-1.92(m, 8H), 1.75-1.70(m, 8H), 1.30 -1.20(m, 4H), 0.89(t, 3H).

$^{13}$C NMR (400 MHz, $CD_3OD$): δ13.8, 17.2, 17.6, 19.0, 19.3, 22.2, 23.1, 26.3, 28.1, 51.8, 56.5, 56.8, 68.4, 79.7, 113.2, 118.6, 118.7, 123.8, 126.8, 127.7, 130.0, 130.7, 137.1, 158.1, 172.0.

### Example 28

(2S)-1-Butyl-2-((2,6-dimethylphenyl)carbamoyl)-1-(4-(3-(trifluoromethyl)phenoxy)but yl)piperidine bromide

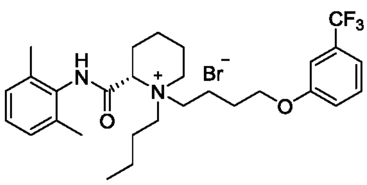

28

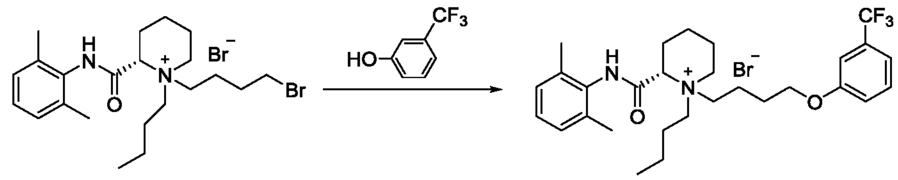


1b 28

Compound 1b (0.71 mmol, 1 eq, 0.3 g) was weighed and dissolved in analytical-grade acetonitrile (10 eq of 1b, V/m, 5.0 ml) with stirring. Analytical-grade potassium carbonate (1.2 eq of 1b, m/m, 0.50 g) and 3-trifluoromethylphenol (1.42 mmol, 2 eq, 0.26 g) were added. The mixture was heated to 80°C for reaction and the reaction progress was monitored to be completed using TLC. The reaction mixture was filtered and the filter cake was washed with acetonitrile (30 eq of 1b, V/m, 15 ml, three times). The filtrate was collected and subjected to preparative separation to obtain a white solid, Compound 28 (173.34 mg, 36.01% yield, HPLC > 98%).

MS m/z=505.40$[M]^+$.

$^1$H NMR (400MHz, $CD_3OD$): δ10.02(s, 1H), 7.25(d, 1H), 7.16-7.15 (m, 2H), 7.05(s, 3H), 6.88 (d, 1H), 4.06(t, 2H), 3.04(t, 1H), 2.34(t, 4H), 2.13(s, 6H), 1.99-1.89(m, 3H), 1.75-1. 72 (m, 3H), 1.55 -1. 30 (m, 10H), 0.89(t, 3H).

$^{13}$C NMR (400 MHz, $CD_3OD$): δ13.8, 17.6, 20.7, 21.5, 22.0, 23.3, 25.7, 27.6, 27.9, 59.1, 60.1, 60.4, 68.4, 78.5, 109.3, 116.7, 117.7, 124.4, 126.8, 127.7, 129.6, 130.7, 131.6, 137.1, 157.7, 172.0.

**Example 29**

(2R)-1-Butyl-2-((2,6-dimethylphenyl)carbamoyl)-1-(4-(m-tolyloxy)butyl)piperidine chloride

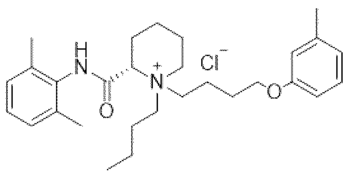

29

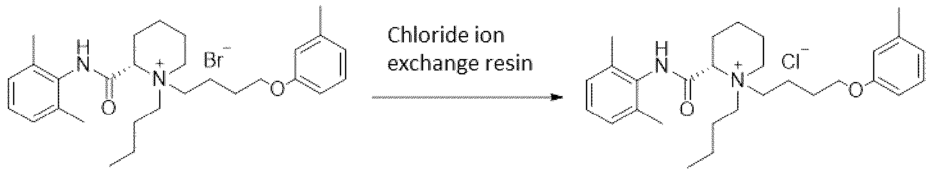


5 29

A chloride ion exchange resin was washed with pure water (20 eq of the chloride ion exchange resin, V/m, 20 ml, three times) until the pH of the aqueous phase became alkaline to yield 1 g of prepared chloride ion exchange resin. Compound 5 (0.19 mmol, 1 eq, 0.1 g) was dissolved in a solvent (10 ml) of acetonitrile: water = 1:1. The prepared chloride ion exchange resin (4 eq of Compound 5, *m/m,* 0.4 g) was added to the solution for the first time and stirred at room temperature for 0.5 h. The mixture was then filtered, and the filtrate was collected. The prepared chloride ion exchange resin (4 eq of Compound 2, m/m, 0.4 g) was added to the filtrate for the second time and stirred at room temperature for 0.5 h. The mixture was filtered, and the filtrate was collected. The prepared chloride ion exchange resin (4 eq of Compound 5, *m/m,* 0.4 g) was added to the filtrate for the third time. The mixture was filtered, and the final filtrate was collected and concentrated by rotary evaporation to yield a white solid, Compound 29 (0.39 g, 20.17% yield, HPLC > 98%).

MS m/z=451.46$[M]^+$.

$^1$H NMR (400MHz, $CD_3OD$): δ12.22 (s, 1H), 10.06 (s, 1H), 7.18-7.15 (t, 1H), 7.05 (s, 3H), 6.79-6.72 (m, 3H), 4.56 (t, 1H), 4.09-4.05 (m, 2H), 3.39-3.12 (m, 6H), 2.30 (s, 3H), 2.28-2.06 (m, 6H), 1.96-1.91 (m, 2H), 1.75-1.59 (m, 8H), 1.45-1.23 (m, 4H), 0.93 (t, 3H).

$^{13}$C NMR (400 MHz, $CD_3OD$): δ13.9, 17.5, 17.9, 19.1, 19.6, 21.8, 22.5, 23.5, 26.4, 28.5, 51.9, 56.9, 57.2, 68.9, 79.9, 111.3, 113.5, 113.9, 120.7, 126.9, 128.2, 129.5, 130.8, 137.9, 139.8, 157.9, 161.8, 172.8.

**Example 30**

(2S)-1-Butyl-2-((2,6-dimethylphenyl)carbamoyl)-1-(4-(o-tolyloxy)butyl)piperidine bromide

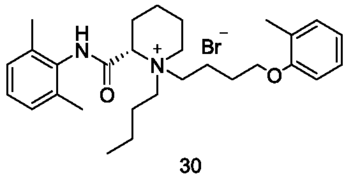

30

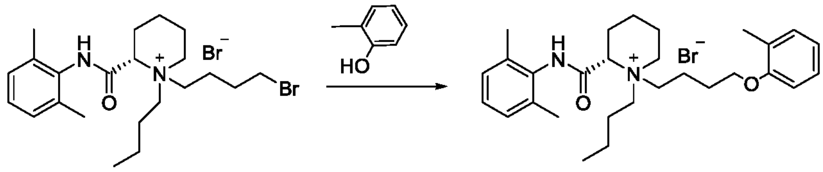

1b

30

Compound 1b (0.70 mmol, 1 eq, 0.30 g) was weighed and dissolved in analytical-grade acetonitrile (10 eq of 1b, V/m, 5.0 ml) with stirring. Analytical-grade potassium carbonate (0.84 mmol of 1b, 1.2 eq, 0.12 g) and 2-methylphenol (0.84 mmol, 1.2 eq, 0.09 g) were added. The mixture was heated to 80°C for reaction and the reaction progress was monitored to be completed using TLC. The reaction mixture was filtered and the filter cake was washed with acetonitrile (30 eq of 1b, V/m, 15 ml, three times). The filtrate was collected and subjected to preparative separation to obtain a white solid, Compound 30 (67.55 mg, 21.3% yield, HPLC > 98%).

MS m/z=451.44$[M]^+$.

$^1$H NMR (400MHz, $CD_3OD$): δ10.02(s, 1H), 7.16(d, 1H), 7.05(s, 3H), 6.93-6.85(m, 3H), 4.58 (t, 1H), 4.06(t, 2H), 3.27-3.17(m, 6H), 2.17-1.92(m, 11H), 1.75-1.71(m, 8H), 1.30-1. 20 (m, 4H), 0.89(t, 3H).

$^{13}$C NMR (400 MHz, $CD_3OD$): δ13.8, 15.4, 17.2, 17.6, 19.0, 19.3, 22.2, 23.1, 26.3, 28.1, 51.8, 56.5, 56.8, 68.7, 79.7, 112.3, 120.0, 126.3, 126.7, 126.8, 127.7, 130.7, 131.2, 137.1, 155.4, 172.0.

**Example 31**

(2S)-1-Butyl-2-((2,6-dimethylphenyl)carbamoyl)-1-(4-(p-tolyloxy)butyl)piperidine bromide

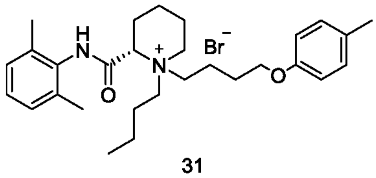

31

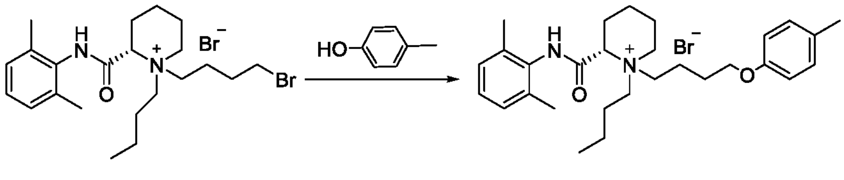

1b

31

Compound 1b (0.70 mmol, 1 eq, 0.30 g) was weighed and dissolved in analytical-grade acetonitrile (10 eq of 1b, V/m, 5.0 ml) with stirring. Analytical-grade potassium carbonate (0.84 mmol of 1b, 1.2 eq, 0.12 g) and 4-methylphenol (0.84 mmol, 1.2 eq, 0.09 g) were added. The mixture was heated to 80°C for reaction and the reaction progress was monitored to be completed using TLC. The reaction mixture was filtered and the filter cake was washed with acetonitrile (30 eq of 1b,

V/m, 15 ml, three times). The filtrate was collected and subjected to preparative separation to obtain a white solid, Compound 31 (55.12 mg, 17.4% yield, HPLC > 98%).

MS m/z=451.44[M]+.

$^1$H NMR (400MHz, $CD_3OD$): δ10.02(s, 1H), 7.10-7.05(m, 5H), 6.81(d, 2H), 4.58 (t, 1H), 4.06(t, 2H), 3.27-3.17(m, 6H), 2.25-1.92(m, 11H), 1.75-1.70(m, 8H), 1.30-1. 20 (m, 4H), 0.89(t, 3H).

$^{13}$C NMR (400 MHz, $CD_3OD$): δ13.8, 17.2, 17.6, 19.0, 19.3, 21.3, 22.2, 23.1, 26.3, 28.1, 51.8, 56.5, 56.8, 68.4, 79.7, 114.3, 126.8, 127.7, 129.6, 130.0, 130.7, 137.1, 156.4, 172.0.

The compounds in Examples 32 to 33 were synthesized using a preparation method similar to that described in Example 4.

| Compound number | Structure | Reactants | MS m/z | NMR |
|---|---|---|---|---|
| Example 32 | 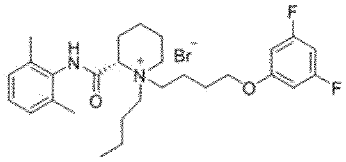 | Intermediate 1b and 3,5-difluorophenol (CAS No. 2713-34-0) | 473.52 [M]+ | $^1$H NMR(400MHz, $CD_3OD$): δ 10.02(s, 1H), 7.27-7.20(m, 2H), 7.06-6.85(m, 5H), 6.33(m, 1H), 4.58(t, 1H), 4.06(t, 2H), 3.31-3.20(m, 6H), 2.17-2.10(m. 6H), 1.75-1.68(m, 8H), 1.40-1.30(m, 4H), 0.89(t, 3H). $^{13}$C NMR (400 MHz, $CD_3OD$): δ 13.8, 16.8, 17.4, 17.6, 19.0, 19.3, 22.2, 26.3, 28.1, 51.8, 56.5, 56.8, 68.4, 79.7, 96.3, 98.1, 98.7, 126.8, 127.7, 127.9, 129.7, 130.7, 137.1, 160.6, 165.1, 170.6. |
| Example 33 | 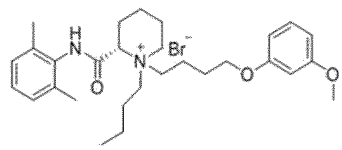 | Intermediate 1b and 3-methoxyphenol (CAS No. 150-19-6) | 467.46 [M]+ | $^1$H NMR(400MHz, $CD_3OD$): δ 10.00(s, 1H), 7.39-7.05(m, 4H), 6.62-6.39(m, 3H), 4.49(m, 1H), 4.06(m, 2H), 3.84(t, 3H), 3.45-3.22(m, 6H), 1.70-1.62(m, 8H), 1.60-1.46(m, 8H), 1.40-1.36(m. 4H), 0.75(t, 3H). $^{13}$C NMR (400 MHz, $CD_3OD$): δ 13.9, 14.6, 17.3, 17.4, 17.5, 19.0, 19.3, 22.2, 26.3, 28.1, 51.8, 56.5, 56.8, 68.4, 79.7, 96.3, 98.1, 98.7, 126.8, 127.4, 127.7, 130.7, 130.9, 137.1, 160.6, 165.1, 165.8. |

**Example** 34

(2S)-1-Butyl-2-((2,6-dimethylphenyl)carbamoyl)-1-(3-phenoxypropyl)piperidine bromide

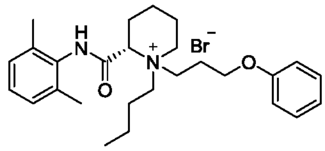

34

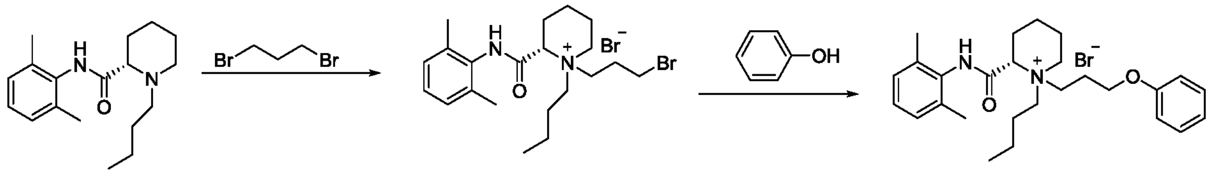

1a 1r 34

Step 1

Levobupivacaine 1a (14.35 mmol, 1.0 eq, 3.0 g) was weighed and added with 1,3-dibromopropane (2.0 eq of 1a, V/m, 10 ml). The mixture was stirred and heated to 100°C. The reaction progress was monitored to be completed using TLC. Column chromatography was performed on silica gel using an eluent system of $CH_2Cl_2$: MeOH=20:1. The eluate was collected and concentrated by rotary evaporation to yield an orange-yellow oily liquid, Compound 1r (2.8g, 59.9% yield, HPLC > 90%). ESI-MS m/z: 409.18[M]$^+$, 411.28[M+2H]$^+$.

Step 2

Compound 1r (0.68 mmol, 1 eq, 0.28 g) was weighed and dissolved in analytical-grade acetonitrile (10 eq of 1r, V/m, 5.0 ml) with stirring. Analytical-grade sodium bicarbonate (1 eq of 1r, *m*/*m,* 0.50 g) and phenol (1.36 mmol, 1.2 eq, 0.14 g) were added. The mixture was heated to 80°C for reaction and the reaction progress was monitored to be completed using TLC. The reaction mixture was filtered and the filter cake was washed with acetonitrile (30 eq of 1r, V/m, 15 ml, three times). The filtrate was collected and subjected to preparative separation to obtain a white solid, Compound 34 (0.33 g, 45% yield, HPLC > 98%).

MS m/z=423.46[M]$^+$.

$^1$H NMR (400MHz, $CD_3OD$): δ 11.32(s, 1H), 10.03(s, 1H), 7.28-7.21(m, 2H), 7.19-7.09(m, 3H), 6.99-6.98(m, 3H), 4.51-4.58(t, 1H), 4.01-3.97(t, 1H), 3.49-3.11(m, 4H), 2.25-2.10(m, 6H), 1.98-1.91(m, 2H), 1.79-1.68(m, 8H), 1.49-1.36(m, 4H), 0.94-0.89(t, 3H).

$^{13}$C NMR (400 MHz, $CD_3OD$): δ 13.7, 17.4, 17.9, 19.4, 19.9, 22.7, 23.4, 26.5, 28.8, 54.3, 55.9, 69.8, 78.9, 113.1, 114.7, 121.7, 125.4, 127.5, 129.5, 130.3, 136.3, 158.3, 162.6, 173.2.

Example 35

(2S)-1-Butyl-2-((2,6-dimethylphenyl)carbamoyl)-1-(3-(m-tolyloxy)propyl)piperidine bromide

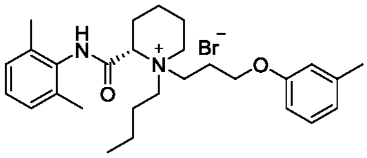

35

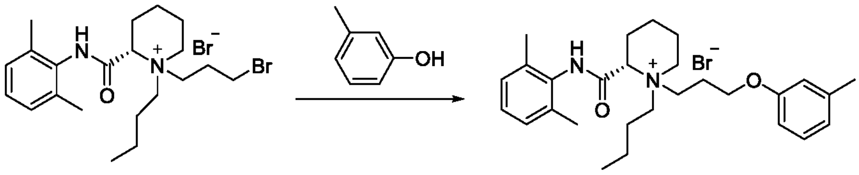

1r 35

Compound 1r (0.68 mmol, 1 eq, 0.28 g) was weighed and dissolved in analytical-grade acetonitrile (10 eq of 1r, V/m, 5.0 ml) with stirring. Analytical-grade sodium bicarbonate (1 eq of 1r, *m/m,* 0.50 g) and m-cresol (1.36 mmol, 1.2 eq, 0.14 g) were added. The mixture was heated to 80°C for reaction and the reaction progress was monitored to be completed using TLC. The reaction mixture was filtered and the filter cake was washed with acetonitrile (30 eq of 1r, V/m, 15 ml, three times). The filtrate was collected and subjected to preparative separation to obtain a white solid, Compound 35 (14.5 mg, 6% yield, HPLC > 98%).

MS m/z=437.43[M]$^+$.

$^1$H NMR (400MHz, $CD_3OD$): δ 11.49(s, 1H), 10.11(s, 1H), 7.32-7.27(m, 2H), 7.22-7.09(m, 2H), 6.99-6.98(m, 3H), 4.51-4.58(t, 1H), 4.23-3.99(t, 1H), 3.50-3.28(m, 4H), 2.25-2.10(m, 6H), 2.01-1.99(m, 3H), 1.95-1.90(m, 2H), 1.77-1.66(m, 8H), 1.44-1.33(m, 4H), 0.99-0.87(t, 3H).

$^{13}$C NMR (400 MHz, $CD_3OD$): δ 13.8, 17.5, 17.8, 19.5, 19.8, 21.5, 22.8, 23.5, 26.7, 28.4, 54.5, 55.2, 69.6, 78.2, 113.8, 114.7, 121.7, 125.8, 127.3, 129.8, 130.1, 136.9, 158.2, 162.8, 173.6.

Example 36

(2S)-1-(Cyclopentylmethyl)-2-((2,6-dimethylphenyl)carbamoyl)-1-(4-phenoxybutyl)pip eridine bromide

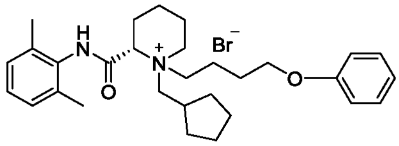

36

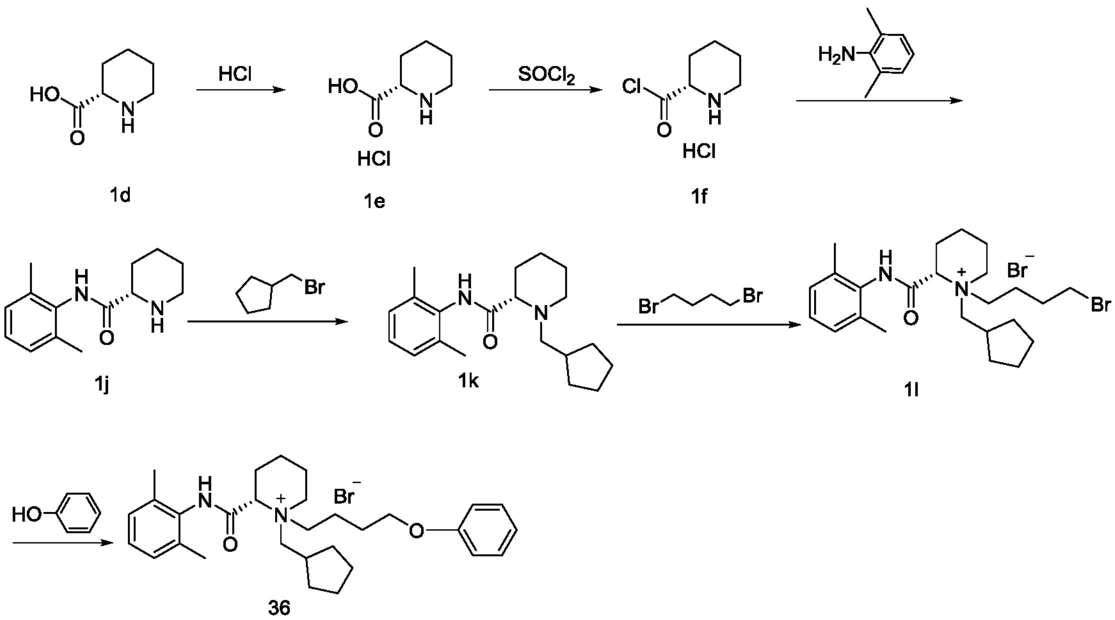


1d 1e 1f

1j 1k 1l

36

Step 1

(L)-2-Piperidinic acid 1d (7.75 mmol, 1 eq, 1 g) was weighed and dissolved in an HCl ethanol solution (20 eq of 1d, V/m, 20 ml) with stirring. The mixture was heated to 30°C and stirred for 2 h. The solvent was then removed by spinning to yield 1.28 g of white solid 1e.

Step 2

1.28 g of white solid 1e was weighed and dissolved in dichlorothionyl (10 eq of 1e, V/m, 10 ml). Then, 5 drops of DMF solution were added. Once the white solid was completely dissolved, the reaction was heated to 40°C and stirred for 3.5 h. The solvent was then removed by spinning to yield 1.41 g of brown solid 1f.

Step 3

2,6-Dimethylaniline (15.5 mmol, 2 eq, 2.09 g) was dissolved in 10 ml of toluene. 1f (7.75 mmol, 1 eq, 1.41 g) was weighed and dissolved in toluene (10 eq of 1f, V/m, 20.0 ml) with stirring. The toluene solution of 2,6-dimethylaniline was added dropwise over 5 min. The reaction progress was monitored to be completed by TLC. The reaction solution was extracted with 50 ml of saturated sodium bicarbonate solution, and the pH was adjusted to 10. The organic phase was collected and concentrated by rotary evaporation. Column chromatography was performed on silica gel using an eluent system of $CH_2Cl_2$: MeOH=20:1. The eluate was collected and concentrated by rotary evaporation to yield a yellow-brown solid, 1j (0.83 g, 43.73% yield, HPLC > 98%). ESI-MS m/z: 233.2$[M+1]^+$.

Step 4

1j (3.73 mmol, 1 eq, 0.83 g) was weighed and dissolved in analytical-grade acetonitrile (10 eq of 1j, V/m, 8.0 ml) with stirring. Analytical-grade potassium carbonate (4.48 mmol, 1.2 eq, 0.57 g) and bromomethylcyclopentane (4.48 mmol, 1.2 eq, 0.61 g) were added. The mixture was heated to 80°C for reaction and the reaction progress was monitored to be completed using TLC. The reaction mixture was filtered and the filter cake was washed with 15 ml of acetonitrile for three times. The filtrate was collected and concentrated by rotary evaporation to yield a yellow-brown solid, 1k (1.35 g, 90.6% yield, HPLC > 98%). ESI-MS m/z: 315.2$[M+1]^+$.

Step 5

1.35 g of yellow-brown solid 1k was dissolved in 1,4-dibromobutane (20 eq of 1h, V/m, 3 ml). The mixture was heated to 100°C for reaction, and the reaction progress was monitored to be completed using TLC. Column chromatography was performed on silica gel using an eluent system of $CH_2Cl_2$: MeOH=20:1. The eluate was collected and concentrated by rotary evaporation to yield a yellow-brown solid, 11 (1.33 g, 13.25% yield, HPLC > 98%). MS m/z=449.35 $[M]^+$, 451.32$[M+2]^+$.

Step 6

11 (0.7 mmol, 1 eq, 0.33 g) was weighed and dissolved in analytical-grade acetonitrile (10 eq of 11, V/m, 5.0 ml) with stirring. Analytical-grade potassium carbonate (0.84 mmol, 1.2 eq, 0.12 g) and phenol (1.4 mmol, 2 eq, 0.13 g) were added. The mixture was heated to 80°C for reaction and the reaction progress was monitored to be completed using TLC. The reaction mixture was filtered and the filter cake was washed with acetonitrile (30 eq of 11, V/m, 10 ml, three times). The filtrate was collected and subjected to preparative separation to obtain a white solid, Compound 36 (41.47 mg, 10.63% yield, HPLC > 98%).

MS m/z=463.33$[M]^+$.

$^1$H NMR (400MHz, $CD_3OD$): δ10.02(s, 1H), 7.27(dd, 2H), 7.05 (s, 3H), 6.93-6.92(m, 3H), 4.58 (t, 1H), 4.06(t, 2H), 3.27-3.16(m, 6H), 2.13-1.92(m, 9H), 1.90-1.63(m, 14H), 1.30-1. 20 (m, 2H).

$^{13}$C NMR (400 MHz, $CD_3OD$): δ17.2, 17.6, 19.3, 22.2, 24.8, 26.3, 28.1, 29.1, 34.9, 52.1, 57.2, 62.2, 68.4, 80.0, 114.4, 120.3, 126.8, 127.7, 129.3, 130.7, 137.1, 159.4, 172.0.

**Example 37**

(2S)-1-(Isobutylmethyl)-2-((2,6-dimethylphenyl)carbamoyl)-1-(4-phenoxybutyl)piperid ine bromide

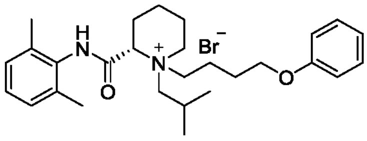

37

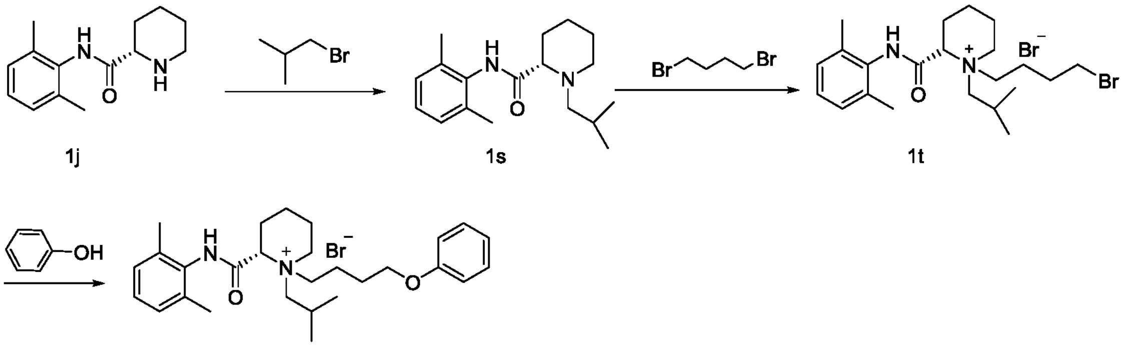

1j 1s 1t

37

Step 1

1j (4.3 mmol, 1 eq, 1 g) was weighed and dissolved in 10.0 ml of analytical-grade acetonitrile with stirring. Analytical-grade potassium carbonate (5.16 mmol, 1.2 eq, 0.71 g) and isobutyl bromide (8.6 mmol, 2 eq, 1.16 g) were added. The mixture was heated to 80°C for reaction and the reaction progress was monitored to be completed using TLC. The reaction mixture was filtered and the filter cake was washed with 15 ml of acetonitrile for three times. The filtrate was collected and concentrated by rotary evaporation to yield a yellow-brown solid, 1s (1.23 g, 97% yield, HPLC > 98%). ESI-MS m/z:289.32$[M+1]^+$.

Step 2

1.23 g of yellow-brown solid 1s was dissolved in 3 ml of 1,4-dibromobutane. The mixture was heated to 100°C, and the reaction progress was monitored to be completed using TLC. Column chromatography was performed on silica gel using an eluent system of $CH_2Cl_2$: MeOH=20:1. The eluate was collected and concentrated by rotary evaporation to yield a yellow-brown solid, 1t (0.18 g, 10% yield, HPLC > 98%). ESI-MS m/z: 423.35$[M]^+$,423.32$[M+2]^+$

Step 6

1t (0.43 mmol, 1 eq, 0.18 g) was weighed and dissolved in analytical-grade acetonitrile (10 eq of 1t, V/m, 3.0 ml) with stirring. Analytical-grade potassium carbonate (0.51 mmol, 1.2 eq, 0.07 g) and phenol (0.86 mmol, 2 eq, 0.7 g) were added. The mixture was heated to 80°C for reaction and the reaction progress was monitored to be completed using TLC. The reaction mixture was filtered and the filter cake was washed with acetonitrile (30 eq of 1t, V/m, 10 ml, three times). The filtrate was collected and subjected to preparative separation to obtain a white solid, Compound 37 (47.97 mg, 12.3% yield, HPLC > 98%).

MS m/z=437.42 $[M]^+$

$^1$H NMR (400MHz, $CD_3OD$): δ11.33(s, 1H), 10.02(s, 1H), 7.33-7.26(m, 2H), 7.19-7.05(m, 3H), 6.99-6.90(m, 3H), 4.57-4.50(t, 1H), 4.19-4.09(t, 1H), 3.46-3.21(m, 5H), 2.24-2.19(m, 6H), 1.99-1.88(m, 2H), 1.76-1.65(m, 8H), 1.47-1.33(m, 4H), 0.98-0.87(t, 3H).

$^{13}$C NMR (400 MHz, CD3OD): δ14.2, 16.9, 18.9, 19.4, 19.9, 23.5, 23.9, 25.9, 27.6, 52.2, 55.9, 57.8, 67.6, 78.5, 114.6, 114.9, 121.6, 125.9, 128.5, 129.9, 131.9, 139.3, 158.6, 161.1, 171.1.

**Example 38**

(2S)-2-((2,6-Dimethylphenyl)carbamoyl)-1-(4-methylpentyl)-1-(4-phenoxybutyl)piperi dine bromide

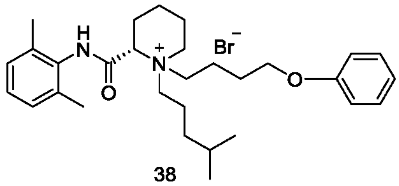

38

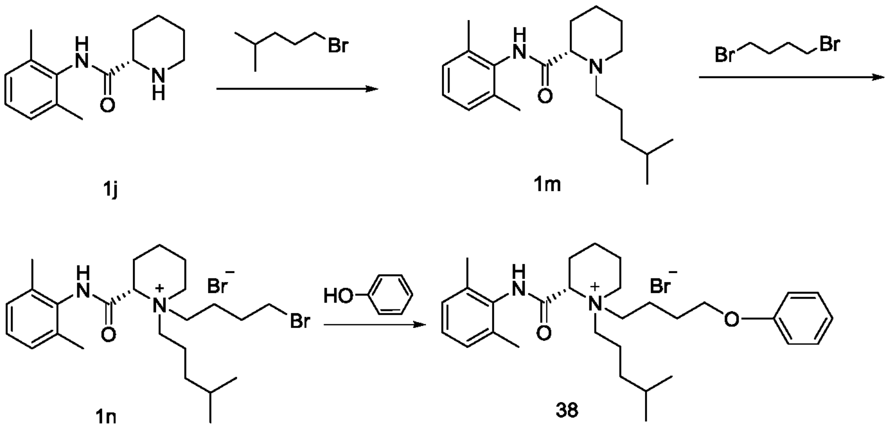

1j

1m

1n

38

Step 1

1j (1.8 mmol, 1 eq, 0.42 g) was weighed and dissolved in analytical-grade acetonitrile (10 eq of 1j, V/m, 8.0 ml) with stirring. Analytical-grade potassium carbonate (2.16 mmol, 1.2 eq, 0.3 g) and isopentyl bromide (4.48 mmol, 1.2 eq, 1.41 g) were added. The mixture was heated to 80°C for reaction and the reaction progress was monitored to be completed using TLC. The reaction mixture was filtered and the filter cake was washed with 15 ml of acetonitrile for three times. The filtrate was collected and concentrated by rotary evaporation to yield a yellow-brown solid, 1m (569 mg, 59.6% yield, HPLC > 98%). ESI-MS m/z:317.34$[M+1]^+$.

Step 2

569 mg of yellow-brown solid 1m was dissolved in 1,4-dibromobutane (20 eq of 1m, V/m, 3 ml). The mixture was heated to 100°C, and the reaction progress was monitored to be completed using TLC. Column chromatography was performed on silica gel using an eluent system of $CH_2Cl_2$: MeOH=20:1. The eluate was collected and concentrated by rotary evaporation to yield a yellow-brown solid, 1n (0.51 g, 62.96% yield, HPLC > 98%). 451.35$[M]^+$, 453.35$[M+2]^+$.

Step 3

1n (0.55 mmol, 1 eq, 0.25 g) was weighed and dissolved in analytical-grade acetonitrile (10 eq of 1n, V/m, 5.0 ml) with stirring. Analytical-grade potassium carbonate (1.1 mmol, 1.2 eq, 0.15 g) and phenol (1.1 mmol, 2 eq, 0.1 g) were added. The mixture was heated to 80°C for reaction and the reaction progress was monitored to be completed using TLC. The reaction mixture was filtered and the filter cake was washed with acetonitrile (30 eq of 1n, V/m, 10 ml, three times). The filtrate was collected and subjected to preparative separation to obtain a white solid, Compound 38 (62.69 mg, 20.42% yield, HPLC > 98%).

MS m/z=465.48$[M]^+$.

$^1$H NMR (400MHz, $CD_3OD$): δ10.02(s, 1H), 7.27(dd, 2H), 7.05(s, 3H), 6.93-6.92(m, 3H), 4.58 (t, 1H), 4.06(t, 2H), 3.27-3.17(m, 6H), 2.17-1.92(m, 8H), 1.75-1.62(m, 9H), 1.30-1. 19 (m, 4H), 0.91(d, 6H).

$^{13}$C NMR (400 MHz, $CD_3OD$): δ17.2, 17.6, 18.1, 19.3, 22.2, 23.2, 26.3, 27.8, 28.1, 38.1, 51.8, 56.8, 57.1, 68.4, 79.7, 114.4, 120.3, 126.8, 127.7, 129.3, 130.7, 137.1, 159.4, 172.0.

**Example 39**

(2S)-1-Butyl-2-((4-fluorophenyl) carbamoyl)-1-(4-phenoxybutyl)piperidine bromide

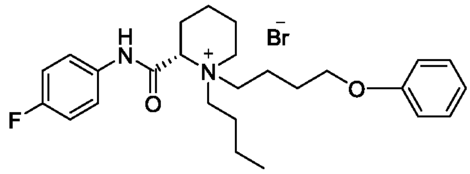

39

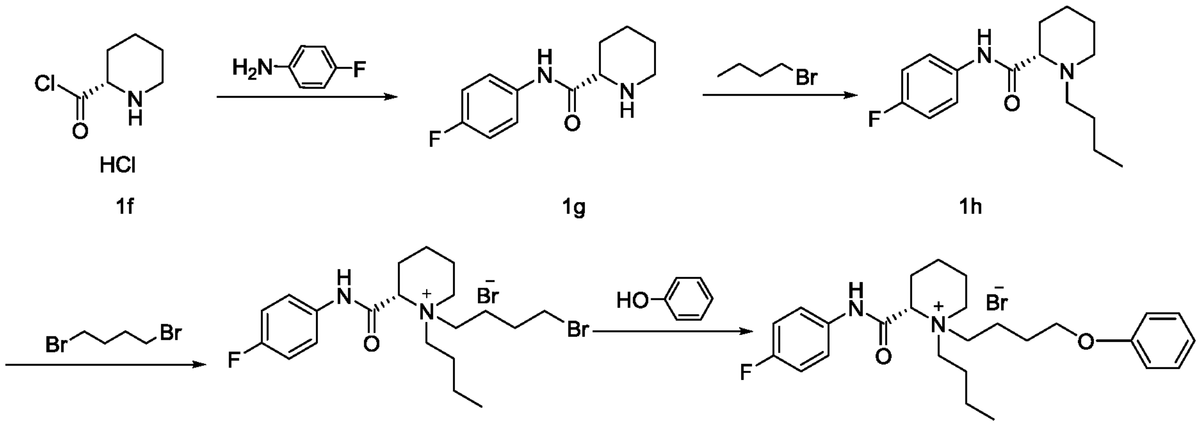

HCl

1f 1g 1h

1i 39

Step 1

4-Fluoroaniline (15.5 mmol, 2 eq, 2.09 g) was dissolved in 10 ml of toluene. 1f (7.75 mmol, 1 eq, 1.41 g) was weighed and dissolved in toluene (10 eq of 1f, V/m, 20.0 ml) with stirring. The toluene solution of 4-fluoroaniline was added dropwise over 5 min. The reaction progress was monitored to be completed by TLC. The reaction solution was extracted with 50 ml of saturated sodium bicarbonate solution, and the pH was adjusted to 10. The organic phase was collected and concentrated by rotary evaporation. Column chromatography was performed on silica gel using an eluent system of $CH_2Cl_2$: MeOH=20:1. The eluate was collected and concentrated by rotary evaporation to yield a yellow-brown solid, 1g (0.83 g, 43.73% yield, HPLC > 98%). ESI-MS m/z:222.3$[M+1]^+$.

Step 2

1g (3.73 mmol, 1 eq, 0.83 g) was weighed and dissolved in analytical-grade acetonitrile (10 eq of 1g, V/m, 8.0 ml) with stirring. Analytical-grade potassium carbonate (4.48 mmol, 1.2 eq, 0.57 g) and bromobutane (4.48 mmol, 1.2 eq, 0.61 g) were added. The mixture was heated to 80°C for reaction and the reaction progress was monitored to be completed using TLC. The reaction mixture was filtered and the filter cake was washed with 15 ml of acetonitrile for three times. The filtrate was collected and concentrated by rotary evaporation to yield a yellow-brown solid, 1h (0.94 g, 90.6% yield, HPLC > 98%). ESI-MS m/z: 279.3$[M+1]^+$.

Step 3

0.94 g of yellow-brown solid 1h was dissolved in 1,4-dibromobutane (20 eq of 1h, V/m, 3 ml). The mixture was heated to 100°C, and the reaction progress was monitored to be completed using TLC. Column chromatography was performed on silica gel using an eluent system of $CH_2Cl_2$: MeOH=20:1. The eluate was collected and concentrated by rotary evaporation to yield a yellow-brown solid, 1i (450 mg, 13.25% yield, HPLC > 98%). MS m/z=413.3$[M]^+$, 415.3$[M+2]^+$.

Step 4

1i (0.37 mmol, 1 eq, 0.15 g) was weighed and dissolved in analytical-grade acetonitrile (10 eq of 1i, V/m, 5.0 ml) with stirring. Analytical-grade potassium carbonate (0.44 mmol, 1.2 eq, 0.06 g) and phenol (0.74 mmol, 2 eq, 0.07 g) were added. The mixture was heated to 80°C for reaction and the reaction progress was monitored to be completed using TLC. The reaction mixture was filtered and the filter cake was washed with acetonitrile (30 eq of 1i, V/m, 10 ml, three times). The filtrate was collected and subjected to preparative separation to obtain a white solid, Compound 39 (33.48 mg, 2.92% yield, HPLC > 98%).

MS m/z=427.41$[M]^+$.

$^1H$ NMR (101 MHz, $CD_3OD$): δ7.66-7.54 (m, 2H), 7.33-7.19 (m, 2H), 7.17-7.02 (m, 2H), 7.02-6.79 (m, 3H), 4.33-4.21 (m, 1H), 4.20-3.98 (m, 3H), 3.94-3.40 (m, 5H), 2.48-2.34 (m, 1H), 2.32-2.17 (m, 1H), 2.11-1.69 (m, 10H), 1.53-1.23 (m, 3H), 1.10-0.86 (m, 3H).

$^{13}C$ NMR (101 MHz, $CD_3OD$): δ 11.8, 12.4, 18.5, 19.0, 19.3, 23.2, 23.4, 23.7, 25.6, 47.1, 47.3, 47.6, 47.8, 48.0, 48.2, 56.7, 66.3, 67.9, 114.0, 115.1, 115.3, 120.5, 120.6, 121.9, 122.0, 129.0, 129.1, 133.4, 158.6, 164.7.

**Example 40**

(2S)-1-Butyl-2-((2,6-dibromophenyl)carbamoyl)-1-(4-phenoxybutyl)piperidine bromide

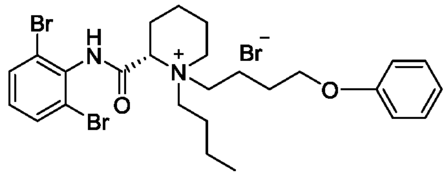

40

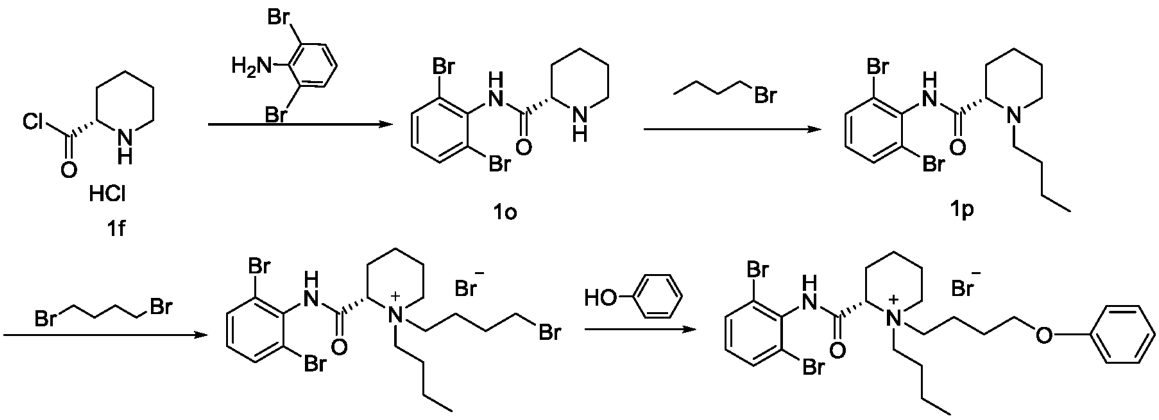

1f 1o 1p 1q 40

Step 1

2,6-Dibromoaniline (43.6 mmol, 2 eq, 1.89 g) was dissolved in 10 ml of toluene. 1f (21.8 mmol, 1 eq, 3.32 g) was weighed and dissolved in toluene (10 eq of 1f, V/m, 20.0 ml) with stirring. The toluene solution of 2,6-dibromoaniline was added dropwise over 5 min. The reaction progress was monitored to be completed by TLC. The reaction solution was extracted with 50 ml of saturated sodium bicarbonate solution, and the pH was adjusted to 10. The organic phase was collected and concentrated by rotary evaporation. Column chromatography was performed on silica gel using an eluent system of $CH_2Cl_2$: MeOH=20:1. The eluate was collected and concentrated by rotary evaporation to yield a yellow-brown solid, 1o (3.2 g, 74% yield, HPLC > 98%). ESI-MS m/z: 363.04$[M+4]^+$.

Step 2

1o (8.81 mmol, 1 eq, 3.2 g) was weighed and dissolved in analytical-grade acetonitrile (10 eq of 1o, V/m, 30 ml)

with stirring. Analytical-grade potassium carbonate (2.16 mmol, 1.2 eq, 0.3 g) and n-butyl bromide (4.48 mmol, 1.2 eq, 1.41 g) were added. The mixture was heated to 80°C for reaction and the reaction progress was monitored to be completed using TLC. The reaction mixture was filtered and the filter cake was washed with 15 ml of acetonitrile for three times. The filtrate was collected and concentrated by rotary evaporation to yield a yellow-brown solid, 1p (3.6 g, 97% yield, HPLC > 98%). ESI-MS m/z:418.01[M+2]$^+$.

Step 3

The yellow-brown solid 1p (8.65 mmol, 1 eq, 3.6 g) was dissolved in 1,4-dibromobutane (18 mmol, 20 eq, 9 ml). The mixture was heated to 100°C for reaction, and the reaction progress was monitored to be completed using TLC. Column chromatography was performed on silica gel using an eluent system of $CH_2Cl_2$: MeOH=20:1. The eluate was collected and concentrated by rotary evaporation to yield a yellow-brown solid, 1q (1.5 g, 16% yield, HPLC > 98%). 554.99 [M+4]$^+$.

Step 4

1q (0.79 mmol, 1 eq, 0.5 g) was weighed and dissolved in analytical-grade acetonitrile (10 eq of 1q, V/m, 5.0 ml) with stirring. Analytical-grade potassium carbonate (0.95 mmol, 1.2 eq, 0.13 g) and phenol (1.58 mmol, 2 eq, 0.14 g) were added. The mixture was heated to 80°C for reaction and the reaction progress was monitored to be completed using TLC. The reaction mixture was filtered and the filter cake was washed with acetonitrile (30 eq of 1q, V/m, 10 ml, three times). The filtrate was collected and subjected to preparative separation to obtain a white solid, Compound 40 (44.91 mg, 8.72% yield, HPLC > 98%).

MS m/z=567.13[M+2]$^+$.

$^1$H NMR (400MHz, $CD_3OD$): δ10.02(s, 1H), 7.74(d, 2H), 7.27(t, 2H), 6.93-6.92 (m, 3H), 6.79(t, 1H), 4.58(t, 1H), 4.06(t, 2H), 3.27-3.17(m, 6H), 2.17-1.92 (m, 2H), 1.75-1.70 (m, 8H), 1.30-1.20 (m, 4H), 0.89(t, 3H).

$^{13}$C NMR (400 MHz, $CD_3OD$): δ13.8, 17.2, 19.0, 19.3, 22.2, 23.1, 26.3, 28.1, 51.8, 56.5, 56.8, 68.4, 79.7, 114.3, 120.3, 124.8, 129.3, 130.8, 147.4, 159.4, 172.0.

**Example 41**

(2S)-1-Butyl-2-(o-tolylcarbamoyl)-1-(4-(m-tolyloxy) butyl)piperidine bromide

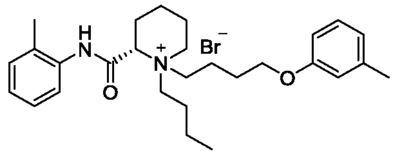

41

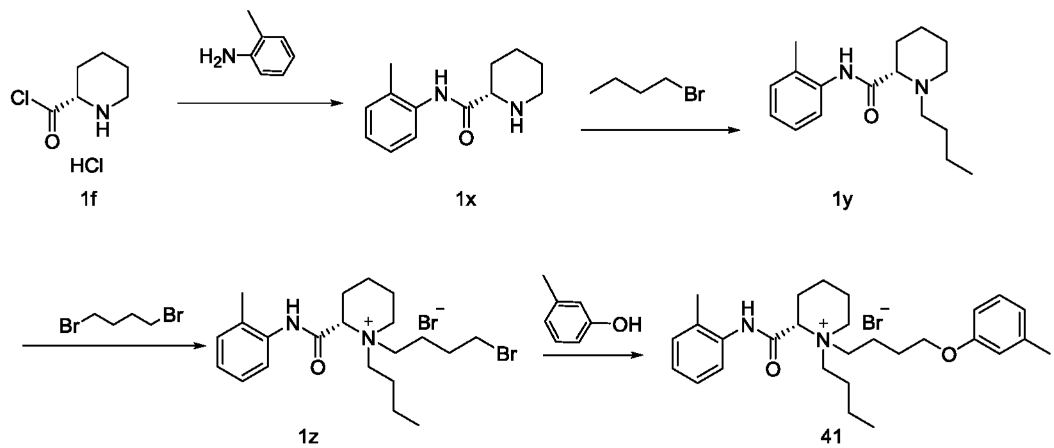

1f 1x 1y

1z 41

Step 1

2-Methylaniline (36.2 mmol, 2 eq, 3.8 g) was dissolved in 10 ml of toluene. 1f (18.1 mmol, 1 eq, 3.32 g) was weighed and dissolved in toluene (10 eq of 1f, V/m, 20.0 ml) with stirring. The toluene solution of 2-methylaniline was added dropwise over 5 min. The reaction progress was monitored to be completed by TLC. The reaction solution was extracted with 50 ml of saturated sodium bicarbonate solution, and the pH was adjusted to 10. The organic phase was collected and concentrated by rotary evaporation. Column = chromatography was performed on silica gel using an eluent system of $CH_2Cl_2$: MeOH=20:1. The eluate was collected and concentrated by rotary evaporation to yield a yellow-brown solid, 1x (1.11 g, 28.02% yield, HPLC > 98%). ESI-MS m/z: 219.35[M+1]$^+$.

Step 2

1x (5.06 mmol, 1 eq, 1.11 g) was weighed and dissolved in analytical-grade acetonitrile (10 eq of 1x, V/m, 11.0 ml) with stirring. Analytical-grade potassium carbonate (6.07 mmol, 1.2 eq, 1 g) and n-butyl bromide (6.07 mmol, 1.2 eq, 1 g) were added. The mixture was heated to 80°C for reaction and the reaction progress was monitored to be completed using TLC. The reaction mixture was filtered and the filter cake was washed with 15 ml of acetonitrile for three times. The filtrate was collected and concentrated by rotary evaporation to yield a yellow-brown solid, 1y (1.39 g, 99% yield, HPLC > 98%). ESI-MS m/z: 275.28[M+1]$^+$.

Step 3

1.39 g of yellow-brown solid 1y was dissolved in 1,4-dibromobutane (20 eq of 1y, V/m, 3 ml). The mixture was heated to 100°C for reaction, and the reaction progress was monitored to be completed using TLC. Column chromatography was performed on silica gel using an eluent system of $CH_2Cl_2$: MeOH=20:1. The eluate was collected and concentrated by rotary evaporation to yield a yellow-brown solid, 1z (1.14 g, 21.8% yield, HPLC > 98%). ESI-MS m/z: 409.34[M]$^+$, 411.35[M+2]$^+$.

Step 4

1z (0.9 mmol, 1 eq, 0.38 g) was weighed and dissolved in analytical-grade acetonitrile (10 eq of 1z, V/m, 4.0 ml) with stirring. Analytical-grade potassium carbonate (1.08 mmol, 1.2 eq, 0.14 g) and m-cresol (1.8 mmol, 2 eq, 0.16 g) were added. The mixture was heated to 80°C for reaction and the reaction progress was monitored to be completed using TLC. The reaction mixture was filtered and the filter cake was washed with acetonitrile (30 eq of 1z, V/m, 10 ml, three times). The filtrate was collected and subjected to preparative separation to obtain a white solid, Compound 41 (84.9 mg, 17.65% yield, HPLC > 98%).

MS m/z=437.47 [M]$^+$

$^1$H NMR (400MHz, $CD_3OD$): δ12.10(s, 1H), 10.31(s, 1H), 7.19-7.19(m, 2H), 7.16-7.11(m, 3H), 6.98-6.89(m, 3H), 4.59-4.50(t, 1H), 4.09-3.98(t, 1H), 3.45-3.28(m, 6H), 2.23-2.12(m, 6H), 1.99-1.88(m, 2H), 1.81-1.67(m, 8H), 1.49-1.39(m, 4H), 0.96-0.84(t, 3H).

$^{13}$C NMR (400 MHz, $CD_3OD$): δ13.,7, 17.2, 17.6, 19.4, 19.9, 22.6, 23.8, 26.4, 28.0, 51.5, 56.6, 56.1, 68.1, 79.6, 113.7, 114.6, 120.1, 126.3, 127.3, 129.6, 130.1, 137.7, 159.1, 161.7, 171.5.

Example 42

(2S)-1-Butyl-2-((2,6-diethylphenyl)carbamoyl)-1-(4-(m-tolyloxy)butyl)piperidine bromide

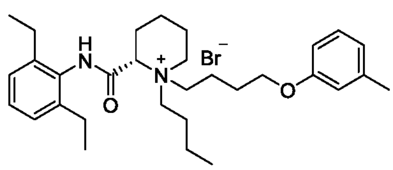

42

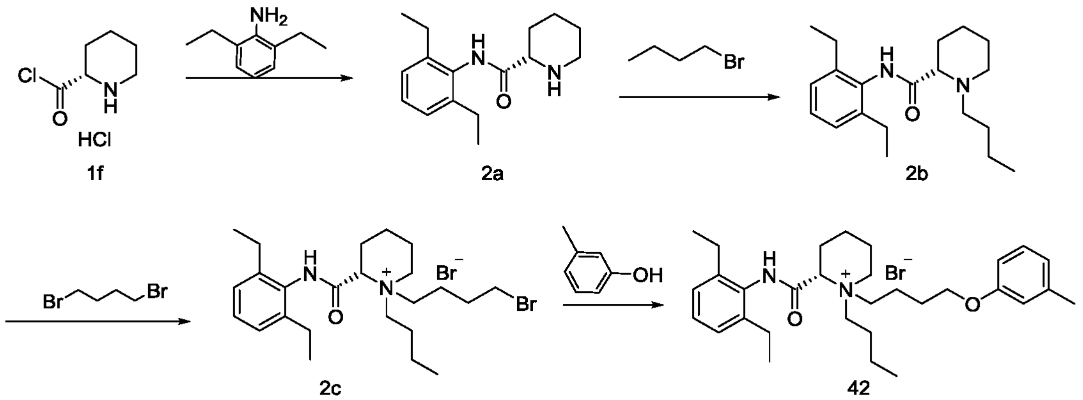

1f 2a 2b

2c 42

Step 1

2,6-Diethylaniline (36.2 mmol, 2 eq, 3.8 g) was dissolved in 10 ml of toluene. 1f (18.1 mmol, 1 eq, 3.32 g) was weighed and dissolved in toluene (10 eq of 1f, V/m, 20.0 ml) with stirring. The toluene solution of 2-methylaniline was added dropwise over 5 min. The reaction progress was monitored to be completed by TLC. The reaction solution was extracted with 50 ml of saturated sodium bicarbonate solution, and the pH was adjusted to 10. The organic phase was collected and concentrated by rotary evaporation. Column chromatography was performed on silica gel using an eluent system of $CH_2Cl_2$: MeOH=20:1. The eluate was collected and concentrated by rotary evaporation to yield a yellow-brown solid, 2a (2.02 g, 70.9% yield, HPLC > 98%). ESI-MS m/z:261.35$[M+1]^+$.

Step 2

2a (6.36 mmol, 1 eq, 1.66 g) was weighed and dissolved in analytical-grade acetonitrile (10 eq of 2a, V/m, 17.0 ml) with stirring. Analytical-grade potassium carbonate (7.63 mmol, 1.2 eq, 1.05 g) and n-butyl bromide (7.63 mmol, 1.2 eq, 1.05 g) were added. The mixture was heated to 80°C for reaction and the reaction progress was monitored to be completed using TLC. The reaction mixture was filtered and the filter cake was washed with 15 ml of acetonitrile for three times. The filtrate was collected and concentrated by rotary evaporation to yield a yellow-brown solid, 2b (2.02 g, 99% yield, HPLC > 98%). ESI-MS m/z:317.33$[M+1]^+$.

Step 3

2.02 g of yellow-brown solid 2b was dissolved in 1,4-dibromobutane (20 eq of 2b, V/m, 4 ml). The mixture was heated to 100°C, and the reaction progress was monitored to be completed using TLC. Column chromatography was performed on silica gel using an eluent system of $CH_2Cl_2$: MeOH=20:1. The eluate was collected and concentrated by rotary evaporation to yield a yellow-brown solid 2c (0.86 g, 8% yield, HPLC > 98%). ESI-MS m/z: 451.37$[M]^+$, 453.37 $[M+2]^+$.

Step 4

2c (0.8 mmol, 1 eq, 0.43 g) was weighed and dissolved in analytical-grade acetonitrile (10 eq of 2c, V/m, 4.0 ml) with stirring. Analytical-grade potassium carbonate (0.96 mmol, 1.2 eq, 0.12 g) and m-cresol (1.6 mmol, 2 eq, 0.14 g) were added. The mixture was heated to 80°C for reaction and the reaction progress was monitored to be completed using TLC. The reaction mixture was filtered and the filter cake was washed with acetonitrile (30 eq of 2c, V/m, 10 ml, three times). The filtrate was collected and subjected to preparative separation to obtain a white solid, Compound 42 (63.67 mg, 9.75% yield, HPLC > 98%).

MS m/z=479.15 $[M]^+$

$^1$H NMR (400MHz, $CD_3OD$): δ12.05(s, 1H), 10.46(s, 1H), 7.22-7.16(m, 2H), 7.11-7.02(m, 3H), 6.99-6.88(m, 3H), 4.58-4.49(t, 1H), 4.11-3.99(t, 1H), 3.46-3.29(m, 6H), 2.26-2.19(m, 6H), 1.98-1.88(m, 2H), 1.83-1.67(m, 8H), 1.49-1.37(m, 4H), 1.28-1.16(m, 4H), 0.96-0.84(t, 3H).

$^{13}$C NMR (400 MHz, $CD_3OD$): δ13.6, 17.2, 17.6, 19.4, 19.9, 22.6, 23.6, 23.6, 23.8, 26.4, 28.9, 51.5, 56.8, 56.1, 68.5, 79.3, 113.8, 114.1, 120.1, 126.9, 126.5, 128.9, 130.3, 137.3, 159.6, 161.9, 171.5.

Example 43

(2S)-1-(4-((6-acetylnaphth-2-yl)oxy)butyl)-1-butyl-2-((2,6-dimethylphenyl)carbamoyl) piperidine bromide

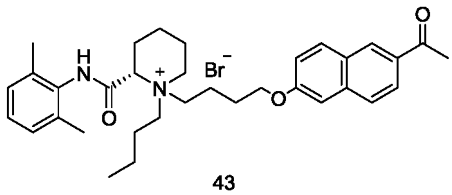

43

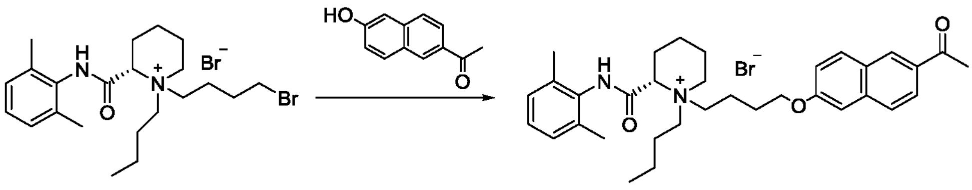

1b

43

Compound 1b (0.71 mmol, 1 eq, 0.30 g) was weighed and dissolved in analytical-grade acetonitrile (10 eq of 1b, V/m, 5.0 ml) with stirring. Analytical-grade potassium carbonate (1.2 eq of 1b, *m/m,* 0.50 g) and 6-acetyl-2-naphthol (1.42 mmol, 2 eq, 0.27 g) were added. The mixture was heated to 80°C for reaction and the reaction progress was monitored to be completed using TLC. The reaction mixture was filtered and the filter cake was washed with acetonitrile (30 eq of 1b, V/m, 15 ml, three times). The filtrate was collected and subjected to preparative separation to obtain a white solid, Compound 43 (116.9 mg, 18.3% yield, HPLC > 98%).

MS m/z=529.43[M]$^+$.

$^1$H NMR (400MHz, $CD_3OD$): δ9.70(s, 1H), 8.40(s, 1H), 8.15(d, 1H), 7.82(d, 1H), 7.69(d, 1H), 7.59(d, 2H), 7.36(s, 1H), 7.14-7.21(m, 3H), 4.16(t, 2H), 3.63(t, 1H), 2.50(s, 3H), 2.34 (t, 4H), 1.83-1.55(m, 4H), 1.4-1.3(m, 10H), 0.89(t, 3H).

$^{13}$C NMR (400 MHz, $CD_3OD$): δ13.8, 20.4, 24.5, 26.0, 26.6, 27.3, 30.4, 33.2, 35.0, 51.0, 68.4, 73.0, 108.0, 115.7, 117.7, 120.6, 124.7, 127.1, 129.6, 131.0, 131.3, 132.0, 134.1, 138.2, 157.9, 162.9, 172.0, 197.0.

**Example** 44

(2S)-1-Butyl-1-(4-((2,2-dimethyl-2,3-dihydrobenzofuran-7-yl)oxy)butyl)-2-((2,6-dimet hylphenyl) carbamoyl)piperidine bromide

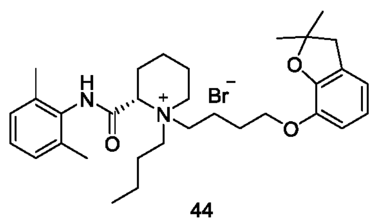

44

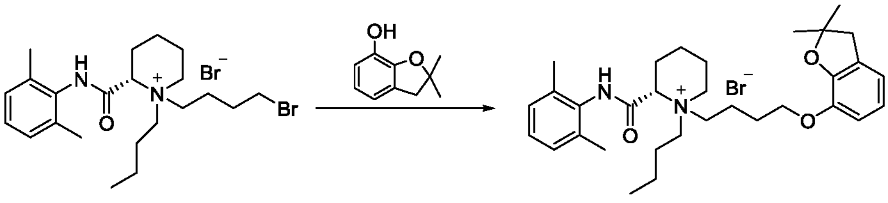

1b

44

Compound 1b (0.71 mmol, 1 eq, 0.30 g) was weighed and dissolved in analytical-grade acetonitrile (10 eq of 1b,

V/m, 5.0 ml) with stirring. Analytical-grade potassium carbonate (1.2 eq of 1b, *m*/*m,* 0.50 g) and 2,3-dimethyl-7-hydroxybenzofuran (1.42 mmol, 2 eq, 0.17 g) were added. The mixture was heated to 80°C for reaction and the reaction progress was monitored to be completed using TLC. The reaction mixture was filtered and the filter cake was washed with acetonitrile (30 eq of 1b, V/m, 15 ml, three times). The filtrate was collected and subjected to preparative separation to obtain a white solid, Compound 44 (210.38 mg, 42.3% yield, HPLC > 98%).

MS m/z=507.46$[M]^+$.

$^1H$ NMR (400MHz, $CD_3OD$): δ10.02(s, 1H), 7.05(s, 3H), 6.90-6.84 (m, 3H), 4.58(t, 1H), 4.06(t, 2H), 3.27-3.17(m, 6H), 2.85(s, 2H), 2.17-1.92(m, 8H), 1.75-1.70(m, 8H), 1.47(s, 6H), 1.30 -1.20(m, 4H), 0.89(t, 3H).

$^{13}C$ NMR (400 MHz, $CD_3OD$): δ13.8, 17.2, 17.6, 19.0, 19.3, 22.2, 23.1, 26.3, 28.1, 28.4, 42.2, 51.8, 56.5, 56.8, 68.7, 79.7, 94.5, 111.8, 120.0, 122.2, 126.8, 127.7, 128.8, 130.7, 137.1, 145.8, 146.6, 172.0.

**Comparative Example 1**

(2S)-1-Butyl-2-((2,6-dimethylphenyl)carbamoyl)-1-(2-(2-phenoxyethoxy)ethyl)piperidi ne bromide

Step 1

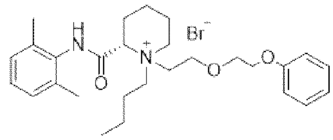

Comparative Example 1 Compound

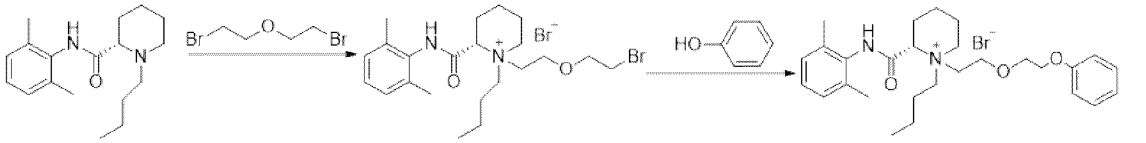

1a 2e Comparative Example 1 Compound

Levobupivacaine 1a (3.67 mmol, 1.0 eq, 1.06 g) was weighed and added with bis-(2-bromoethyl) ether (4.25 eq of levobupivacaine 1a, V/m, 3 ml). The mixture was stirred and heated to 100°C. The reaction progress was monitored to be completed using TLC. Column chromatography was performed on silica gel using an eluent system of $CH_2Cl_2$: MeOH=20:1. The eluate was collected and concentrated by rotary evaporation to yield an orange-yellow oily liquid, Compound 2e (1.0 g, 30% yield, HPLC > 90%). ESI-MS m/z: 439.36$[M]^+$, 441.48$[M+2H]^+$.

Step 2

Compound 2e (1 mmol, 1 eq, 0.51 g) was weighed and dissolved in analytical-grade acetonitrile (10 eq of 2e, V/m, 5.0 ml) with stirring. Analytical-grade sodium bicarbonate (1 eq of 2d, *m*/*m,* 0.50 g) and phenol (1.2 mmol, 1.2 eq, 0.12 g) were added. The mixture was heated to 80°C for reaction and the reaction progress was monitored to be completed using HPLC. The reaction mixture was filtered and the filter cake was washed with acetonitrile (30 eq of 2e, V/m, 15 ml, three times). The filtrate was collected and subjected to preparative separation to obtain a white powdery solid, Comparative Example 1 Compound (0.21 g, 39.62% yield, HPLC > 98%).

MS m/z=453.38$[M]^+$.

$^1H$ NMR (400MHz, $CD_3OD$): δ12.09(s, 1H), 10.13(s, 1H), 7.20-7.23(m, 2H), 7.19-7.09(m, 2H), 6.98-6.91(m, 3H), 6.42(s, 1H), 4.51-4.56(t, 1H), 4.08-4.01(t, 1H), 3.49-3.29(m, 6H), 2.26-2.18(m, 6H), 1.93-1.88(m, 2H), 1.73-1.66(m, 8H), 1.44-1.32(m, 4H), 0.98-0.89(t, 3H).

$^{13}C$ NMR (400 MHz, $CD_3OD$): δ13.8, 17.8, 17.8, 19.6, 19.8, 22.9, 24.3, 28.2, 29.6, 51.4, 56.7, 57.3, 66.6, 78.5, 113.2, 114.1, 120.7, 126.3, 127.6, 129.5, 130.3, 136.6, 158.8, 160.7, 170.4.

Biological Testing

The experimental methods described in the following test examples were standard procedures unless otherwise specified. All experimental animals used in the following test examples were SD rats purchased from Hunan Slac Jingda

Experimental Animal Co., Ltd. Other test materials, unless stated otherwise, were purchased from conventional biochemical reagent suppliers.

Test Example 1: Assessment of the Local Anesthetic Effect of Compounds of the present application

Experimental Methods:

The compounds listed in Table 1 and levobupivacaine hydrochloride (positive control) were administered to three groups of rats fully acclimated to the experimental environment, with one rat per group.

Solution Preparation: 2 mg/ml Levobupivacaine Hydrochloride Injection: 2.670 ml of 7.5 mg/ml levobupivacaine hydrochloride injection (Batch No. 92S0702) was pipetted into a 10 ml volumetric flask. 0.9% NaCl solution was added until the meniscus was level with the calibration line, and the solution was inverted and mixed thoroughly, to prepare 2 mg/ml levobupivacaine hydrochloride injection. 2 mg/ml Example Compound Solution: 6.6 mg of the example compound was accurately weighed and dissolved in 3.3 ml of 0.9% NaCl solution to prepare a 2 mg/ml example compound solution.

Both the positive control and the example compound were administered to each rat at 2 mg/ml, with an administration volume of 5 ml/kg, in a single injection near the sciatic nerve of the rat. After the administration, the rat was placed on a preheated hot plate (50±0.1°C) of an intelligent hot plate instrument. The time until the rat exhibited clear behavioral responses, such as lifting or licking its foot to avoid the thermal stimulus on the administration side, was recorded as thermal latency. The maximum thermal latency was controlled at 60 s. If the rat did not lick its foot within 60 s, its hind feet were manually lifted to prevent tissue damage or hyperalgesia, and 60 s was recorded as the thermal latency of the rat. Any urine or feces on the hot plate was promptly cleaned and dried before subsequent tests.

Thermal latency results were expressed as the maximum proportional effect (MPE), calculated using the following formula:

$$\text{MPE (\%)} = (\text{C-B}) / (\text{P-B}) * 100\%$$

Wherein: B represented the basal thermal latency of the rats; P was the maximum thermal latency; C was the observed thermal latency at the detection time point.

Measurements were considered invalid if the MPE was less than 50% of the maximum proportional effect. If two consecutive measurements yielded an MPE of less than 50%, the experiment was terminated. The specific experimental results were detailed in Table 1.

Test Results:

Table 1: Local anesthetic effect of the compounds of the present application

| Tested Drug | Onset Time/min | Duration/h |
|---|---|---|
| Compound of Example 1 | 1 | 24-48 |
| Compound of Example 2 | 1 | 48-72 |
| Compound of Example 3 | 1 | 48-72 |
| Compound of Example 4 | 1 | 48-72 |
| Compound of Example 5 | 1 | 48-72 |
| Compound of Example 6 | 1 | 24-48 |
| Compound of Example 7 | 2 | 24-48 |
| Compound of Example 8 | 1 | 24-48 |
| Compound of Example 9 | 1 | 24-48 |
| Compound of Example 10 | 2 | 48-72 |
| Compound of Example 11 | 1 | 24-48 |
| Compound of Example 12 | 1 | 24-48 |
| Compound of Example 13 | 1 | 24-48 |
| Compound of Example 14 | 2 | 24-48 |
| Compound of Example 15 | 1 | 24-48 |
| Compound of Example 16 | 1 | 24-48 |

(continued)

| Tested Drug | Onset Time/min | Duration/h |
|---|---|---|
| Compound of Example 18 | 2 | 17 |
| Compound of Example 19 | 2 | 2-18 |
| Compound of Example 20 | 1 | 24-48 |
| Compound of Example 21 | 1 | 19-24 |
| Compound of Example 22 | 1 | 48-72 |
| Compound of Example 23 | 1 | 48-72 |
| Compound of Example 24 | 1 | 48-72 |
| Compound of Example 25 | 1 | 72-96 |
| Compound of Example 26 | 1 | 24-42 |
| Compound of Example 27 | 1 | 24-48 |
| Compound of Example 28 | 1 | 24-48 |
| Compound of Example 29 | 1 | 24-30 |
| Compound of Example 30 | 1 | 72-96 |
| Compound of Example 31 | 1 | 24-30 |
| Compound of Example 34 | No effect observed | 0 |
| Compound of Example 35 | No effect observed | 0 |
| Compound of Example 36 | 1 | 30-48 |
| Compound of Example 37 | 2 | 2-18 |
| Compound of Example 38 | 1 | 48-72 |
| Compound of Example 39 | 1 | 72-96 |
| Compound of Example 40 | 1 | 32-48 |
| Compound of Example 41 | 2 | 2-24 |
| Compound of Example 42 | 2 | 2-24 |
| Compound of Example 43 | 1 | 48-72 |
| Compound of Example 44 | 1 | 48-72 |
| Comparative Example 1 (Compound 34) | 2 | 1 (the rat died 4 h after administration) |
| Levobupivacaine Hydrochloride | | 3 |

Experimental results demonstrated that at a concentration of 2 mg/ml (equivalent to a molar concentration of 3.0 mmol/L to 4.0 mmol/L), the compounds from the examples of the present application produced local anesthesia in the sciatic nerve block model for more than 24 hours (with some exceeding 17 hours). These compounds exhibited a rapid onset of action, and some provided local anesthesia for over 48 hours. The duration of local anesthesia of these compounds was significantly longer than that of the 2 mg/ml bupivacaine control group (equivalent to a molar concentration of 6.9 mmol/L). Additionally, the nerve block effect was reversible. However, the comparative compound obtained by introducing a heteroatom into the L chain of the compound of the general formula did not produce a local anesthetic effect and was notably toxic to rats. Similarly, the compound with L being $C_3$ alkylene also failed to exhibit local anesthetic effects.

Test Example 2: Local Anesthetic Effect of the Compound of the Present Application by Subcutaneous Infiltration

Experimental Methods:

After shaving and disinfecting the backs of SD rats (half male and half female) weighing 190-210 grams, a circle with a diameter of approximately 1.5 cm was drawn on one side of the exposed skin. 0.5 mL of a solution containing the drug was subcutaneously injected into the skin in the center. The solution, using physiological saline as the solvent,

contained either 2 mg/ml bupivacaine hydrochloride or 1 mg/ml concentration of the compounds listed in Table 2 of the present application. Each group consisted of 10 rats. A Von Frey fiber with a 100-gram force was attached to a needle for local skin stimulation. One minute after drug administration, the rat's response to the stimulus was measured. Testing was conducted at 6 different points within the marked area of drug administration. Skin contraction, avoidance, and other behaviors following stimulation were observed and recorded. Three points in the center and three points around the circle were stimulated, and the number of skin contraction and avoidance behaviors was recorded as N/6. For evaluation: if 4 or more instances of avoidance or skin contraction were observed, the drug was considered ineffective; if reactions were observed around the circle (≤3) but not in the center (≥1), the drug was considered still effective; if reactions were observed only at the central point, the drug was deemed ineffective. Each compound was tested on 10 rats. Specific experimental results are detailed in Table 2.

Experimental Results:

Table 2 Anesthesia effect of the compounds of the present application by skin infiltration

| Tested Drug | Onset Time/min | Duration/h |
|---|---|---|
| Compound of Example 1 | 1 | 48-72 |
| Compound of Example 2 | 1 | 72-96 |
| Compound of Example 3 | 1 | 72-96 |
| Compound of Example 4 | 1 | 72-96 |
| Compound of Example 5 | 1 | 72-96 |
| Compound of Example 6 | 1 | 48-72 |
| Compound of Example 7 | 2 | 48-72 |
| Compound of Example 8 | 1 | 48-72 |
| Compound of Example 9 | 1 | 48-72 |
| Compound of Example 10 | 2 | 48-72 |
| Compound of Example 11 | 1 | 48-72 |
| Compound of Example 12 | 1 | 48-72 |
| Compound of Example 13 | 1 | 48-72 |
| Compound of Example 14 | 2 | 48-72 |
| Compound of Example 15 | 1 | 48-72 |
| Compound of Example 16 | 1 | 48-72 |
| Compound of Example 18 | 2 | 24-28 |
| Compound of Example 22 | 1 | 48-72 |
| Compound of Example 24 | 1 | 48-72 |
| Compound of Example 25 | 1 | 72-96 |
| Compound of Example 26 | 1 | 48-72 |
| Compound of Example 27 | 1 | 48-72 |
| Compound of Example 28 | 1 | 48-72 |
| Compound of Example 30 | 1 | 72-96 |
| Compound of Example 31 | 1 | 48-72 |
| Compound of Example 36 | 1 | 48-72 |
| Compound of Example 38 | 1 | 48-72 |
| Compound of Example 39 | 1 | 72-96 |
| Compound of Example 40 | 1 | 48-72 |

(continued)

| Tested Drug | Onset Time/min | Duration/h |
|---|---|---|
| Compound of Example 44 | 1 | 48-72 |
| Levobupivacaine Hydrochloride | | 3 |

Experimental results indicated that at a concentration of 1 mg/ml (equivalent to a molar concentration of 1.5 mmol/L to 2.0 mmol/L), the compounds listed in the table above from the examples of the present application produced local anesthesia for more than 48 hours in the rat subcutaneous infiltration model. Some of these compounds provided local anesthesia for over 72 hours, significantly longer than that of the 2 mg/ml bupivacaine control group (equivalent to a molar concentration of 6.9 mmol/L), and exhibited a rapid onset of action.

Since local anesthesia lasting more than 72 hours may potentially cause irreversible nerve damage, activity tests were subsequently conducted after reducing the concentration of some compounds with durations exceeding 72 hours. This was to determine whether the local anesthetic effect in the skin infiltration model could be restored.

Table 3: Local anesthetic effect of compounds of the present application by subcutaneous infiltration

| Tested Drug | Drug Concentration | Duration/h |
|---|---|---|
| Compound 3 | 1 mmol/L | 48-72 |
| | 0.3 mmol/L | 24-48 |
| Compound 5 | 1 mmol/L | 48-72 |
| | 0.3 mmol/L | 24-48 |
| Compound 25 | 1 mmol/L | 48-72 |
| | 0.3 mmol/L | 24-48 |
| Compound 30 | 1 mmol/L | 48-72 |
| | 0.3 mmol/L | 24-48 |
| Compound 39 | 1 mmol/L | 48-72 |
| | 0.3 mmol/L | 24-48 |
| Levobupivacaine Hydrochloride | 6.9 mmol/L | 3 |

Experimental results demonstrated that the compounds in the present disclosure exhibited a clear dose-effect relationship in the rat subcutaneous infiltration nerve block model. The duration of the anesthetic effect increased with the drug dosage. Notably, at a concentration of 0.3 mmol/L, the compounds of the present application still provided a local anesthetic effect lasting over 24 hours in the rat subcutaneous infiltration model, and the anesthetic effect could be restored.

**Test Example** 3: **Evaluation of Neuropathological Damage of the Compounds of the Present Application**

Example compounds 3, 4, 5, 24, 25, 26, 28, 30, 36, 38, 39, levobupivacaine hydrochloride (positive control), and a vehicle control were administered to SD rats (half male and half female) weighing 190-210 grams and fully acclimated to the experimental environment, with 8 rats per group.

Dosing Concentrations: Physiological saline as solvent. 2 mg/mL levobupivacaine hydrochloride. Two concentrations of the compounds from the present application: 0.6 mg/mL and 0.2 mg/mL. Vehicle control group: physiological saline.

Each rat received an injection at a volume of 1.0 mL near the sciatic nerve. On the 7th and 14th days after the injection, the experimental rats were euthanized via cardiac injection of levobupivacaine hydrochloride under isoflurane anesthesia. Approximately 1.5 cm of the sciatic nerve at the injection site was excised, fixed in 10% formaldehyde for 48 h, stained with HE, and sectioned into 5 μm thick slices.

Example compounds 3, 4, 5, 24, 25, 26, 28, 30, 36, 38, 39, levobupivacaine hydrochloride (positive control), and a vehicle control were administered to SD rats (half male and half female) weighing 190-210 grams and fully acclimated to the experimental environment, with 8 rats per group.

Dosing Concentrations: Physiological saline as solvent. 2 mg/mL levobupivacaine hydrochloride. Two concentrations of the compounds from the present application: 0.6 mg/mL and 0.2 mg/mL. Vehicle control group: physiological

saline.

Each rat received a subcutaneous injection at a volume of 0.5 mL on the back. On the 7th and 14th days after the injection, the experimental rats were euthanized via cardiac injection of levobupivacaine hydrochloride under isoflurane anesthesia. Skin tissue at the injection site was excised, fixed in 10% formaldehyde for 48 h, stained with HE, and sectioned into 5 μm thick slices.

Neuropathological damage assessments revealed that, compared with the levobupivacaine hydrochloride positive control and the vehicle control groups, the example compounds did not significantly affect nerve damage, vascular proliferation, demyelination, muscle inflammation, or connective tissue inflammation, exhibiting good safety profiles.

### Test Example 4: Toxicity study of the compounds of the present application at single administration

Compounds 3, 4, and 5 were administered to SD rats weighing 190-210 grams and fully acclimated to the experimental environment, with 4 rats per group.

Administration amount: Using physiological saline as the solvent, the compounds 3, 4, and 5 were administered at an amount of 6 mg/kg.

Administration method: Each rat was injected with a volume of 1 mL/kg into the tail vein.

Compounds 3, 4, 5, and levobupivacaine hydrochloride (positive control) were administered to SD rats weighing 190-210 grams and fully acclimated to the experimental environment, with 4 rats per group.

Administration amount: Using physiological saline as the solvent, the compounds 3, 4, and 5 were administered at an amount of 100 mg/kg.

Administration method: Each rat was injected subcutaneously with a volume of 5 mL/kg.

The results showed that administration of the compounds of the present application at 6 mg/kg via tail vein and 100 mg/kg subcutaneously did not produce any notable abnormalities, and the rats remained active with no significant differences compared to normal rats.

Although the Examples disclosed in the present application are as above, the contents described are only examples adopted to facilitate understanding of the present application and are not intended to limit the present application. Modifications and changes to the form and details of implementation can be made by those skilled in the art without departing from the spirit and scope of the present application. However, the scope of protection of the present application shall still be based on the scope defined by the appended claims.

## Claims

**1.** A cyclic quaternary ammonium salt compound represented by formula (I), or a stereoisomer or solvate thereof:

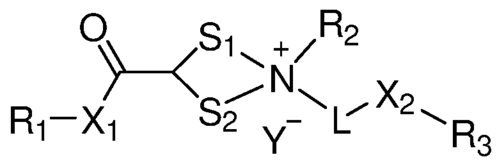

**(I)**

wherein:

$R_1$ is selected from the group consisting of aryl and heteroaryl, alternatively, $R_1$, together with $X_1$, form non-condensed or condensed azacycloalkyl;

$R_2$ is selected from the group consisting of $C_{1-8}$ alkyl and $C_{3-12}$ cycloalkyl;

$R_3$ is selected from the group consisting of aryl, heteroaryl, and heterocycloalkyl, alternatively, $R_3$, together with $X_2$, form condensed azacycloalkyl;

$X_1$ is selected from the group consisting of O, S, and $NR_4^A$, wherein $R_4^A$ is selected from the group consisting of hydrogen, deuterium, $C_{1-8}$ alkyl, $C_{3-8}$ cycloalkyl, and $C_{1-4}$ alkoxy $C_{1-4}$ alkyl, alternatively, $R_4^A$ and the N it is attached to, together with $R_1$, form non-condensed or condensed azacycloalkyl;

$X_2$ is selected from the group consisting of O, S, and $NR_4^B$, wherein $R_4^B$ is selected from the group consisting of

hydrogen, deuterium, $C_{1-8}$ alkyl, $C_{3-8}$ cycloalkyl, and $C_{1-4}$ alkoxy $C_{1-4}$ alkyl, alternatively, $R_4^B$ and the N it is attached to, together with $R_3$, form condensed azacycloalkyl;
L is selected from $C_{1-8}$ alkylene;
$S_1$ and $S_2$ are each independently selected from the group consisting of a bond and $C_{1-6}$ alkylene, with the provision that $S_1$ and $S_2$ are not both bonds; and
$Y^-$ represents a pharmaceutically acceptable anion.

**2.** The compound according to claim 1, wherein:

$R_1$ is selected from the group consisting of aryl and heteroaryl, alternatively, $R_1$, together with $X_1$, form non-condensed or condensed azacycloalkyl; wherein the aryl, the heteroaryl, the non-condensed azacycloalkyl or the condensed azacycloalkyl is optionally substituted by a group selected from the group consisting of $C_{1-6}$ alkyl, $C_{1-6}$ alkoxy, cyano, halogen, hydroxyl, amino, nitro, ester, mono-$C_{1-6}$ alkylamino, di-$C_{1-6}$ alkylamino, $C_{2-4}$ alkenyl, $C_{2-4}$ alkynyl, $C_{1-6}$ haloalkyl, $C_{1-6}$ haloalkoxy, and a combination thereof; the condensed azacycloalkyl is condensed by aryl or heteroaryl; and/or
$R_2$ is selected from the group consisting of $C_{1-8}$ alkyl and $C_{3-12}$ cycloalkyl, wherein the $C_{1-8}$ alkyl or $C_{3-12}$ cycloalkyl is optionally substituted by a group selected from the group consisting of $C_{1-6}$ alkyl, $C_{1-6}$ alkoxy, $C_{1-6}$ haloalkyl, $C_{3-6}$ cycloalkyl, halogen, hydroxyl, cyano, amino, and a combination thereof; and/or
$R_3$ is selected from the group consisting of aryl, heteroaryl, and heterocycloalkyl, alternatively, $R_3$, together with $X_2$, form condensed azacycloalkyl; wherein the aryl, the heteroaryl, the heterocycloalkyl, or the condensed azacycloalkyl is optionally substituted by a group selected from the group consisting of $C_{1-6}$ alkyl, $C_{1-6}$ alkoxy, $C_{1-6}$ alkoxy $C_{1-4}$ alkyl, $C_{1-6}$ haloalkyl, $C_{1-6}$ haloalkoxy, $C_{3-6}$ cycloalkyl, halogen, hydroxyl, cyano, amino, ester, nitro, sulfonyl, thiol, mono-$C_{1-6}$ alkylamino, di-$C_{1-6}$ alkylamino, $C_{2-4}$ alkenyl, $C_{2-4}$ alkynyl, optionally substituted aryl, optionally substituted heteroaryl, optionally substituted heterocycloalkyl, and a combination thereof; wherein the optionally substituted aryl, the optionally substituted heteroaryl, and the optionally substituted heterocycloalkyl refer to unsubstituted aryl, unsubstituted heteroaryl, and unsubstituted heterocycloalkyl, or are substituted by a group selected from the group consisting of $C_{1-6}$ alkyl, $C_{1-6}$ alkoxy, $C_{1-6}$ haloalkyl, $C_{1-6}$ haloalkoxy, $C_{3-6}$ cycloalkyl, halogen, hydroxyl, cyano, amino, ester, nitro, mono-$C_{1-6}$ alkylamino, di-$C_{1-6}$ alkylamino, $C_{2-4}$ alkenyl, $C_{2-4}$ alkynyl, and a combination thereof; the condensed azacycloalkyl is condensed by aryl or heteroaryl; and/or
$X_1$ is selected from the group consisting of O, S, and $NR_4^A$, wherein $R_4^A$ is selected from the group consisting of hydrogen, deuterium, $C_{1-8}$ alkyl, $C_{3-8}$ cycloalkyl, and $C_{1-4}$ alkoxy $C_{1-4}$ alkyl, alternatively, $R_4^A$ and the N it is attached to, together with $R_1$, form non-condensed or condensed azacycloalkyl; the condensed azacycloalkyl is condensed by aryl or heteroaryl; and/or
$X_2$ is selected from the group consisting of O, S, and $NR_4^B$, wherein $R_4^B$ is selected from the group consisting of hydrogen, deuterium, $C_{1-8}$ alkyl, $C_{3-8}$ cycloalkyl, and $C_{1-4}$ alkoxy $C_{1-4}$ alkyl, alternatively, $R_4^B$ and the N it is attached to, together with $R_3$, form condensed azacycloalkyl; the condensed azacycloalkyl is condensed by aryl or heteroaryl; and/or
L is selected from $C_{1-8}$ alkylene; wherein the $C_{1-8}$ alkylene is optionally substituted by a group selected from the group consisting of $C_{1-4}$ alkyl, $C_{1-4}$ alkoxy, $C_{1-4}$ haloalkyl, $C_{1-4}$ haloalkoxy, $C_{3-6}$ cycloalkyl, halogen, hydroxy, cyano, amino, ester, nitro, mono-$C_{1-4}$ alkylamino, di-$C_{1-4}$ alkylamino, $C_{2-4}$ alkenyl, $C_{2-4}$ alkynyl, and a combination thereof; and/or
$S_1$ and $S_2$ are each independently selected from the group consisting of a bond and $C_{1-6}$ alkylene, with the provision that $S_1$ and $S_2$ are not both bonds, wherein the $C_{1-6}$ alkylene optionally contains, on the main chain, one heteroatom selected from the group consisting of O, S, and $NR_5$, wherein $R_5$ is hydrogen or deuterium.

**3.** The compound according to claim 1, wherein $R_1$ is phenyl or naphthyl; wherein the phenyl or naphthyl is optionally substituted by a group selected from the group consisting of $C_{1-6}$ alkyl, $C_{1-6}$ alkoxy, cyano, halogen, hydroxyl, amino, nitro, ester, mono-$C_{1-6}$ alkylamino, di-$C_{1-6}$ alkylamino, $C_{2-4}$ alkenyl, $C_{2-4}$ alkynyl, $C_{1-6}$ haloalkyl, $C_{1-6}$ haloalkoxy, and a combination thereof; or

$R_1$ is phenyl, and the phenyl is optionally substituted by a group selected from the group consisting of methyl, ethyl, n-propyl, isopropyl, n-butyl, sec-butyl, tert-butyl, fluorine, chlorine, bromine, iodine, hydroxyl, amino, nitro, carbomethoxy, carboethoxy, and a combination thereof; or
$R_1$ is selected from the group consisting of phenyl, 2-methylphenyl, 2-methoxyphenyl, 4-methylphenyl, 4-methoxyphenyl, 4-fluorophenyl, 4-trifluoromethylphenyl, 2-chlorophenyl, 4-chlorophenyl, 2-bromophenyl, 3-bromophenyl, 4-bromophenyl, 3-hydroxyphenyl, 2,6-dimethylphenyl, 2,6-dimethoxyphenyl, 3-nitrophenyl, 2,6-difluorophenyl, 3-chloro-2-methylphenyl, 2,3-dichlorophenyl, 4-hydroxyphenyl, 2,4,6-trimethylphenyl, 2,4,6-trimethoxyphenyl, and 2,4,6-trifluorophenyl, preferably 2,6-dimethylphenyl.

**4.** The compound according to claim 1, wherein $R_2$ is selected from the group consisting of $C_{1-8}$ alkyl and $C_{3-8}$ cycloalkyl; wherein the $C_{1-8}$ alkyl or $C_{3-8}$ cycloalkyl is optionally substituted by a group selected from the group consisting of $C_{1-6}$ alkyl, $C_{1-6}$ alkoxy, $C_{1-6}$ haloalkyl, $C_{3-6}$ cycloalkyl, halogen, hydroxyl, cyano, amino, and a combination thereof; or
$R_2$ is selected from the group consisting of methyl, ethyl, n-propyl, isopropyl, n-butyl, sec-butyl, tert-butyl, n-pentyl, isopentyl, n-hexyl, cyclopropyl, cyclobutyl, cyclopropylmethyl, cyclobutylmethyl, cyclopentylmethyl, n-octyl, and n-heptyl, preferably the group consisting of ethyl, n-propyl, n-butyl, n-pentyl, n-hexyl, n-octyl, and n-heptyl, more preferably n-butyl.

**5.** The compound according to claim 1, wherein $R_3$ is selected from the group consisting of aryl, heteroaryl, and three-membered to eight-membered heterocycloalkyl, alternatively, $R_3$, together with $X_2$, form condensed azacycloalkyl; wherein the aryl, the heteroaryl, the three-membered to eight-membered heterocycloalkyl, or the condensed azacycloalkyl is optionally substituted by a group selected from the group consisting of $C_{1-6}$ alkyl, $C_{1-6}$ alkoxy, $C_{1-6}$ alkoxy, $C_{1-4}$ alkyl, $C_{1-6}$ haloalkyl, $C_{1-6}$ haloalkoxy, $C_{3-6}$ cycloalkyl, halogen, hydroxyl, cyano, amino, ester, nitro, sulfonyl, thiol, mono-$C_{1-6}$ alkylamino, di-$C_{1-6}$ alkylamino, $C_{2-4}$ alkenyl, $C_{2-4}$ alkynyl, optionally substituted phenyl, and a combination thereof; wherein the optionally substituted phenyl refers to unsubstituted phenyl, or is substituted by a group selected from the group consisting of $C_{1-6}$ alkyl, $C_{1-6}$ alkoxy, $C_{1-6}$ haloalkyl, $C_{1-6}$ haloalkoxy, $C_{3-6}$ cycloalkyl, halogen, hydroxy, cyano, amino, ester, nitro, mono-$C_{1-6}$ alkylamino, di-$C_{1-6}$ alkylamino, $C_{2-4}$ alkenyl, $C_{2-4}$ alkynyl, and a combination thereof; the condensed azacycloalkyl is condensed with phenyl or heteroaryl; or

$R_3$ is selected from the group consisting of phenyl, 4-fluorobenzyl, 2-methylphenyl, 2-methoxyphenyl, 2-fluorophenyl, 2-chlorophenyl, 2-bromophenyl, 2-hydroxyphenyl, 3-methylphenyl, 3-methoxyphenyl, 3-fluorophenyl, 3-chlorophenyl, 3-bromophenyl, 3-hydroxyphenyl, 4-methylphenyl, 4-methoxyphenyl, 4-fluorophenyl, 4-chlorophenyl, 4-bromophenyl, 4-hydroxyphenyl, 4-trifluoromethylphenyl, 2,4-dimethylphenyl, 2,4-dimethoxyphenyl, 2,4-difluorophenyl, 2,4-dichlorophenyl, 2,4-dihydroxyphenyl, 2,6-dimethylphenyl, 2,6-dimethoxyphenyl, 2,6-difluorophenyl, 2,6-dichlorophenyl, 2,6-dihydroxyphenyl, 2,4,6-trimethylphenyl, 2,4,6-trimethoxyphenyl, 2,4,6-trifluorophenyl, 2-amino-5-fluorophenyl, 2-isopropyl-5-methylphenyl, 4-cyanophenyl, 4-ethoxycarbonylphenyl, 2,6-di-tert-butyl-4-methylphenyl, 4-nitrophenyl, and 4-sulfonylphenyl; alternatively, $R_3$, together with $X_2$, form condensed piperidinyl, condensed piperazinyl, or condensed pyrrolidinyl, wherein the condensed piperidinyl, the condensed piperazinyl, or the condensed pyrrolidinyl is condensed with a group selected from the group consisting of phenyl, thienyl, furanyl, pyrrolyl, thiazolyl, isothiazolyl, imidazolyl, triazolyl, pyridinyl, and pyrimidinyl; and the condensed piperidinyl, the condensed piperazinyl or the condensed pyrrolidinyl is optionally substituted by a group selected from the group consisting of methyl, ethyl, n-propyl, isopropyl, n-butyl, sec-butyl, tert-butyl, fluorine, chlorine, bromine, iodine, hydroxyl, amino, nitro, carbomethoxy, carboethoxy, phenyl, substituted phenyl, and a combination thereof; or
$R_3$ is selected from the group consisting of phenyl, 2-methylphenyl, 3-methylphenyl, 4-methylphenyl, 4-fluorophenyl, 2-amino-5-fluorophenyl, 2-isopropyl-5-methylphenyl, 4-cyanophenyl, 4-ethoxycarbonylphenyl, 2,6-di-tert-butyl-4-methylphenyl, 4-nitrophenyl, 4-sulfonylphenyl, and 4-hydroxyphenyl; alternatively, $R_3$, together with $X_2$, form imidazopiperazinyl, thienopiperazinyl, or pyrrolopiperazinyl; optionally, the imidazopiperazinyl, thienopiperazinyl, or pyrrolopiperazinyl is substituted by a group selected from the group consisting of methyl, ethyl, n-propyl, isopropyl, n-butyl, sec-butyl, tert-butyl, fluorine, chlorine, bromine, iodine, hydroxyl, amino, nitro, carbomethoxy, carboethoxy, phenyl, and a combination thereof.

**6.** The compound according to claim 1, wherein L is selected from the group consisting of $C_{3-6}$ alkylene; wherein the $C_{3-6}$ alkylene is optionally substituted by a group selected from the group consisting of $C_{1-4}$ alkyl, $C_{1-4}$ alkoxy, $C_{1-4}$ haloalkyl, $C_{1-4}$ haloalkoxy, $C_{3-6}$ cycloalkyl, halogen, hydroxyl, cyano, amino, ester, nitro, mono-$C_{1-4}$ alkylamino, di-$C_{1-4}$ alkylamino, $C_{2-4}$ alkenyl, $C_{2-4}$ alkynyl, and a combination thereof; or
L is selected from the group consisting of $-(CH_2)_3-$, $-(CH_2)_4-$, $-(CH_2)_5-$, $-(CH_2)_6-$, and $-(CH_2)_2CHCH_3(CH_2)_2-$, preferably $-(CH_2)_4-$ or $-(CH_2)_5-$.

**7.** The compound according to claim 1, wherein L is not $C_3$ alkylene.

**8.** The compound according to claim 1, wherein:

$R_1$ is selected from the group consisting of aryl and heteroaryl, alternatively, $R_1$, together with $X_1$, form non-condensed or condensed azacycloalkyl; wherein the aryl, the heteroaryl, the non-condensed azacycloalkyl or the condensed azacycloalkyl is optionally substituted by a group selected from the group consisting of $C_{1-6}$ alkyl, $C_{1-6}$ alkoxy, cyano, halogen, hydroxyl, amino, nitro, ester, mono-$C_{1-6}$ alkylamino, di-$C_{1-6}$ alkylamino, $C_{2-4}$ alkenyl, $C_{2-4}$ alkynyl, $C_{1-6}$ haloalkyl, $C_{1-6}$ haloalkoxy, and a combination thereof; the condensed azacycloalkyl is condensed by aryl or heteroaryl; and/or

$R_2$ is selected from the group consisting of $C_{1-8}$ alkyl and $C_{3-12}$ cycloalkyl, wherein the $C_{1-8}$ alkyl or $C_{3-12}$ cycloalkyl is optionally substituted by a group selected from the group consisting of $C_{1-6}$ alkyl, $C_{1-6}$ alkoxy, $C_{1-6}$ haloalkyl, $C_{3-6}$ cycloalkyl, halogen, hydroxyl, cyano, amino, and a combination thereof; and/or

$R_3$ is selected from the group consisting of aryl, heteroaryl, and heterocycloalkyl, alternatively, $R_3$, together with $X_2$, form condensed azacycloalkyl; wherein the aryl, the heteroaryl, the heterocycloalkyl, or the condensed azacycloalkyl is optionally substituted by a group selected from the group consisting of $C_{1-6}$ alkyl, $C_{1-6}$ alkoxy, $C_{1-6}$ alkoxy $C_{1-4}$ alkyl, $C_{1-6}$ haloalkyl, $C_{1-6}$ haloalkoxy, $C_{3-6}$ cycloalkyl, halogen, hydroxyl, cyano, amino, $C_{1-6}$ alkanoyl, ester, nitro, sulfonyl, thiol, mono-$C_{1-6}$ alkylamino, di-$C_{1-6}$ alkylamino, $C_{2-4}$ alkenyl, $C_{2-4}$ alkynyl, optionally substituted aryl, optionally substituted heteroaryl, optionally substituted heterocycloalkyl, and a combination thereof; optionally, adjacent substituents, together with the atoms they are attached to, form a ring; wherein the optionally substituted aryl, the optionally substituted heteroaryl, and the optionally substituted heterocycloalkyl refer to unsubstituted aryl, unsubstituted heteroaryl, and unsubstituted heterocycloalkyl, or are substituted by a group selected from the group consisting of $C_{1-6}$ alkyl, $C_{1-6}$ alkoxy, $C_{1-6}$ haloalkyl, $C_{1-6}$ haloalkoxy, $C_{3-6}$ cycloalkyl, halogen, hydroxyl, cyano, amino, $C_{1-6}$ alkanoyl, ester, nitro, mono-$C_{1-6}$ alkylamino, di-$C_{1-6}$ alkylamino, $C_{2-4}$ alkenyl, $C_{2-4}$ alkynyl, and a combination thereof; the condensed azacycloalkyl is condensed by aryl or heteroaryl; and/or

$X_1$ is selected from the group consisting of O, S, and $NR_4^A$, wherein $R_4^A$ is selected from the group consisting of hydrogen, deuterium, $C_{1-8}$ alkyl, $C_{3-8}$ cycloalkyl, and $C_{1-4}$ alkoxy $C_{1-4}$ alkyl, alternatively, $R_4^A$ and the N it is attached to, together with $R_1$, form non-condensed or condensed azacycloalkyl; the condensed azacycloalkyl is condensed by aryl or heteroaryl; and/or

$X_2$ is selected from the group consisting of O, S, and $NR_4^B$, wherein $R_4^B$ is selected from the group consisting of hydrogen, deuterium, $C_{1-8}$ alkyl, $C_{3-8}$ cycloalkyl, and $C_{1-4}$ alkoxy $C_{1-4}$ alkyl, alternatively, $R_4^B$ and the N it is attached to, together with $R_3$, form condensed azacycloalkyl; the condensed azacycloalkyl is condensed by aryl or heteroaryl; and/or

L is selected from $C_{1-8}$ alkylene, with the provision that L is not $C_3$ alkylene; wherein the $C_{1-8}$ alkylene is optionally substituted by a group selected from the group consisting of $C_{1-4}$ alkyl, $C_{1-4}$ alkoxy, $C_{1-4}$ haloalkyl, $C_{1-4}$ haloalkoxy, $C_{3-6}$ cycloalkyl, halogen, hydroxy, cyano, amino, ester, nitro, mono-$C_{1-4}$ alkylamino, di-$C_{1-4}$ alkylamino, $C_{2-4}$ alkenyl, $C_{2-4}$ alkynyl, and a combination thereof; and/or

$S_1$ and $S_2$ are each independently selected from the group consisting of a bond and $C_{1-6}$ alkylene, with the provision that $S_1$ and $S_2$ are not both bonds, wherein the $C_{1-6}$ alkylene optionally contains, on the main chain, one heteroatom selected from the group consisting of O, S, and $NR_5$, wherein $R_5$ is hydrogen or deuterium.

**9.** The compound according to claim 1, wherein $R_1$ is phenyl or naphthyl; the phenyl or naphthyl is optionally substituted by a group selected from the group consisting of $C_{1-6}$ alkyl, $C_{1-6}$ alkoxy, cyano, halogen, hydroxyl, amino, nitro, ester, mono-$C_{1-6}$ alkylamino, di-$C_{1-6}$ alkylamino, $C_{2-4}$ alkenyl, $C_{2-4}$ alkynyl, $C_{1-6}$ haloalkyl, $C_{1-6}$ haloalkoxy, and a combination thereof; or

$R_1$ is phenyl, and the phenyl is optionally substituted by a group selected from the group consisting of methyl, ethyl, n-propyl, isopropyl, n-butyl, sec-butyl, tert-butyl, fluorine, chlorine, bromine, iodine, hydroxyl, amino, nitro, carbomethoxy, carboethoxy, and a combination thereof; or

$R_1$ is selected from the group consisting of phenyl, 2-methylphenyl, 2-methoxyphenyl, 4-methylphenyl, 4-methoxyphenyl, 4-fluorophenyl, 4-trifluoromethylphenyl, 2-chlorophenyl, 4-chlorophenyl, 2-bromophenyl, 3-bromophenyl, 4-bromophenyl, 3-hydroxyphenyl, 2,6-dimethylphenyl, 2,6-diethylphenyl, 2,6-dimethoxyphenyl, 3-nitrophenyl, 2,6-difluorophenyl, 2,6-dibromophenyl, 3-chloro-2-methylphenyl, 2,3-dichlorophenyl, 4-hydroxyphenyl, 2,4,6-trimethylphenyl, 2,4,6-trimethoxyphenyl, and 2,4,6-trifluorophenyl, preferably the group consisting of 2-methylphenyl, 4-fluorophenyl, 2,6-dibromophenyl, 2,6-diethylphenyl, and 2,6-dimethylphenyl, more preferably 2,6-dimethylphenyl.

**10.** The compound according to claim 1, wherein $R_2$ is selected from the group consisting of $C_{1-8}$ alkyl and $C_{3-8}$ cycloalkyl;

wherein the $C_{1-8}$ alkyl or $C_{3-8}$ cycloalkyl is optionally substituted by a group selected from the group consisting of $C_{1-6}$ alkyl, $C_{1-6}$ alkoxy, $C_{1-6}$ haloalkyl, $C_{3-6}$ cycloalkyl, halogen, hydroxyl, cyano, amino, and a combination thereof; or $R_2$ is selected from the group consisting of methyl, ethyl, n-propyl, isopropyl, n-butyl, sec-butyl, isobutyl, tert-butyl, n-pentyl, isopentyl, 4-methylpentyl, n-hexyl, cyclopropyl, cyclobutyl, cyclopropylmethyl, cyclobutylmethyl, cyclopentylmethyl, n-octyl, and n-heptyl, preferably the group consisting of ethyl, n-propyl, n-butyl, isobutyl, n-pentyl, 4-methylpentyl, n-hexyl, n-octyl, and n-heptyl, more preferably the group consisting of n-butyl, isobutyl, and 4-methylpentyl, and particularly preferably n-butyl.

**11.** The compound according to claim 1, wherein $R_3$ is selected from the group consisting of aryl, heteroaryl, and three-membered to eight-membered heterocycloalkyl, alternatively, $R_3$, together with $X_2$, form condensed azacycloalkyl; wherein the aryl, the heteroaryl, the three-membered to eight-membered heterocycloalkyl, or the condensed azacycloalkyl is optionally substituted by a group selected from the group consisting of $C_{1-6}$ alkyl, $C_{1-6}$ alkoxy, $C_{1-6}$ alkoxy, $C_{1-4}$ alkyl, $C_{1-6}$ haloalkyl, $C_{1-6}$ haloalkoxy, $C_{3-6}$ cycloalkyl, halogen, hydroxyl, cyano, amino, $C_{1-6}$ alkanoyl, ester, nitro, sulfonyl, thiol, mono-$C_{1-6}$ alkylamino, di-$C_{1-6}$ alkylamino, $C_{2-4}$ alkenyl, $C_{2-4}$ alkynyl, optionally substituted phenyl, and a combination thereof; optionally, adjacent substituents, together with the atoms they are attached to, form a ring; wherein the optionally substituted phenyl refers to unsubstituted phenyl, or is substituted by a group selected from the group consisting of $C_{1-6}$ alkyl, $C_{1-6}$ alkoxy, $C_{1-6}$ haloalkyl, $C_{1-6}$ haloalkoxy, $C_{3-6}$ cycloalkyl, halogen, hydroxy, cyano, amino, ester, nitro, mono-$C_{1-6}$ alkylamino, di-$C_{1-6}$ alkylamino, $C_{2-4}$ alkenyl, $C_{2-4}$ alkynyl, and a combination thereof; the condensed azacycloalkyl is condensed with phenyl or heteroaryl; or

$R_3$ is selected from the group consisting of phenyl, 4-fluorobenzyl, 2-methylphenyl, 2-methoxyphenyl, 2-fluorophenyl, 2-chlorophenyl, 2-bromophenyl, 2-hydroxyphenyl, 3-methylphenyl, 3-methoxyphenyl, 3-fluorophenyl, 3-chlorophenyl, 3-bromophenyl, 3-hydroxyphenyl, 3-cyanophenyl, 3-trifluoromethylphenyl, 4-methylphenyl, 4-methoxyphenyl, 4-fluorophenyl, 4-chlorophenyl, 4-bromophenyl, 4-hydroxyphenyl, 4-trifluoromethylphenyl, 2,4-dimethylphenyl, 2,4-dimethoxyphenyl, 2,4-difluorophenyl, 2,4-dichlorophenyl, 2,4-dihydroxyphenyl, 3,4-difluorophenyl, 3,4-dichlorophenyl, 3,5-difluorophenyl, 2,6-dimethylphenyl, 2,6-dimethoxyphenyl, 2,6-difluorophenyl, 2,6-dichlorophenyl, 2,6-dihydroxyphenyl, 2,4,6-trimethylphenyl, 2,4,6-trimethoxyphenyl, 2,4,6-trifluorophenyl,
2-amino-5-fluorophenyl, 2-isopropyl-5-methylphenyl, 4-cyanophenyl, 4-ethoxycarbonylphenyl, 2,6-di-tert-butyl-4-methylphenyl, 4-nitrophenyl, 4-sulfonylphenyl, 2,2-dimethyl-2,3-dihydrobenzofuran-7-yl, naphthyl, and 6-acetylnaphth-2-yl; alternatively, $R_3$, together with $X_2$, form condensed piperidinyl, condensed piperazinyl, or condensed pyrrolidinyl, wherein the condensed piperidinyl, the condensed piperazinyl, or the condensed pyrrolidinyl is condensed with a group selected from the group consisting of phenyl, thienyl, furanyl, pyrrolyl, thiazolyl, isothiazolyl, imidazolyl, triazolyl, pyridinyl, and pyrimidinyl; and the condensed piperidinyl, the condensed piperazinyl or the condensed pyrrolidinyl is optionally substituted by a group selected from the group consisting of methyl, ethyl, n-propyl, isopropyl, n-butyl, sec-butyl, tert-butyl, fluorine, chlorine, bromine, iodine, hydroxyl, amino, nitro, carbomethoxy, carboethoxy, phenyl, substituted phenyl, and a combination thereof; or
$R_3$ is selected from the group consisting of phenyl, 2-methylphenyl, 3-methylphenyl, 4-methylphenyl, 4-fluorophenyl, 3-cyanophenyl, 3-trifluoromethylphenyl, 3,4-difluorophenyl, 3,4-dichlorophenyl, 3,5-difluorophenyl, 2-amino-5-fluorophenyl, 2-isopropyl-5-methylphenyl, 4-cyanophenyl, 4-ethoxycarbonylphenyl, 2,6-di-tert-butyl-4-methylphenyl, 4-nitrophenyl, 4-sulfonylphenyl, 4-hydroxyphenyl, 2,2-dimethyl-2,3-dihydrobenzofuran-7-yl, and 6-acetylnaphth-2-yl; alternatively, $R_3$, together with $X_2$, form imidazopiperazinyl, thienopiperazinyl, or pyrrolopiperazinyl; optionally, the imidazopiperazinyl, thienopiperazinyl, or pyrrolopiperazinyl is substituted by a group selected from the group consisting of methyl, ethyl, n-propyl, isopropyl, n-butyl, sec-butyl, tert-butyl, fluorine, chlorine, bromine, iodine, hydroxyl, amino, nitro, carbomethoxy, carboethoxy, phenyl, and a combination thereof.

**12.** The compound according to claim 1, wherein L is selected from $C_{3-6}$ alkylene, with the provision that L is not $C_3$ alkylene; wherein the $C_{3-6}$ alkylene is optionally substituted by a group selected from the group consisting of $C_{1-4}$ alkyl, $C_{1-4}$ alkoxy, $C_{1-4}$ haloalkyl, $C_{1-8}$ haloalkoxy, $C_{3-6}$ cycloalkyl, halogen, hydroxyl, cyano, amino, ester, nitro, mono-$C_{1-4}$ alkylamino, di-$C_{1-4}$ alkylamino, $C_{2-4}$ alkenyl, $C_{2-4}$ alkynyl, and a combination thereof; or
L is selected from the group consisting of $-(CH_2)_4-$, $-(CH_2)_5-$, $-(CH_2)_6-$, and $-(CH_2)_2CHCH_3(CH_2)_2-$, preferably $-(CH_2)_4-$ or $-(CH_2)_5-$.

**13.** The compound according to claim 1, wherein $X_1$ is selected from the group consisting of O, NH, $NCH_3$, $NCH_2CH_3$, and $N(CH_2)_2CH_3$, preferably, $X_1$ is NH.

**14.** The compound according to claim 1, wherein $X_2$ is selected from the group consisting of O, NH, $NCH_3$, $NCH_2CH_3$, and $N(CH_2)_2CH_3$, alternatively, $X_2$, together with $R_3$, form condensed piperidinyl, condensed piperazinyl or condensed

pyrrolidinyl, wherein the condensed piperidinyl, the condensed piperazinyl or the condensed pyrrolidinyl is condensed with a group selected from the group consisting of phenyl, thienyl, furanyl, pyrrolyl, thiazolyl, isothiazolyl, imidazolyl, triazolyl, pyridinyl, and pyrimidinyl; and the condensed piperidinyl, the condensed piperazinyl or the condensed pyrrolidinyl is optionally substituted by a group selected from the group consisting of methyl, ethyl, n-propyl, isopropyl, n-butyl, sec-butyl, tert-butyl, fluorine, chlorine, bromine, iodine, hydroxyl, amino, nitro, carbomethoxy, carboethoxy, phenyl, substituted phenyl, and a combination thereof;

preferably, $X_2$ is O or NH, alternatively, $X_2$, together with $R_3$, form imidazopiperazinyl, thienopiperazinyl, or pyrrolopiperazinyl; optionally, the imidazopiperazinyl, thienopiperazinyl, or pyrrolopiperazinyl is substituted by a group selected from the group consisting of methyl, ethyl, n-propyl, isopropyl, n-butyl, sec-butyl, tert-butyl, fluorine, chlorine, bromine, iodine, hydroxyl, amino, nitro, carbomethoxy, carboethoxy, phenyl, and a combination thereof.

**15.** The compound according to claim 1, wherein when one of $S_1$ and $S_2$ is a bond, the other is selected from the group consisting of $-(CH_2)_2-$, $-(CH_2)_3-$, $-(CH_2)_4-$, $-(CH_2)_5-$, and $-(CH_2)_6-$.

**16.** The compound according to claim 1, wherein $Y^-$ is selected from the group consisting of a halogen anion, sulfate, acetate, tartrate, p-toluenesulfonate, methanesulfonate, and citrate, preferably the group consisting of $Cl^-$, $Br^-$, and $CH_3COO^-$; more preferably $Br^-$.

**17.** The compound according to any one of claims 1 to 6 and 13 to 16, wherein the compound represented by formula (I) is a compound represented by formula (IV):

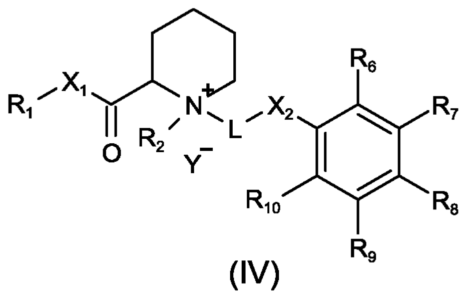

**(IV)**

wherein $X_1$ is $NR_4^A$, wherein $R_4^A$ is selected from the group consisting of hydrogen, deuterium, $C_{1-8}$ alkyl, $C_{3-8}$ cycloalkyl, and $C_{1-4}$ alkoxy $C_{1-4}$ alkyl, preferably $X_1$ is NH;

$X_2$ is selected from the group consisting of O, S and $NR_4^B$, wherein $R_4^B$ is selected from the group consisting of hydrogen, deuterium, $C_{1-8}$ alkyl, $C_{3-8}$ cycloalkyl, and $C_{1-4}$ alkoxy $C_{1-4}$ alkyl; preferably $X_2$ is selected from the group consisting of O, NH, $NCH_3$, $NCH_2CH_3$, and $N(CH_2)_2CH_3$, more preferably $X_2$ is O or NH;

$R_6$, $R_7$, $R_8$, $R_9$, and $R_{10}$ are each independently selected from the group consisting of hydrogen, fluorine, chlorine, bromine, iodine, hydroxyl, cyano, nitro, amino, ester, $C_{1-4}$ alkyl, $C_{1-4}$ alkoxy, and $C_{1-4}$ alkoxy$C_{1-4}$ alkyl;

$R_1$, $R_2$, L and $Y^-$ are as defined in formula (I) according to any one of claims 1 to 6, 13 to 16.

**18.** The compound according to any one of claims 7 to 12, wherein the compound represented by formula (I) is a compound represented by formula (IV):

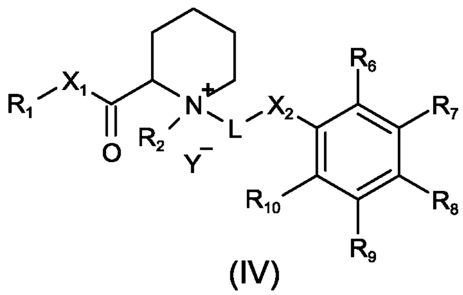

**(IV)**

wherein $X_1$ is $NR_4^A$, wherein $R_4^A$ is selected from the group consisting of hydrogen, deuterium, $C_{1-8}$ alkyl, $C_{3-8}$

cycloalkyl, and $C_{1-4}$ alkoxy $C_{1-4}$ alkyl, preferably $X_1$ is NH;

$X_2$ is selected from the group consisting of O, S and $NR_4^B$, wherein $R_4^B$ is selected from the group consisting of hydrogen, deuterium, $C_{1-8}$ alkyl, $C_{3-8}$ cycloalkyl, and $C_{1-4}$ alkoxy $C_{1-4}$ alkyl; preferably $X_2$ is selected from the group consisting of O, **NH,** $NCH_3$, $NCH_2CH_3$, and $N(CH_2)_2CH_3$, more preferably $X_2$ is O or NH;
$R_6$, $R_7$, $R_8$, $R_9$, and $R_{10}$ are each independently selected from the group consisting of hydrogen, fluorine, chlorine, bromine, iodine, hydroxyl, cyano, nitro, amino, ester, $C_{1-4}$ alkyl, $C_{1-4}$ alkoxy, and $C_{1-4}$ alkoxy $C_{1-4}$ alkyl, and optionally, adjacent substituents, together with the atoms they are attached to, form a ring;
$R_1$, $R_2$, L and $Y^-$ are as defined in formula (I) according to any one of claims 7 to 12.

**19.** The compound according to any one of claims 1 to 6 and 13 to 16, wherein the compound represented by formula (I) is a compound represented by formula (V):

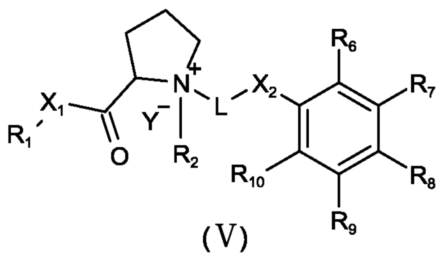

(V)

wherein $X_1$ is $NR_4^A$, wherein $R_4^A$ is selected from the group consisting of hydrogen, deuterium, $C_{1-8}$ alkyl, $C_{3-8}$ cycloalkyl, and $C_{1-4}$ alkoxy $C_{1-4}$ alkyl, preferably $X_1$ is NH;

$X_2$ is selected from the group consisting of O, S and $NR_4^B$, wherein $R_4^B$ is selected from the group consisting of hydrogen, deuterium, $C_{1-8}$ alkyl, $C_{3-8}$ cycloalkyl, and $C_{1-4}$ alkoxy $C_{1-4}$ alkyl, preferably $X_2$ is selected from the group consisting of O, NH, $NCH_3$, $NCH_2CH_3$, and $N(CH_2)_2CH_3$, more preferably $X_2$ is O or NH;
$R_6$, $R_7$, $R_8$, $R_9$, and $R_{10}$ are each independently selected from the group consisting of hydrogen, fluorine, chlorine, bromine, iodine, hydroxyl, cyano, nitro, amino, ester, $C_{1-4}$ alkyl, $C_{1-4}$ alkoxy, and $C_{1-4}$ alkoxy $C_{1-4}$ alkyl;
$R_1$, $R_2$, L and $Y^-$ are as defined in formula (I) according to any one of claims 1 to 6 and 13 to 16.

**20.** The compound according to any one of claims 7 to 12, wherein the compound represented by formula (I) is a compound represented by formula (V):

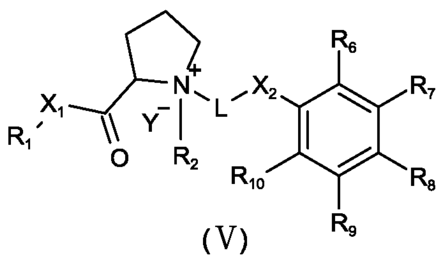

(V)

wherein $X_1$ is $NR_4^A$, wherein $R_4^A$ is selected from the group consisting of hydrogen, deuterium, $C_{1-8}$ alkyl, $C_{3-8}$ cycloalkyl, and $C_{1-4}$ alkoxy $C_{1-4}$ alkyl, preferably $X_1$ is NH;

$X_2$ is selected from the group consisting of O, S and $NR_4^B$, wherein $R_4^B$ is selected from the group consisting of hydrogen, deuterium, $C_{1-8}$ alkyl, $C_{3-8}$ cycloalkyl, and $C_{1-4}$ alkoxy $C_{1-4}$ alkyl, preferably $X_2$ is selected from the group consisting of O, NH, $NCH_3$, $NCH_2CH_3$, and $N(CH_2)_2CH_3$, more preferably $X_2$ is O or NH;
$R_6$, $R_7$, $R_8$, $R_9$, and $R_{10}$ are each independently selected from the group consisting of hydrogen, fluorine, chlorine, bromine, iodine, hydroxyl, cyano, nitro, amino, ester, $C_{1-4}$ alkyl, $C_{1-4}$ alkoxy, and $C_{1-4}$ alkoxy $C_{1-4}$ alkyl, and optionally, adjacent substituents, together with the atoms they are attached to, form a ring;
$R_1$, $R_2$, Land $Y^-$ are as defined in formula (I) according to any one of claims 7 to 12.

**21.** The compound according to any one of claims 1 to 16, wherein the compound represented by formula (I) is a

compound represented by formula (VI):

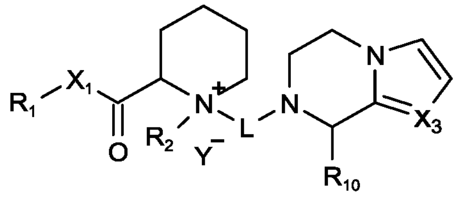

(VI)

wherein $X_1$ is $NR_4^A$, wherein $R_4^A$ is selected from the group consisting of hydrogen, deuterium, $C_{1-8}$ alkyl, $C_{3-8}$ cycloalkyl, and $C_{1-4}$ alkoxy $C_{1-4}$ alkyl, preferably $X_1$ is NH;
$X_3$ is C or N;
$R_{10}$ is selected from the group consisting of hydrogen, $C_{1-4}$ alkyl, $C_{1-4}$ alkoxy, $C_{1-4}$ alkoxy $C_{1-4}$ alkyl, and aryl, wherein the aryl is optionally substituted by a group selected from the group consisting of fluorine, chlorine, bromine, iodine, hydroxyl, cyano, nitro, amino, ester, $C_{1-4}$ alkyl, $C_{1-4}$ alkoxy, and $C_{1-4}$ alkoxy $C_{1-4}$ alkyl;
$R_1$, $R_2$, Land $Y^-$ are as defined in formula (I) according to any one of claims 1 to 16.

**22.** The compound according to any one of claims 1 to 6 and 13 to 16, wherein the compound represented by formula (I) is a compound represented by formula (VII):

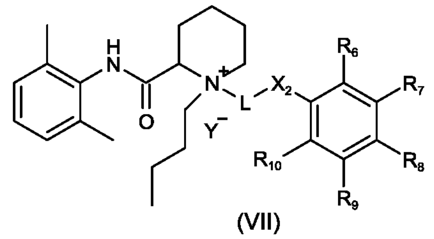

(VII)

wherein $X_2$ is selected from the group consisting of O, S and $NR_4^B$, wherein $R_4^B$ is selected from the group consisting of hydrogen, deuterium, $C_{1-8}$ alkyl, $C_{3-8}$ cycloalkyl, and $C_{1-4}$ alkoxy $C_{1-4}$ alkyl, preferably $X_2$ is selected from the group consisting of O, NH, $NCH_3$, $NCH_2CH_3$, and $N(CH_2)_2CH_3$, more preferably $X_2$ is O or NH;
$R_6$, $R_7$, $R_8$, $R_9$, and $R_{10}$ are each independently selected from the group consisting of hydrogen, fluorine, chlorine, bromine, iodine, hydroxyl, cyano, nitro, amino, ester, $C_{1-4}$ alkyl, $C_{1-4}$ alkoxy, and $C_{1-4}$ alkoxy $C_{1-4}$ alkyl;
L and $Y^-$ are as defined in formula (I) according to any one of claims 1 to 6 and 13 to 16.

**23.** The compound according to any one of claims 7 to 12, wherein the compound represented by formula (I) is a compound represented by formula (VII):

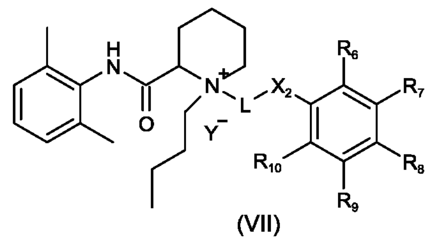

(VII)

wherein $X_2$ is selected from the group consisting of O, S and $NR_4^B$, wherein $R_4^B$ is selected from the group consisting of hydrogen, deuterium, $C_{1-8}$ alkyl, $C_{3-8}$ cycloalkyl, and $C_{1-4}$ alkoxy $C_{1-4}$ alkyl, preferably $X_2$ is selected from the group consisting of O, NH, $NCH_3$, $NCH_2CH_3$, and $N(CH_2)_2CH_3$, more preferably $X_2$ is O or NH;
$R_6$, $R_7$, $R_8$, $R_9$, and $R_{10}$ are each independently selected from the group consisting of hydrogen, fluorine, chlorine, bromine, iodine, hydroxyl, cyano, nitro, amino, ester, $C_{1-4}$ alkyl, $C_{1-4}$ alkoxy, and $C_{1-4}$ alkoxy $C_{1-4}$ alkyl, and

optionally, adjacent substituents, together with the atoms they are attached to, form a ring;
L and $Y^-$ are as defined in formula (I) according to any one of claims 7 to 12.

**24.** The compound according to claim 1, which is selected from the group consisting of:

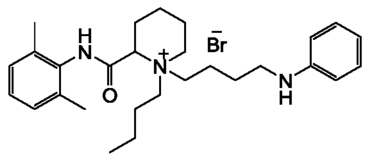
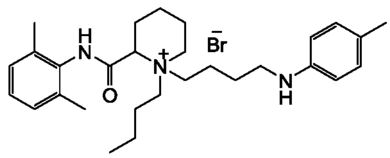
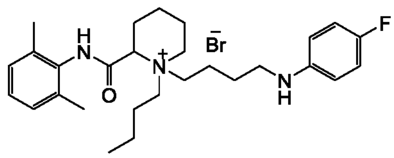
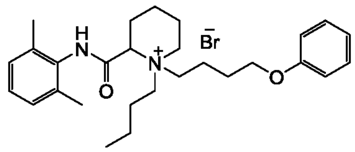
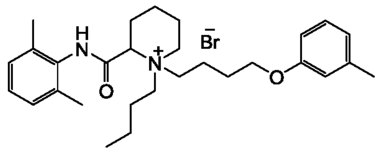
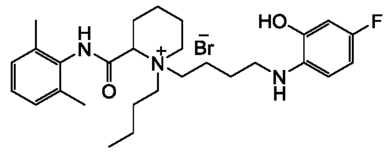
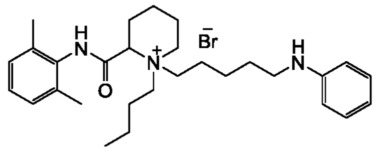
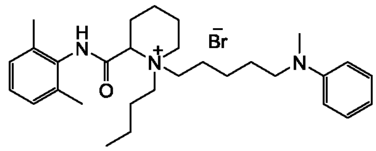
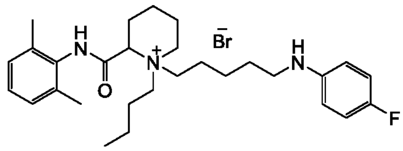
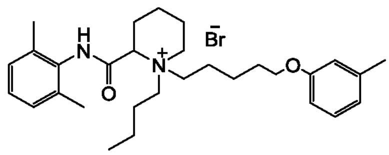
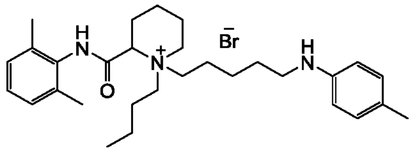
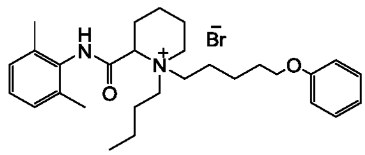
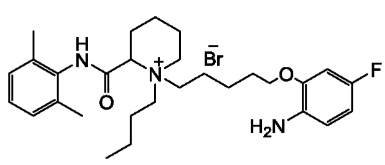
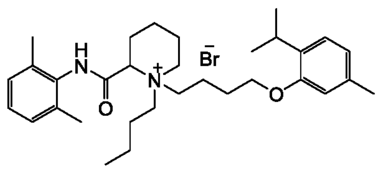
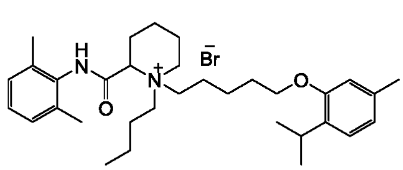
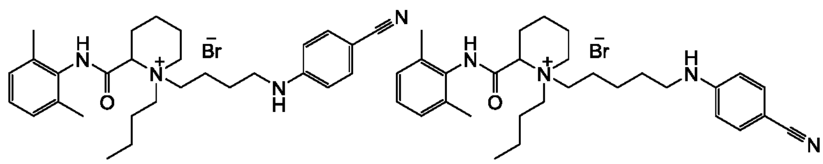
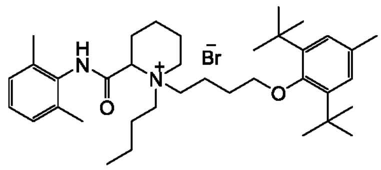
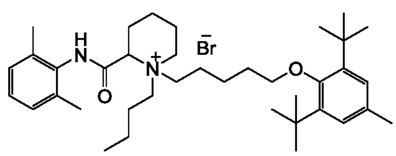
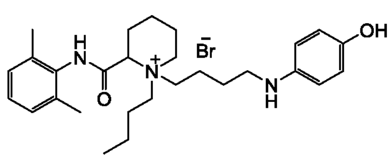
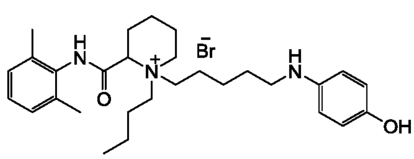
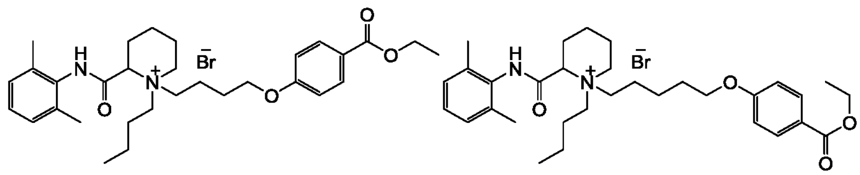
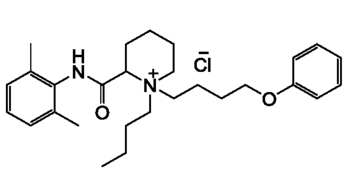

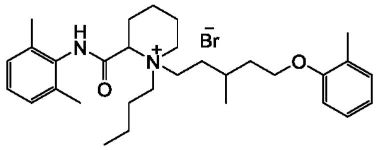
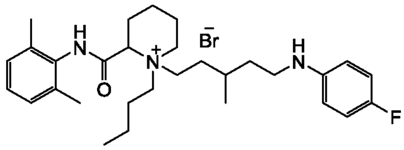
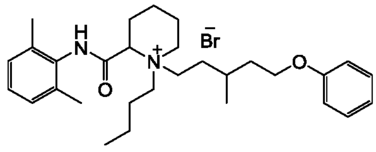

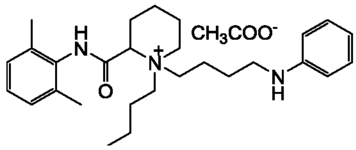
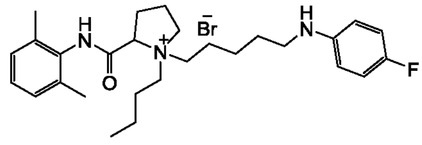
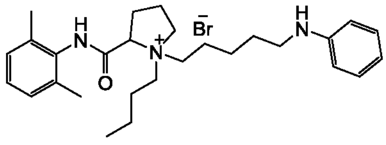

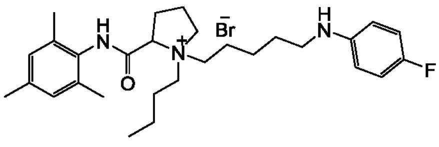
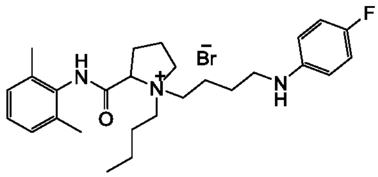
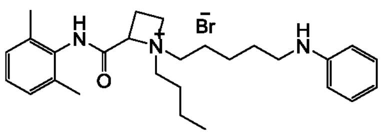

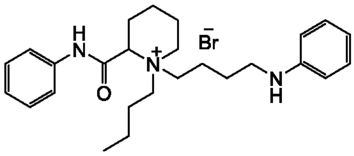
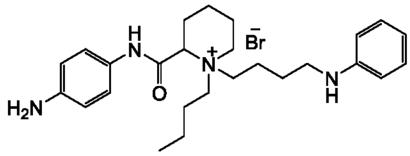
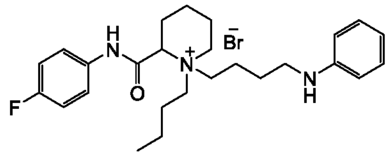

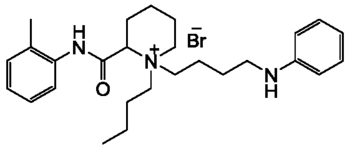
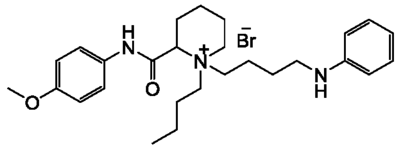
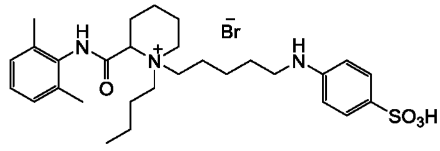

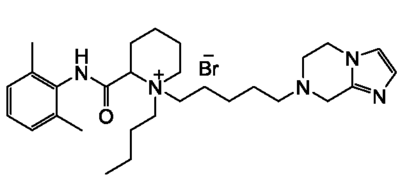
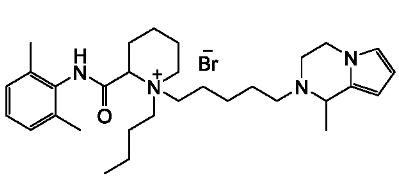
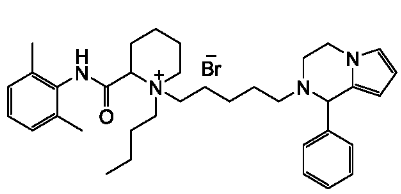

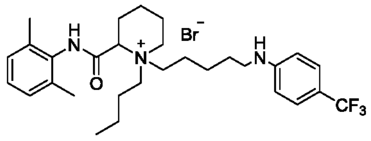
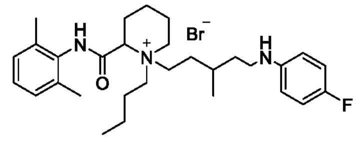
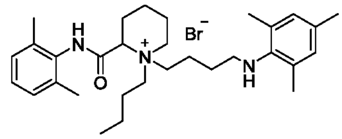

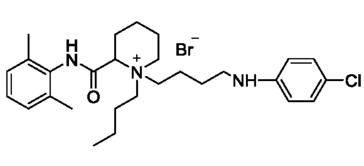
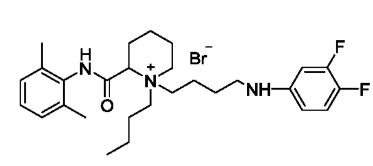
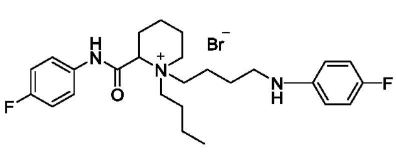

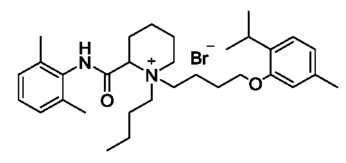
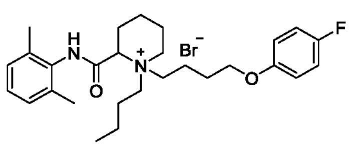
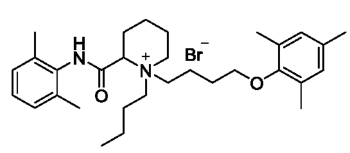

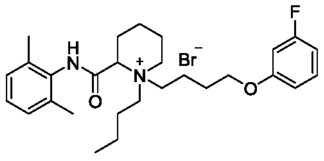 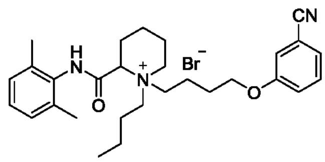 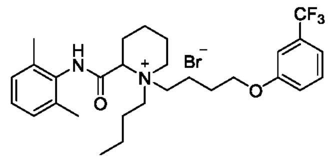

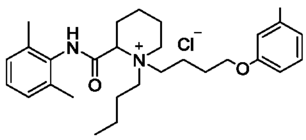 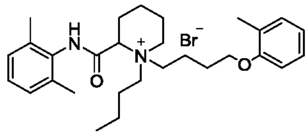 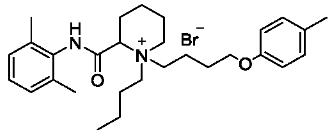

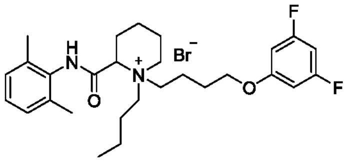 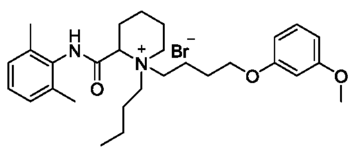

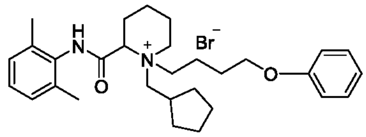 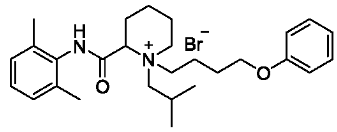 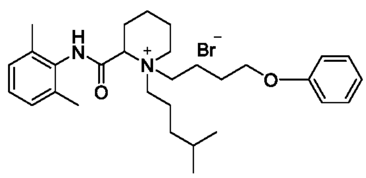

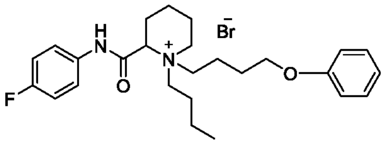 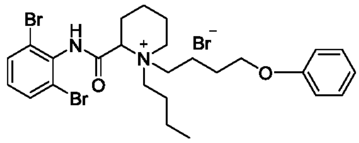 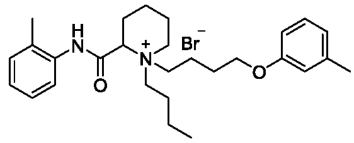

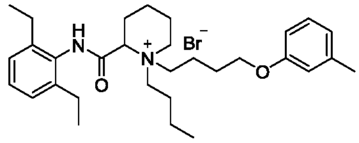 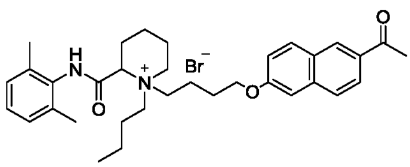 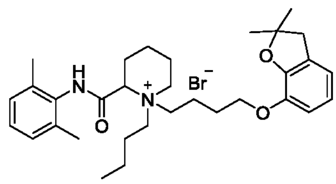 .

**25.** The compound according to claim 24, which is selected from the group consisting of:

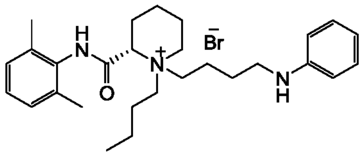 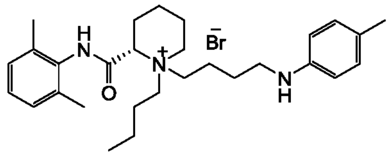 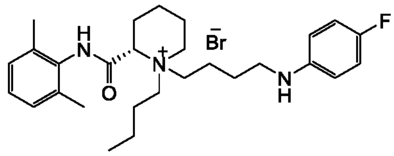

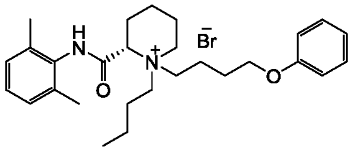 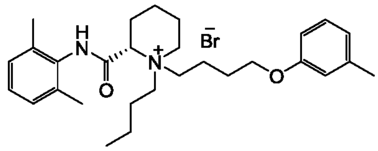 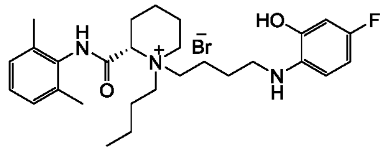

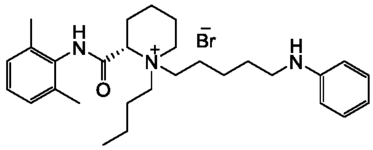
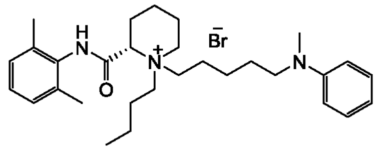
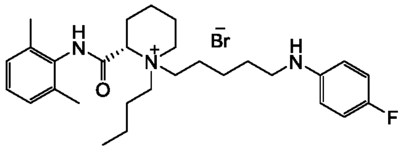
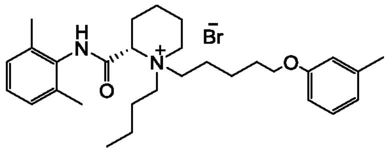
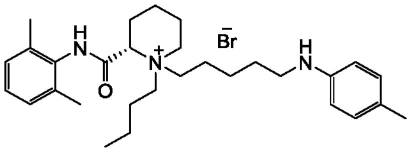
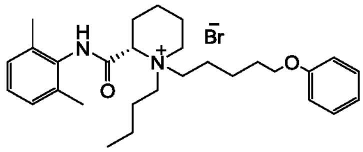
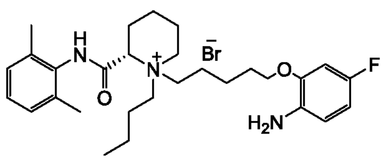
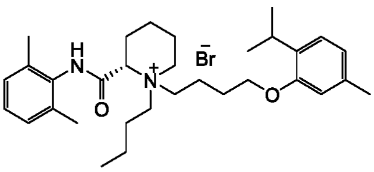
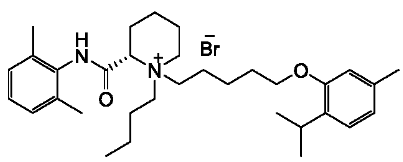
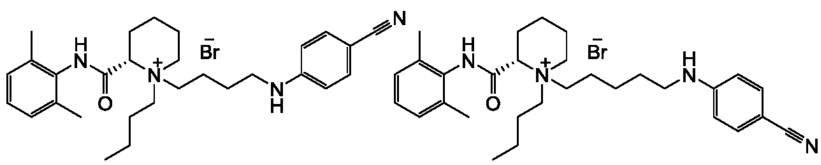
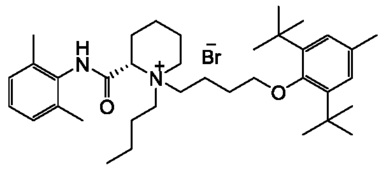
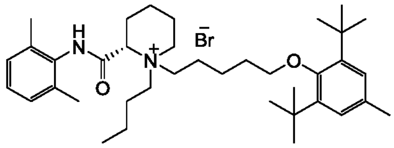
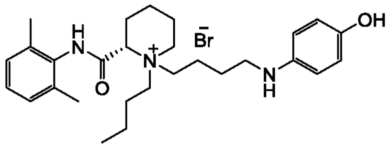
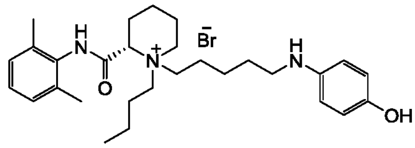
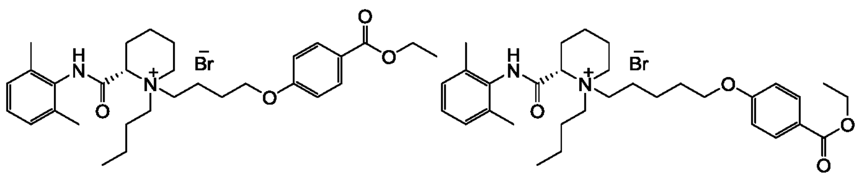
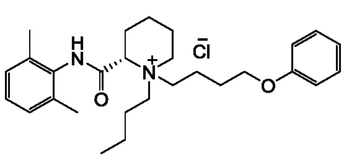
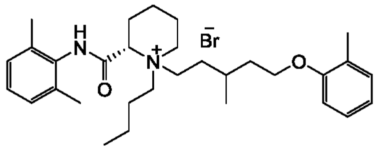
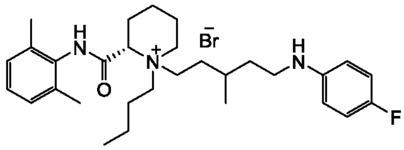
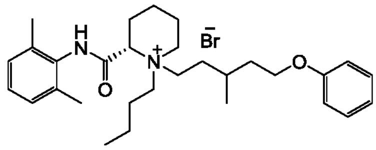
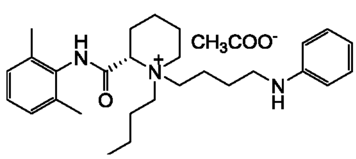
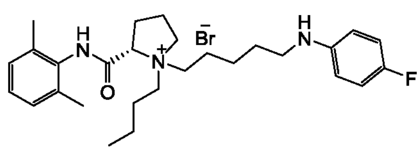
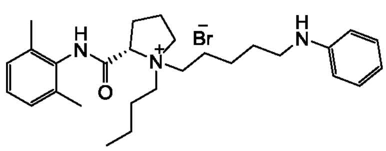
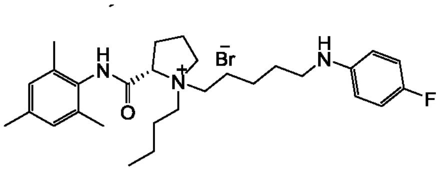
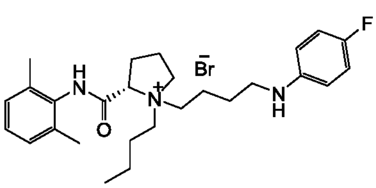
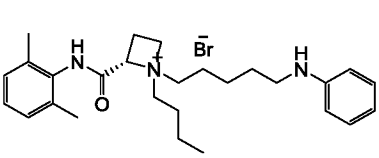

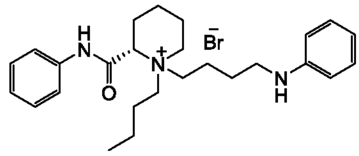
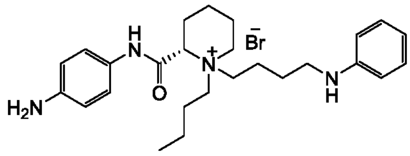
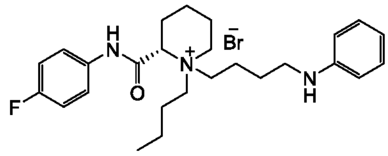

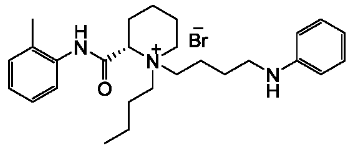
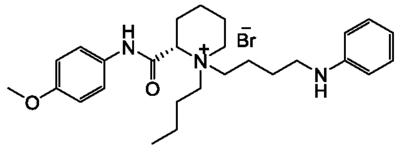
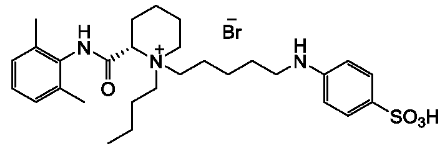

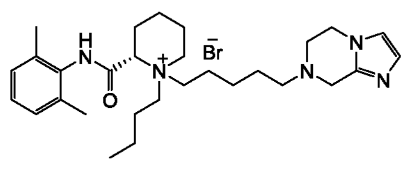
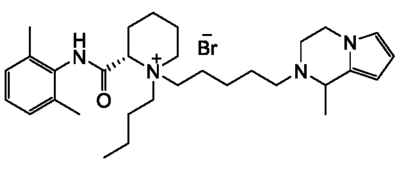
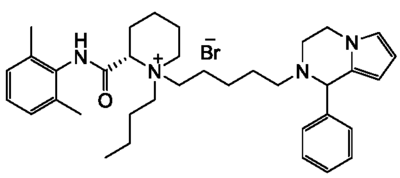

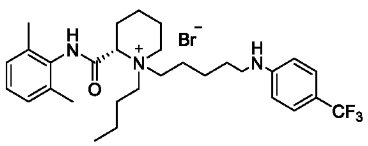
17

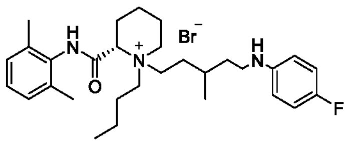
18

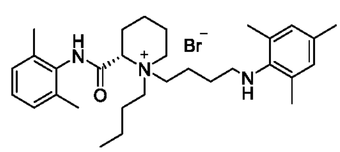
19

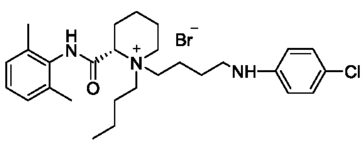
20

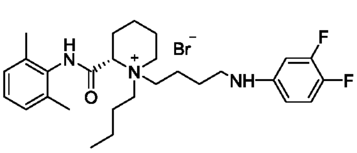
21

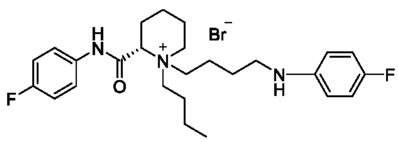
22

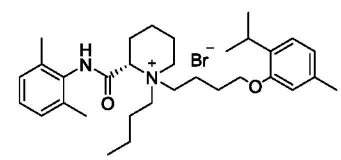
23

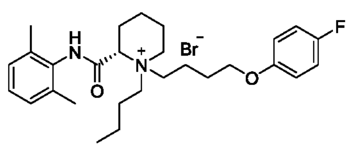
24

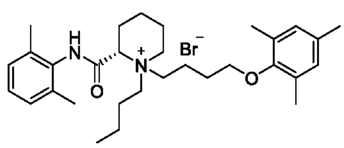
25

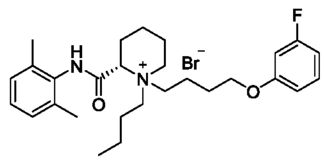
26

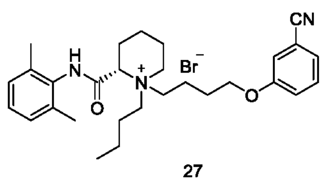
27

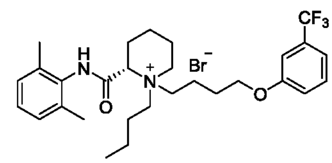
28

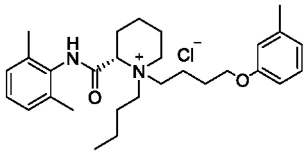
29

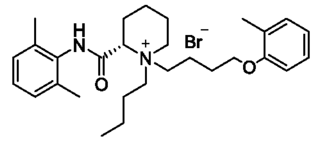
30

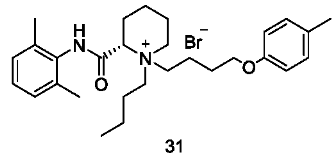
31

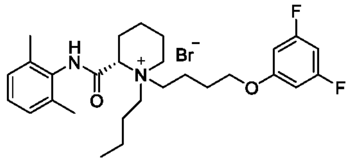

32

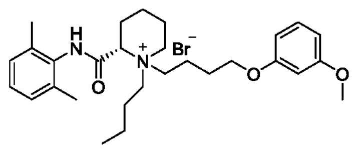

33

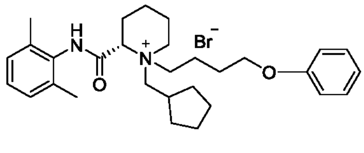

36

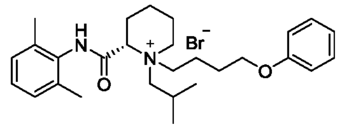

37

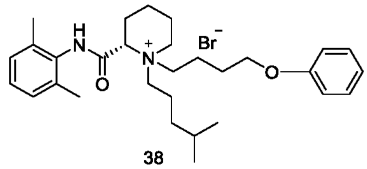

38

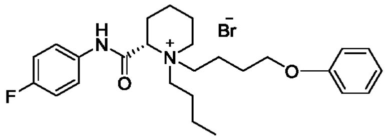

39

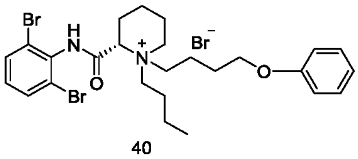

40

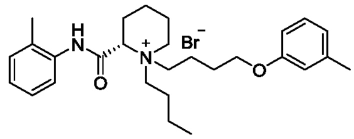

41

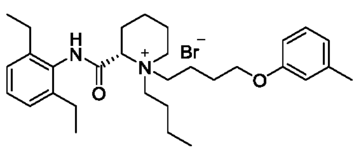

42

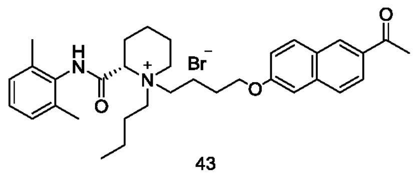

43

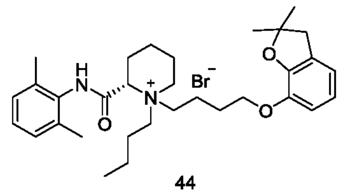

44

.

**26.** A method for producing the compound according to claim 1 or the stereoisomer or solvate thereof, comprising:

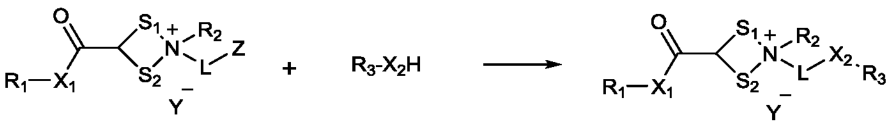

(II) (III) (I)

reacting the compound represented by formula (II) with the compound represented by formula (III) to obtain the compound represented by formula (I);
wherein, Z in formula (II) is an electron-withdrawing leaving group, and the groups in formula (II) and formula (III) are defined as in formula (I).

**27.** A pharmaceutical composition comprising the compound according to any one of claims 1 to 25 or the stereoisomer or solvate thereof and a pharmaceutically acceptable carrier, excipient or diluent.

**28.** Use of the compound according to any one of claims 1 to 25 or the stereoisomer or solvate thereof or the pharmaceutical composition according to claim 27 in the manufacture of a medicament for local anesthesia or analgesia.

**29.** The use according to claim 28, wherein the local anesthesia is selected from the group consisting of conduction anesthesia, surface anesthesia, and infiltration anesthesia; and the analgesia is suitable for pain selected from the group consisting of chronic pain, acute pain, inflammatory pain, cancer pain, neuropathic pain, musculoskeletal pain, primary pain, intestinal pain, and idiopathic pain.

**30.** The use according to claim 28, wherein the medicament is administered topically via transdermal, subcutaneous, intradermal, intramuscular, near nerves, intrapulpal, intraspinal, epidural, intravenous, or mucosal routes such as eye

drops.

**31.** A method of local anesthesia or analgesia, comprising administering to a subject in need thereof a therapeutically effective amount of the compound according to any one of claims 1 to 25 or the stereoisomer or solvate thereof, or the pharmaceutical composition according to claim 27.

**32.** The method according to claim 31, wherein the administration is topical administration via transdermal, subcutaneous, intradermal, intramuscular, near nerves, intrapulpal, intraspinal, epidural, intravenous, or mucosal routes such as eye drops.

**INTERNATIONAL SEARCH REPORT**

International application No.
**PCT/CN2023/094390**

**A. CLASSIFICATION OF SUBJECT MATTER**

C07D211/60(2006.01)i; C07D205/04(2006.01)i; C07D207/16(2006.01)i; A61K31/452(2006.01)i; A61K31/395(2006.01)i; A61K31/397(2006.01)i; A61P23/02(2006.01)i

According to International Patent Classification (IPC) or to both national classification and IPC

**B. FIELDS SEARCHED**

Minimum documentation searched (classification system followed by classification symbols)

IPC: C07D211/-, C07D205/-, C07D207/-, A61K31/-

Documentation searched other than minimum documentation to the extent that such documents are included in the fields searched

Electronic data base consulted during the international search (name of data base and, where practicable, search terms used)

CNKI, CNTXT, VEN, STN: 季铵, 麻醉, 哌啶, 苯基, quaternary ammonium, anesth+, piperidin, phenyl, 根据式(VII)及实施例化合物的结构进行了子结构检索, sub-structure search according to formula (VII) and the structure of compounds of embodiments

**C. DOCUMENTS CONSIDERED TO BE RELEVANT**

| Category* | Citation of document, with indication, where appropriate, of the relevant passages | Relevant to claim No. |
|---|---|---|
| A | CN 106928127 A (SICHUAN HAISCO PHARMACEUTICAL CO., LTD.) 07 July 2017 (2017-07-07)<br>claim 1 and embodiments | 1-30 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **08 September 2023** | **11 September 2023** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2023/094390**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **31, 32**
because they relate to subject matter not required to be searched by this Authority, namely:

The subject matter of claims 31 and 33 relates to a treatment method implemented on the human/animal body (PCT Rule 39.1(iv)).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2023/094390**

| Patent document cited in search report | Publication date (day/month/year) | Patent family member(s) | Publication date (day/month/year) |
|---|---|---|---|
| CN 106928127 A | 07 July 2017 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202210551906


### Non-patent literature cited in the description


- **BECKER D. E. et al.** Local anesthetics: review of pharmacological considerations. *Anesth Prog.*, 2012, vol. 59 (2), 90-102
- **COURTNEY K. R.** Mechanism of frequency-dependent inhibition of sodium currents in frog myelinated nerve by the lidocaine derivative GEA. *J Pharmacol Exp Ther.*, 1975, vol. 195, 225-236
- **ALSALEM M. et al.** Anti-nociceptive and desensitizing effects of olvanil on capsaicin-induced thermal hyperalgesia in the rat. *BMC Pharmacol Toxicol.*, 2016, vol. 17 (1), 31
- *CHEMICAL ABSTRACTS*, 2713-34-0